(19) Europäisches Patentamt
European Patent Office
Office européen des brevets

(11) **EP 1 896 123 B1**

(12) **EUROPÄISCHE PATENTSCHRIFT**

(45) Veröffentlichungstag und Bekanntmachung des Hinweises auf die Patenterteilung:
**13.11.2019 Patentblatt 2019/46**

(21) Anmeldenummer: **06742869.8**

(22) Anmeldetag: **10.05.2006**

(51) Int Cl.:
*A61N 1/36* *(2006.01)*          *G10L 15/02* *(2006.01)*
*A61B 5/04* *(2006.01)*          *A61B 5/12* *(2006.01)*

(86) Internationale Anmeldenummer:
**PCT/EP2006/004399**

(87) Internationale Veröffentlichungsnummer:
**WO 2007/000210 (04.01.2007 Gazette 2007/01)**

(54) **VORRICHTUNG, VERFAHREN UND COMPUTERPROGRAMM ZUR ANALYSE EINES AUDIOSIGNALS**

SYSTEM, METHOD AND COMPUTER PROGRAM FOR ANALYSING AN AUDIO SIGNAL

SYSTEME, PROCEDE ET PROGRAMME INFORMATIQUE POUR ANALYSER UN SIGNAL AUDIO

(84) Benannte Vertragsstaaten:
AT CH LI

(30) Priorität: **29.06.2005 DE 102005030326**

(43) Veröffentlichungstag der Anmeldung:
**12.03.2008 Patentblatt 2008/11**

(73) Patentinhaber: **Fraunhofer-Gesellschaft zur Förderung der angewandten Forschung e.V.**
**80686 München (DE)**

(72) Erfinder:
• **KLEFENZ, Frank**
**68159 Mannheim (DE)**
• **KÁTAI, András**
**98693 Ilmenau (DE)**
• **SZEPANNEK, Gero**
**44227 Dortmund (DE)**
• **HARCZOS, Tamás**
**H-1082 Budapest (HU)**

(74) Vertreter: **Zinkler, Franz et al**
**Schoppe, Zimmermann, Stöckeler**
**Zinkler, Schenk & Partner mbB**
**Patentanwälte**
**Radlkoferstrasse 2**
**81373 München (DE)**

(56) Entgegenhaltungen:
WO-A-01/99470    WO-A-02/084539
US-A- 4 536 844    US-A- 4 980 918

US-A- 5 381 512    US-A- 5 388 182
US-A- 6 064 913

• HOLMBERG M ET AL: "Auditory information processing with nerve-action potentials", ACOUSTICS, SPEECH, AND SIGNAL PROCESSING, 2004. PROCEEDINGS. (ICASSP '04). IEEE INTERNATIONAL CONFERENCE ON MONTREAL, QUEBEC, CANADA 17-21 MAY 2004, PISCATAWAY, NJ, USA,IEEE, PISCATAWAY, NJ, USA, vol. 4, 17 May 2004 (2004-05-17), pages 193-196, XP010718438, DOI: 10.1109/ICASSP.2004.1326796 ISBN: 978-0-7803-8484-2
• Shihab A. Shamma ET AL: "A biophysical model of cochlear processing: Intensity dependence of pure tone responses", The Journal of the Acoustical Society of America, vol. 80, no. 1, 1 July 1986 (1986-07-01), pages 133-145, XP055579502, New York, NY, US ISSN: 0001-4966, DOI: 10.1121/1.394173
• SUMNER CHRISTIAN J ET AL: "A revised model of the inner-hair cell and auditory-nerve complex", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 111, no. 5, 1 May 2002 (2002-05-01), pages 2178-2188, XP012002880, ISSN: 0001-4966, DOI: 10.1121/1.1453451

**Beschreibung**

Die vorliegende Erfindung bezieht sich im Allgemeinen auf eine Vorrichtung, ein Verfahren und ein Computerprogramm zur Analyse eines Audiosignals, um eine Analysedarstellung des Audiosignals zu erhalten, im Speziellen auf eine Vorrichtung, ein Verfahren und ein Computerprogramm zur neurophysiologisch parametrisierten Simulation der ersten Stufen des Hörsystems.

Analyse und Modellierung des menschlichen Gehörsystems bilden schon seit geraumer Zeit einen Schwerpunkt sowohl bei der Erkennung und Klassifizierung von Audiosignalen als auch in der Medizintechnik. Dabei wurde insbesondere der Aufbau des menschlichen Ohrs bereits seit geraumer Zeit studiert. Um ein Verständnis der vorliegenden Erfindung zu erleichtern, werden im Folgenden einige wesentliche Erkenntnisse zu den Grundlagen der Hörwahrnehmung dargestellt.

Physiologie: Auditorische Peripherie und zentrales Gehör

Die physiologischen Gegebenheiten der menschlichen auditorischen Peripherie sind mittlerweile gut erforscht und können in einer Vielzahl wissenschaftlicher Abhandlungen nachgeschlagen werden. Daher sollen an dieser Stelle nur die wesentlichen und zum weiteren Verständnis späterer Ausführungen notwendigen Grundlagen dargestellt werden.

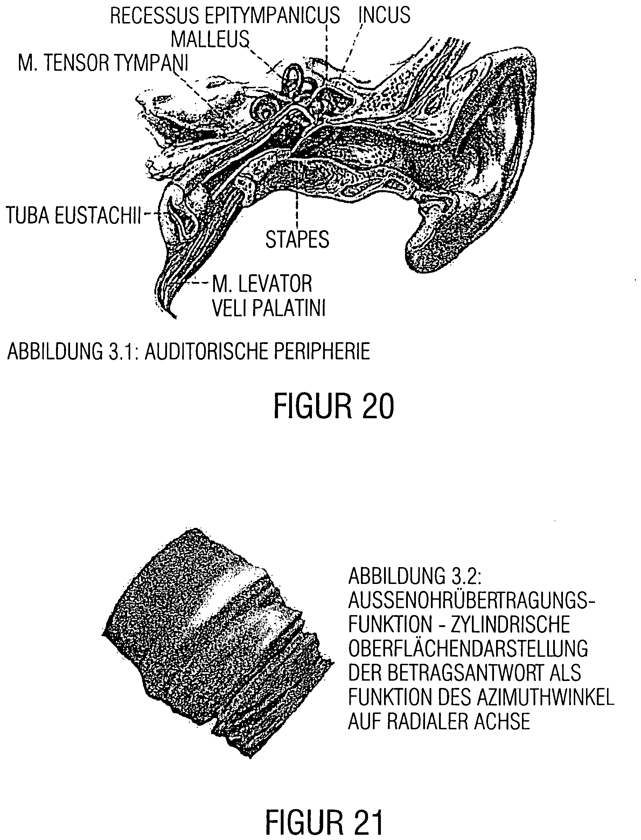

Der periphere Schallverarbeitungsapparat des Menschen (siehe Fig. 20 besteht aus der Gesamtheit von Außen-, Mittel- und Innenohr. Durch den äußeren Gehörgang gelangt Schall zum Trommelfell und wird im Mittelohr über die Gehörknöchelchen weitergeleitet. Anschließende Verarbeitung im Innenohr bewirkt eine frequenzabhängige Transduktion der mechanischen Schwingungen in neuronale Nervenaktionspotentiale und Weitergabe dieser an die angeschlossenen Hörnervenfasern.

Außenohr:

Das äußere Ohr bildet einen Trichter, der die einfallenden Schallwellen zum Trommelfell leitet. Ohrmuschel, Gehörgang, Schädelform und Schulter modifizieren das Schallsignal.

Da der Gehörgang (inkl. Ohrmuschel) an einem Ende geöffnet und am anderen geschlossen ist, wird er physikalisch näherungsweise als halboffenes Rohr aufgefasst. Somit kann im Resonanzfall, d.h. wenn ein Viertel der Schallwellenlänge der effektiven Gehörkanallänge entspricht, ein Schalldruckpegelgewinn beobachtet werden. Im Resonanzmaximum bei ungefähr 2500 Hz beträgt die Verstärkung bis zu 20 dB. Eine zweite Resonanz ("Cavum-Conchae-Resonanz") wird zwischen 2000 Hz und 2500 Hz allein durch die Ohrmuschel hervorgerufen.

In Abhängigkeit von der Schalleinfallsrichtung werden als Resultat der Außenohrform durch sogenannte "richtungsbestimmende Bänder" einzelne schmale Frequenzbereiche angehoben bzw. abgesenkt. Dadurch wird bis zu einem gewissen Maß die Lokalisation eintreffenden Schalls auch ohne binaurale Zeit- und Intensitätsunterschiede insbesondere in der vertikalen Ebene (Median-Sagittal-Ebene) möglich.

Zusammenfassen kann man die beschriebenen Phänomene in der Außenohr-Übertragungsfunktion ("Head related transfer function" HRTF), die in Fig. 21 dargestellt ist.

Mittelohr:

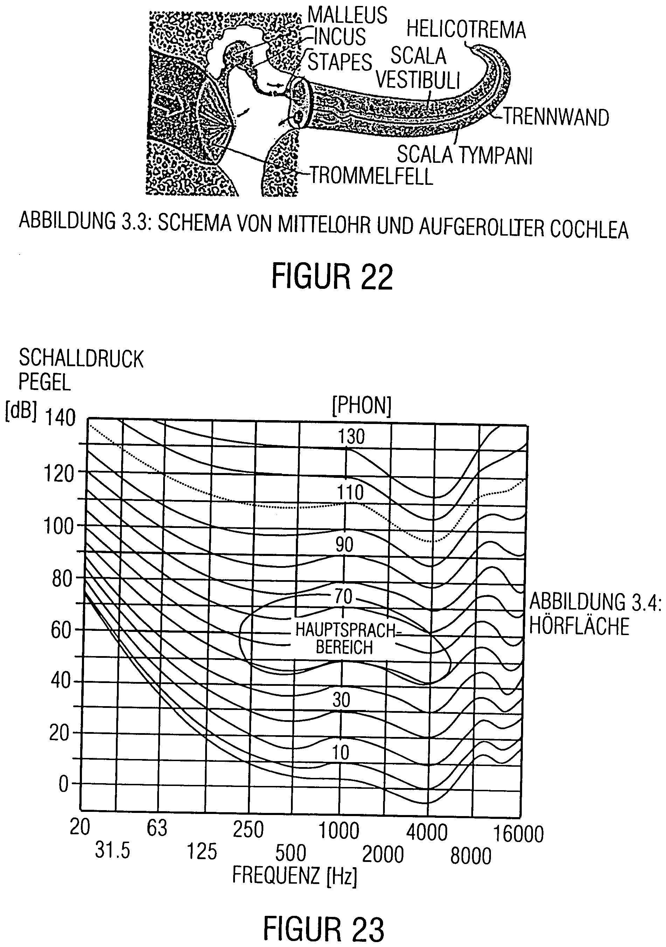

Die wesentliche Aufgabe des Mittelohres (MO) besteht in der Anpassung der Schallkennimpedanzen von Luft und den Flüssigkeiten im Innenohr. Bei Fehlen einer solchen Funktionalität würden wie im Fall der Schallleitungsschwerhörigkeit bis zu 98 % der einfallenden Schallenergie reflektiert. Bei gesundem Mittelohr können aber ungefähr 60 % der Signalintensität an das Innenohr weitergegeben werden. Die hierfür notwendige Schalldruckverstärkung wird möglich durch die aneinandergereihte Kopplung von Trommelfell, den drei Gehörknöchelchen (Hammer, Amboss und Steigbügel) sowie dem ovalen Fenster als Kontaktstelle zum Innenohr (siehe Fig. 22) .

Drei unterschiedliche Mechanismen sind verantwortlich für diese Impedanztransformation:

1. Flächenverhältnis zwischen Trommelfell AT und Steigbügelfußplatte AS:

$$\frac{A_T}{A_S} \cong 17$$

2. Verhältnis der Hebelarme von Hammer $l_H$ und Amboss $l_A$:

$$\frac{l_H}{l_A} \cong 1{,}3$$

3. Hebelarm durch die Biegung des Trommelfells und die unsymmetrische Aufhängung des Hammers:

$$F_T \cong 1{,}4$$

Die Gesamtverstärkung errechnet sich zu:

$$\frac{p_{ges}}{p_T} = F_T \frac{A_T}{A_S} \frac{l_H}{l_A} \cong 30 dB$$

(pT : Schalldruck am Trommelfell).

Bemerkenswert ist die Bedeutung der Transferfunktion des MO, die sich wie ein Bandpassfilter mit breitem Durchlassbereich verhält. Im Niederfrequenzbereich wird sie begrenzt durch die mechanischen Eigenschaften von Trommelfell und ovalem Fenster. Bei hohen Frequenzen limitieren die Trägheitsmomente und Reibungs- bzw. Biegungsverluste der Gehörknöchelchen die Übertragung. Vergleicht man den Verlauf der MO-Übertragungsfunktion mit dem der Hörschwelle (siehe Fig. 23), so sieht man, dass die Hörempfindlichkeitskurve weitestgehend durch die mechanischen Eigenschaften von mittlerem und äußerem Ohr bestimmt wird.

Eine zusätzliche Aufgabe erfüllen die Muskeln des MO (M. tensor tympanus und M. stapedius, siehe Fig. 20). Durch reflektorische Kontraktion kann die MO-Steifigkeit erhöht und so eine Dämpfung tiefer Frequenzen erreicht werden. Begrenzter Schutz gegenüber hohen Pegeln und Verringerung der Wahrnehmung selbstproduzierter Laute sind die Folge.

Innenohr:

Die Struktur des Innenohres setzt sich aus zwei Einheiten zusammen. Während das Vestibularorgan einen Bestandteil des Gleichgewichtssystems darstellt, bildet der Aufbau der Cochlea den abschließenden Teil der auditorischen Peripherie (siehe Fig. 22). Anatomisch gleicht die Cochlea einem Schneckenhaus mit zweieinhalb Windungen. Sie ist durch die cochleäre Trennwand (CT) in die zwei Perilymphflüssigkeit enthaltenden Kammern "Scala vestibuli" (SV) und "Scala tympani" (ST) geteilt (siehe Fig. 22).

Die Arbeitsweise der Cochlea lässt sich wiederum in zwei Abschnitten beschreiben. Der hydromechanische Teil wird bestimmt durch die makro- und mikromechanischen Eigenschaften des Schneckeninneren. Die eigentliche Funktionseinheit zur Umwandlung der Eingangssignale in neuronale Repräsentationen befindet sich innerhalb der cochleären Trennwand. Die Scala Vestibuli ist mit dem Mittelohr verbunden über das ovale Fenster (OF). Dieses schwingt mit der Steigbügelbewegung und zwingt so die inkompressible Lymphflüssigkeit zum Ausweichen. Die Ausweichbewegung wird an die cochleäre Trennwand weitergegeben und bildet eine Wanderwelle in Richtung des Helicotrema (HC), Cochlea-Spitze, aus. Aufgrund der längs ihrer Ausdehnung kontinuierlich veränderlichen mechanischen Eigenschaften (Massenbelag, Steifigkeit, Breite, etc.) bildet die Trennwand an bestimmten Stellen frequenzabhängige Resonanzen aus. Diese tonotopische Frequenzselektivität wird auch als Ortstheorie bezeichnet.

Den charakteristischen Frequenzen können auf der Trennwand Orte der maximalen Wellenamplituden zugeordnet werden, die kontinuierlich von hohen Frequenzen im Bereich des ovalen Fensters (Basis der Basilarmembran) bis zu tiefen Frequenzen am Helicotrema reichen (Ende bzw. Apex der Basilarmembran). Über diese Dispersionseigenschaft können Frequenzinhalte im einkommenden Audiosignal bis zu einem gewissen Grad aufgespalten werden.

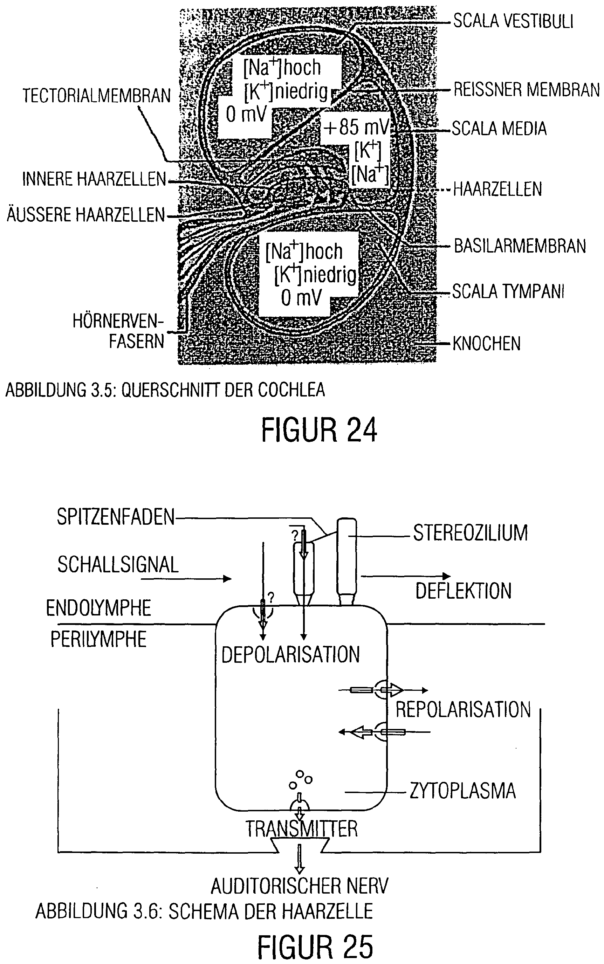

Unterstützt wird diese Funktionalität durch die Eigenschaften der cochleären Trennwand (s. Fig. 24). Diese wird zur Scala Vestibuli hin abgeschlossen durch die Reissnersche Membran (RM). Die Grenzfläche zur Scala Tympani besteht aus der Basilarmembran (BM) mit aufsitzendem Cortischen Organ (CO), auf dessen Oberseite sich in Längsrichtung drei Reihen äußerer Haarzellen und eine Reihe innerer Haarzellen befinden. Diese Haarzellen werden wiederum überspannt von der Tektorialmembran (TM).

Im Bereich dazwischen befindet sich die Endolymphflüssigkeit der Scala Media. Bei Bewegung der cochleären Trennwand geraten die Tektorialmembran und das Cortische Organ in Relativbewegung, was zu einer Auslenkung der auf den Haarzellen befindlichen Sinneshärchen führt. Dies geschieht teils durch direkten Kontakt, teils aber auch durch hydrodynamische Kopplung. Die äußeren Haarzellen besitzen nun die Fähigkeit, sich in Abhängigkeit der Trennwandschwingung sehr schnell zu verkürzen bzw. zu verlängern. Dies führt zu einer bis zu 1000-fachen Verstärkung der Wanderwellenamplituden und liefert spitze und ausgeprägte Schwingungsmaxima.

Ebenso wie die Sinneshärchen der äußeren Haarzellen werden diejenigen der inneren Haarzellen durch die Relativbewegung von Tektorialmembran und Cortischem Organ ausgelenkt. Die nachgemessene 3-dimensionale Bewegung der Cochlea ist kompliziert und wurde beispielsweise von Zenner und Gummert an der Universität Ulm bestimmt. In Folge dieser Bewegung werden biochemische Prozesse in Gang gesetzt, die eine Transduktion der mechanischen Bewegungen in neuronale Aktionspotentiale bewirken (siehe Fig. 25).

Im Ruhezustand besitzen die inneren Haarzellen ein Ruhemembranpotential von ungefähr -40 mV sowie eine niedrige Kalium-Konzentration. Die umgebende Flüssigkeit der Scala Media weist hingegen einen ungewöhnlich hohen Anteil an Kaliumionen auf und ist positiv geladen. Bei Deflektion der Sinneshärchen in eine Richtung öffnen sich sogenannte Transduktionsionenkanäle, durch die zwecks Potentialausgleich ein Einstrom positiv geladener Kaliumionen in die Haarzelle erfolgt. Auslenkung der Sinneshärchen in die entgegengesetzte Richtung verschließt diese Kanäle und durch Ionenverbindungen in die basolaterale Zellmembran kann das ursprüngliche Potential wiederhergestellt werden. Bei geöffneten Kanälen bewirkt das geänderte Sensorpotential eine vermehrte Freisetzung von afferenter Transmittersubstanz.

Diese diffundiert durch den synaptischen Spalt in Richtung Hörnerv. In Abhängigkeit von der Transmitterkonzentration im synaptischen Spalt steigt die Wahrscheinlichkeit der Auslösung eines Nervenaktionspotentials (NAP).

Bis zu einer Frequenz von knapp 5000 Hz folgt die Freisetzung von Transmittersubstanz hochgradig synchron der Deflektion der Sinneshärchen. Somit kann eine lineare Frequenzübertragung über zeitliche Kodierung erfolgen, was in der Literatur auch unter dem Begriff "Phase-Locking" zusammengefasst wird.

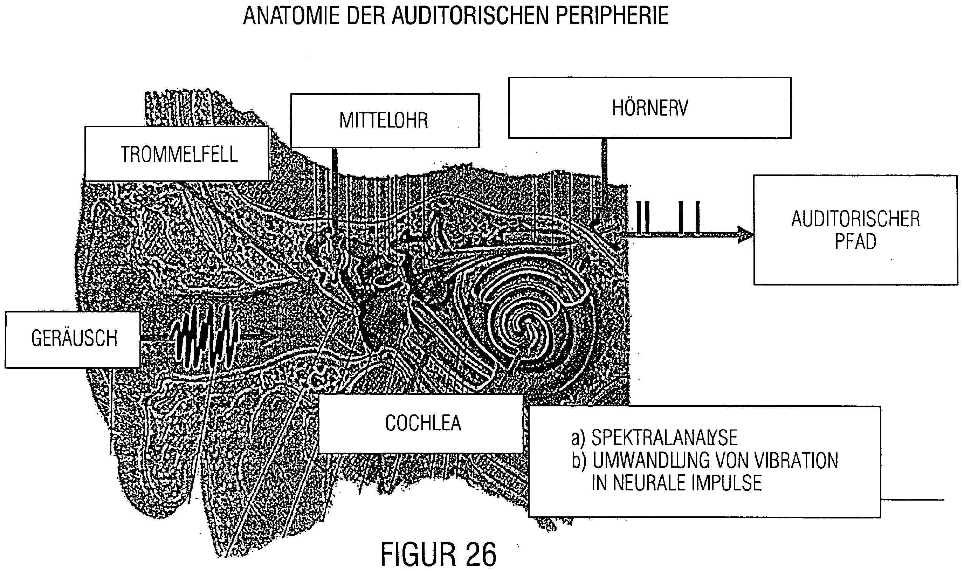

Weiterhin wird auch auf die Fig. 26 verwiesen, die noch einmal die Anatomie der auditorischen Peripherie zeigt. Die Fig. 26 zeigt hierbei die Umwandlung bzw. Weiterleitung eines Geräuschs über Trommelfell und Mittelohr zur Schnecke (Cochlea). Die Cochlea ermöglicht dabei eine Spektralanalyse des eintreffenden Geräuschs sowie eine Umwandlung von Vibrationen in neurale Impulse. Die Cochlea weist ferner Nervenzellen auf, die Nervenimpulse (Aktionspotenziale) erzeugen, welche über den Hörnerv an das Gehirn weitergeleitet werden.

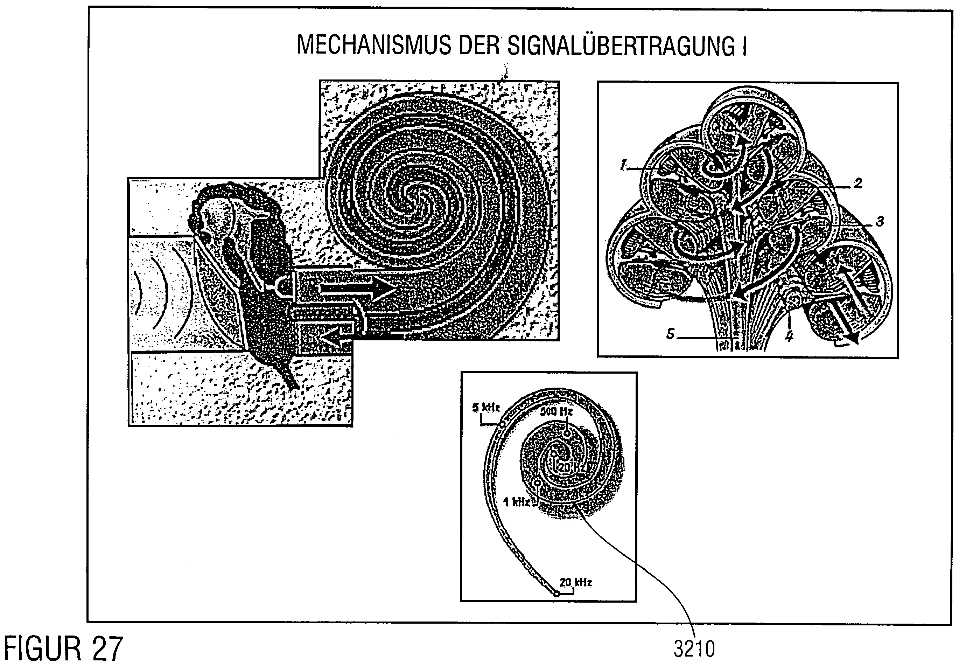

Fig. 27 zeigt noch einmal in schematischer Form den Mechanismus der Signalübertragung im menschlichen Ohr. Aus der Fig. 27 ist ersichtlich, dass die Cochlea 3210 unterschiedliche Frequenzen an unterschiedlichen Orten wahrnimmt (Ortstheorie). Es werden beispielsweise hohe Frequenzen (z.B. mit einer Frequenz von 20 KHz) an dem Anfang der Cochlea in Nervensignale umgewandelt, während niedrige Frequenzen (z.B. mit einer Frequenz von 20 Hz) an dem Ende der Cochlea in Nervensignale umgewandelt werden. Dadurch kann in der Chochlea gleichsam eine Spektralanalyse eines Geräuschs bzw. eines Audiosignals erfolgen, wobei für eine vorgegebene Frequenz jeweils die Nervenzellen am stärksten angeregt werden, die für eine Wahrnehmung der jeweiligen Frequenz optimal ausgelegt sind.

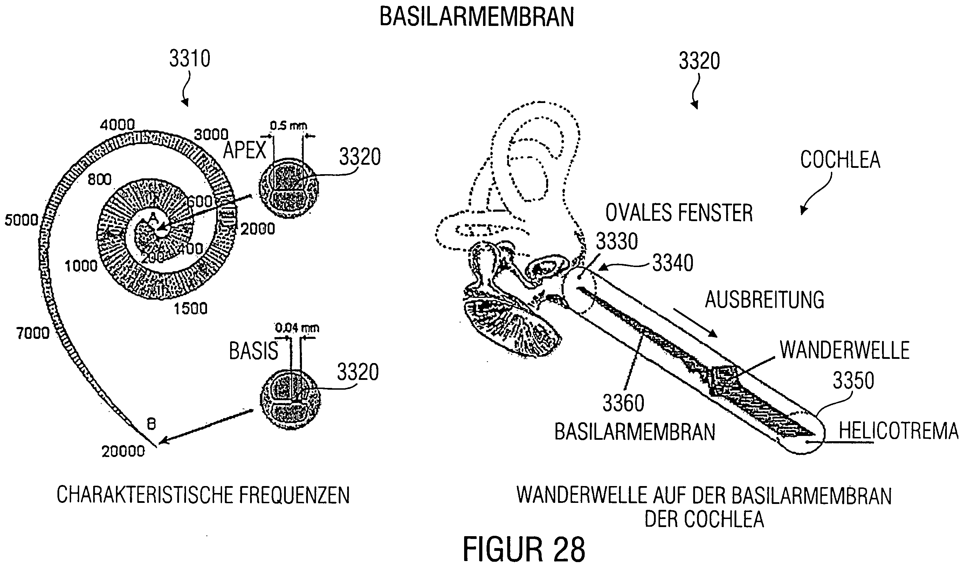

Die Fig. 28 zeigt den Aufbau der Schnecke, wobei auch auf die Geometrie der Basilarmembran eingegangen wird. Eine graphische Darstellung 3310 zeigt hierbei, dass die Breite der Basilarmembran 3320 von der Basis der Cochlea zu dem Ende (Apex) der Cochlea hin um den Faktor 10 zunimmt.

Eine graphische Darstellung 3320 zeigt ferner eine Einkopplung einer akustischen Welle in die Cochlea über ein ovales Fenster 3330. Die Einkopplung über das ovale Fenster 3330 erzeugt eine Wanderwelle in der Cochlea, die von der Basis 3340 der Cochlea zu dem Apex 3350 der Cochlea läuft und dabei die Basilarmembran 3360 der Cochlea auslenkt. Es ist hierbei zu beachten, dass Nervenzellen, die näher bei der Basis 3340 der Cochlea gelegen sind, früher angeregt werden als Nervenzellen, die weiter von der Basis 3340 der Cochlea entfernt sind. Mit anderen Worten, der Ort der Wanderwelle als Funktion der Zeit kann als Trajektorie der Wanderwelle angesehen werden. Die Trajektorie kann freilich auch auf diskrete Nervenzellen abgebildet werden, so dass eine Trajektorie ebenso beschreibt, in welcher zeitlichen Folge mehrere räumlich getrennte Nervenzellen durch eine Wanderwelle angeregt werden.

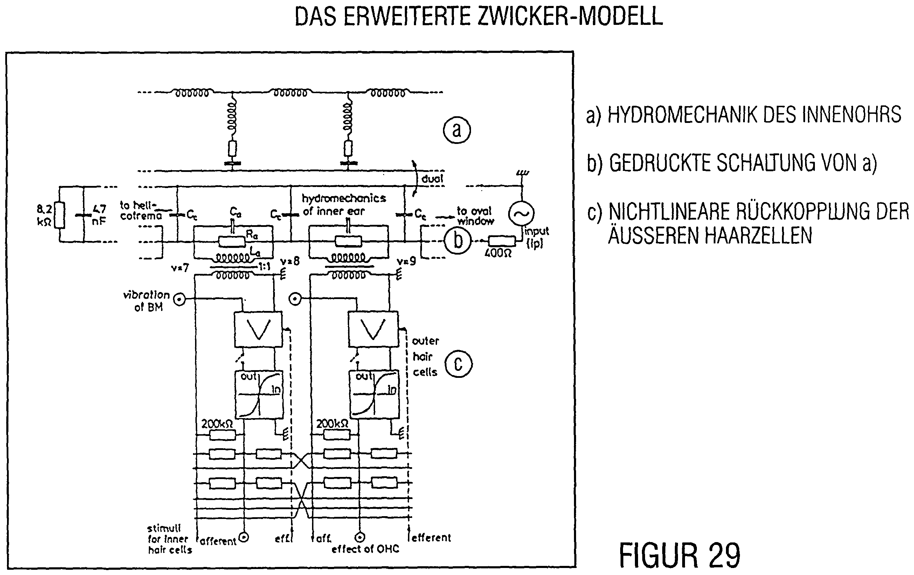

Fig. 29 zeigt ein beispielhaftes elektrisches Ersatzmodell, mit dessen Hilfe die Ausbreitung von Schallwellen durch die Cochlea bis zur Anregung der inneren Haarzellen modelliert werden kann. Das gezeigte Modell ist als "erweitertes Zwicker-Modell" bekannt. Das Modell beschreibt beispielsweise die Hydromechanik des Innenohrs und die nichtlineare Rückkopplung der äußeren Haarzellen. Es wird allerdings darauf hingewiesen, dass das gezeigte Modell nur eines von vielen möglichen Modellen zur Berechnung der Anregung der inneren Haarzellen ist.

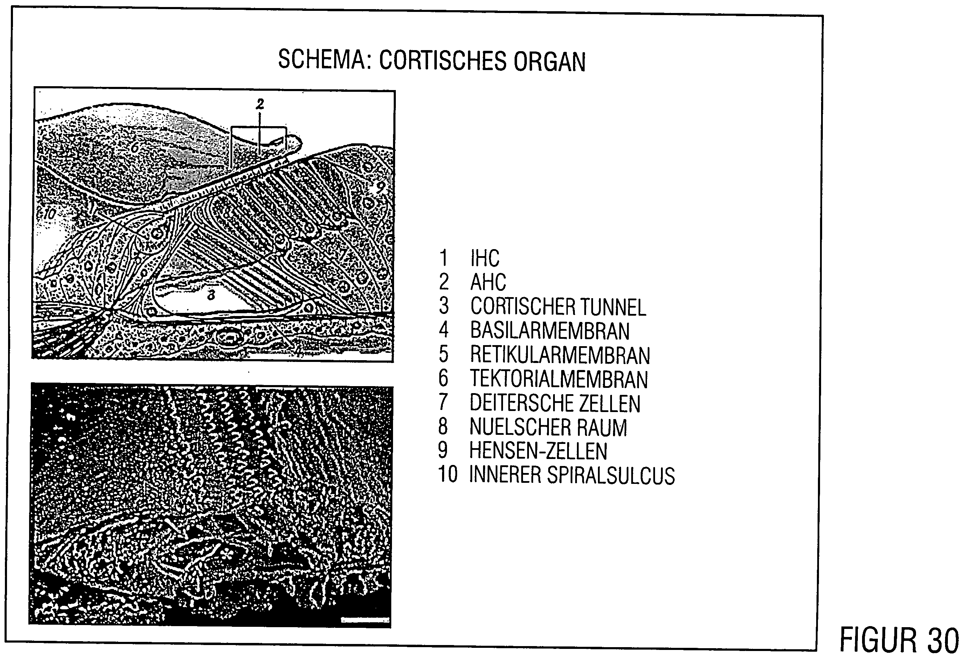

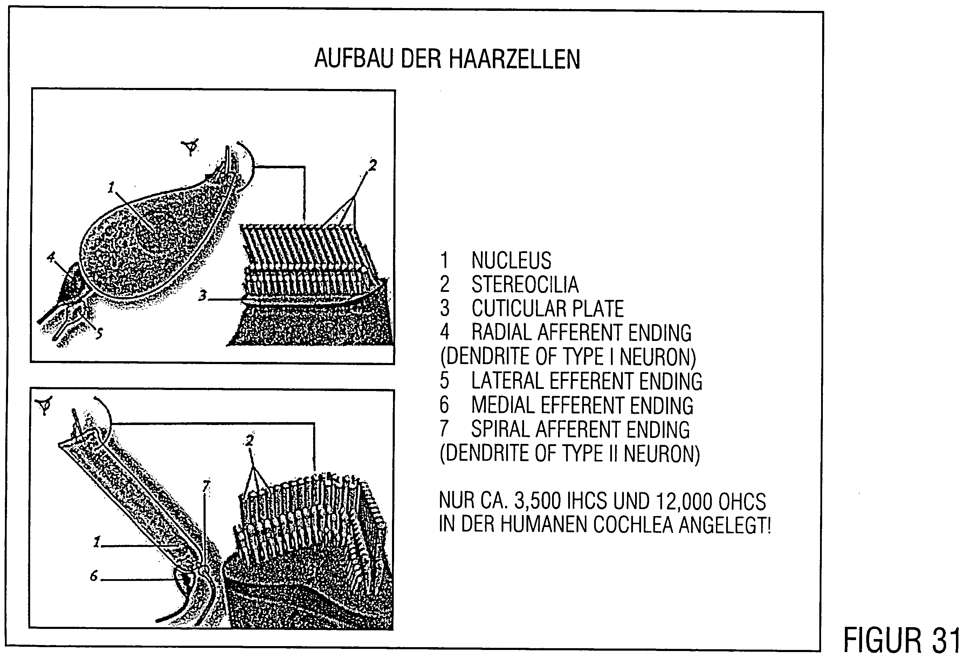

Fig. 30 beschreibt in einer schematischen Darstellung das Cortische Organ, und Fig. 31 beschreibt den Aufbau von zwei verschiedenen Sorten von Haarzellen.

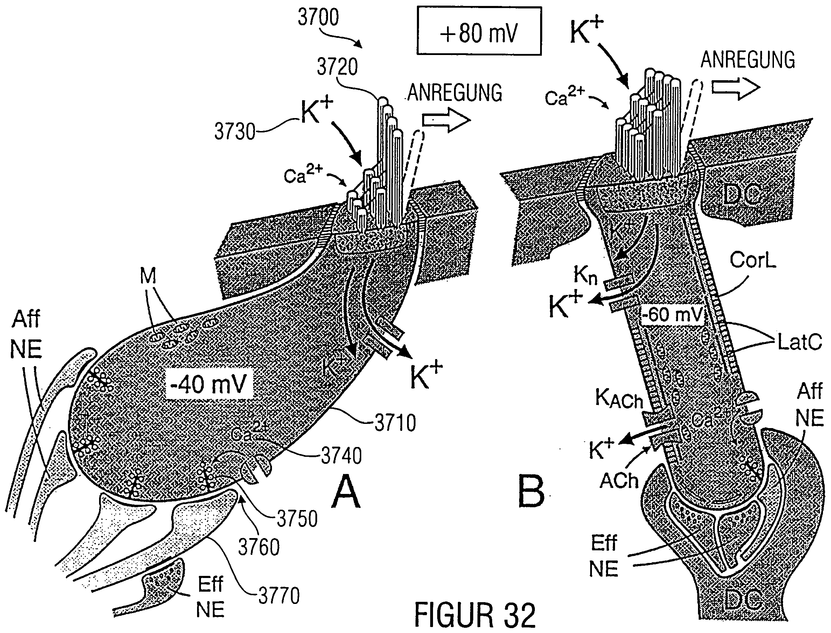

Fig. 32 zeigt eine detaillierte schematische Darstellung von zwei Haarzellen. Die schematische Darstellung der Fig. 32 ist in der Gesamtheit mit 3700 bezeichnet. Anhand der graphischen Darstellung 3700 werden hier zur Verbesserung des Verständnisses die chemischen Abläufe in einer inneren Haarzelle kurz skizziert.

Die Haarzelle 3710 weist eine Mehrzahl von Stereozilien 3720 auf, die die Form von feinen Härchen haben. Eine Anregung bzw. Auslenkung der Stereozilien bewirkt, dass sich die Durchlässigkeit bzw. Leitfähigkeit einer Zellmembran verändert, so dass positiv geladene Kalium-Ionen 3730 in die Haarzelle eintreten können. Dadurch verändert sich das intrazelluläre Potenzial der Haarzellen, das oft mit V(t) bezeichnet wird. In Abhängigkeit von dem intrazellulären Haarzellenpotenzial V(t) können positiv geladene Kalzium-Ionen 3740 in die Zelle eindringen, so dass sich damit die Konzentration an Kalzium-Ionen 3740 erhöht. Die Kalzium-Ionen wirken dann auf die Freigabe von Neurotransmitter-Molekülen 3750 in einen synaptischen Spalt 3760 zwischen der Haarzelle 3710 und einer Nervenfaser 3770. Die Freigabe der Neurotransmitter-Moleküle 3750 erfolgt typischerweise gequantelt in Vesikeln von mehreren tausend Molekülen.

Die Konzentration an Neurotransmittern in dem synaptischen Spalt 3760 verändert dann das Potenzial in dem synaptischen Spalt 3760. Übersteigt das Potenzial in dem synaptischen Spalt 3760 einen bestimmten Schwellwert, so wird schließlich ein Aktionspotenzial in der Nervenfaser 3770 erzeugt.

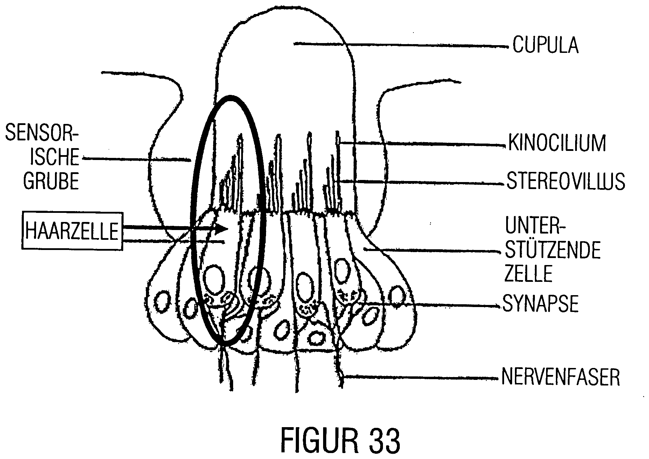

Fig. 33 zeigt schließlich zur Verdeutlichung die Anordnung einer Mehrzahl von Haarzellen in einer sensorischen Grube (sensory pit) einer menschlichen Cochlea. Aus der Darstellung der Fig. 33 geht hervor, dass eine einzige Haarzelle typischerweise eine Mehrzahl von Stereozilien (Härchen) aufweist und mit einer Mehrzahl von Nervenfasern gekoppelt ist.

Es existieren bereits einige Ansätze, um in Anlehnung an die Vorgänge in dem menschlichen Gehör Audiosignale zu verarbeiten bzw. zu identifizieren. Beispielsweise beschreiben Thorsten Heinz und Andreas Brückmann in dem Artikel "Using a physiological ear model for automatic melody transcription and sound source recognition" (Die Verwendung eines physiologischen Ohrmodells zur automatischen Melodieübersetzung und Geräuschquellenerkennung), der auf der 114. Versammlung der Audio-Ingenieur-Gemeinschaft (Audio Engineering Society) in Amsterdam, Niederlande im März 2003 vorgestellt wurde, eine Audiosignalanalyse und an der Wahrnehmung orientierte Modifikationen von konventionellen Signalverarbeitungsalgorithmen.

Der genannte Artikel beschreibt eine Nachbildung der Funktionalität des Innenohrs einschließlich der Umwandlung von mechanischen Vibrationen in Information über eine Konzentration einer Transmittersubstanz in den Spalten der inneren Haarzellen. Die Basilarmembran wird hierbei in 251 Regionen von gleichmäßiger Breite aufgeteilt, und jedes Segment wird an eine innere Haarzelle angeschlossen, wobei die innere Haarzelle durch Vibrationen des entsprechenden Abschnitts der Basilarmembran angeregt wird. Für eine Tonhöhenerkennung wird dann die Konzentration der Transmittersubstanz in den Spalten der 251 beschriebenen Haarzellen ausgewertet.

Dazu werden Tonhöhen-Trajektorien gebildet und segmentiert. Weiterhin beschreibt der genannte Artikel kurz die Erkennung einer Klangfarbe sowie eine Melodieerkennung.

Ferner beschreiben Toshio Irino und Roy D. Patterson in ihrem Artikel "Segregating Information about the size and shape of the vocal tract using a time domain auditory model: The Stabilized Wavelet-Mellin Transform" (Trennen von Informationen über die Größe und die Form des Vokaltrakts unter Verwendung eines Zeitdomänen-Gehörmodells: Die stabilisierte Wavelet-Mellin Transformation)(veröffentlicht in dem Elsevier-Journal for Speech Communication 36, 2002, Seiten 181-203) die Anwendung einer zweidimensionalen Mellin-Transformtion auf ein Gehörbild (Auditory Image). Gemäß dem genannten Artikel erzeugt die Mellin-Transformation aus dem Gehörbild (Auditory Image) ein Mellin-Abbild (Mellin Image), das invariant gegenüber der Größe eines Vokaltrakts eines Sprechers ist, auf dessen Sprachsignal das Gehörbild (Auditory Image) basiert.

Der genannte Artikel schlägt eine Spracherkennung anhand eines sogenannten Mellin-Abbilds vor, das durch eine räumliche Fourier-Transformation aus einem Größen-Form-Bild (Size-Shape-Image) entsteht. Das Größen-Form-Bild wird hingegen gemäß dem T. Irino und R.D. Patterson aus einem stabilisierten Gehörbild (Stabilized Auditory Image) durch eine Mehrzahl von Umwandlungsschritten gewonnen.

Ferner beschreiben A. Brückmann, F. Klefenz und A. Wünsche in dem Artikel "A neural net for 2D-slope and sinusoidal shape detection" (erschienen in dem CIST International Scientific Journal of Computing, ISSN 1727-6209) (Ein neuronales Netzwerk zur Erkennung von zweidimensionalen geraden und sinusförmigen Formen) ein neuronales Netzwerk zur Mustererkennung. Das beschriebene neuronale Netzwerk kann Geraden in verschiedener Steigung oder einem Satz von sinusförmigen Kurven verschiedener Frequenzen lernen und die entsprechenden Muster nach der Lernphase erkennen. Das entsprechende neuronale Netzwerk realisiert damit eine Hough-Transformation und ermöglicht also eine Erkennung von zweidimensionalen Mustern.

Die US 5,381,512 A beschreibt eine Verarbeitung einer Anregungs-Wellenform unter Verwendung eines Modells des menschlichen Gehörsystems, um eine Mehrzahl von Ausgangs-Wellenformen bereitzustellen. Jede Ausgangs-Wellenform entspricht einer Anregung an unterschiedlichen Orten entlang der Basilarmembran in der Cochlea und ist an die schmale Frequenzbandbreite, die kurze Zeitantwort und die Wellenausbreitungscharakteristika der menschlichen Cochlea angepasst. Eine primäre Merkmalsdetektion wird erreicht, indem Antwort-Wellenformen und deren räumliche und zeitliche Ableitung mit vorbestimmen Vergleichsmustern verglichen werden. Eine sekundäre Merkmalsdetektion wird erreicht, indem räumliche und zeitliche Muster von primären Merkmalen mit Mustern von menschlichen Sprachelementen verglichen werden.

Die WO 01/99470 A1 beschreibt einen Audioprozessor für ein Cochlea-Implantat. Der Audioprozessor verwendet ein Modell einer Basilarmembranbewegung, um Stimuli basierend auf einer vorhergesagten Bewegung, die das akustische Signal in einer akustisch angeregten normal-hörenden Cochlea erzeugen würde, zu berechnen. Die verwendeten Filter decken mehrere Kanäle ab und überlappen sich. Die vorhergesagten Elektroden-Stimuli basieren auf einer instantanen vorhergesagten Amplitude an dem Ort der Elektrode.

Die US 4,536,844 beschreibt eine Vorrichtung und ein Verfahren zum Simulieren einer Gehör-Antwort-Information. Sprache und ähnliche Signale werden basierend auf einem Ohrmodell der Funktion des menschlichen Gehörsystems analysiert. Das Modell des Innenohrs wird durch Signalverarbeitungsoperationen ausgedrückt, die akustische Signale auf neurale Darstellungen abbilden. Eine Übertragungsfunktion hoher Ordnung wird durch ein kaskadiertes/paralleles Filterbank-Netzwerk von einfachen linearen, zeitinvarianten Filterabschnitten zweiter Ordnung modelliert. Signalübertragung und Kompression basieren auf einer Halbwellengleichrichtung mit einem nichtlinear-gekoppelten Netzwerk mit automatischer Verstärkungsregelung.

Der Artikel "Auditory Information Processing with Nerve-Action Potentials" von Marcus Holmberg und Werner Hemmert (veröffentlicht in den Proceedings der IEEE International Conference on Acoustics, Speech and Signal Processing, Montreal, Quebec, Canada, band 4, 17. Mai 2004) beschreibt ein Modell eines äußeren Hörsystems, ein Modell einer Innenohr-Hydrodynamik, ein Modell einer Kompressionsstufe und ein Modell einer inneren Haarzelle.

Der Artikel "A biophysical model of cochelar processing: Intensity dependence of pure tone responses" von Shihab A. Shamma und Richard S. Chadwick (veröffentlicht in J. Acoust. Soc. Am 80 (1), Juli 1986) beschreibt eine Berechnung einer Auslenkung der Sterozilien erster Ordnung.

Es ist die Aufgabe der vorliegenden Erfindung, ein Konzept zur Bestimmung einer effizient verarbeitbaren Analysedarstellung eines Audiosignals, die für einen menschlichen Hörer relevante Information umfasst, zu schaffen.

Diese Aufgabe wird durch eine Vorrichtung zur Analyse eines Audiosignals gemäß Anspruch 1, ein Verfahren zur Analyse eines Audiosignals gemäß Anspruch 35 sowie durch ein Computerprogramm gemäß Anspruch 37 gelöst.

Die vorliegende Erfindung schafft eine Vorrichtung zur Analyse eines Audiosignals, um eine Analysedarstellung des Audiosignals zu erhalten. Die erfindungsgemäße Vorrichtung umfasst eine Einrichtung zum Umsetzen des Audiosignals in eine Darstellung, die ein Neurotransmitter-Vesikel-Auftreten in Spalten zwischen einer Mehrzahl von Nervenfasern und inneren Hörzellen eines Ohrmodells wiedergibt, sowie eine Einrichtung zum Berechnen eines Nervenaktivitätsmusters über der Zeit auf der Mehrzahl von Nervenfasern, das sich aufgrund des Neurotransmitter-Vesikel-Auftretens ergibt, wobei das Nervenaktivitätsmuster die Analysedarstellung des Audiosignals ist.

Die vorliegende Erfindung beruht auf der Erkenntnis, dass ein Nervenaktivitätsmuster, das Aktionspotenziale auf Nervenfasern, die mit Hörzellen gekoppelt sind, beschreibt, eine besonders aussagekräftige und effiziente Beschreibung des Audiosignals darstellt. Das Nervenaktivitätsmuster ist nämlich sehr ähnlich einer von einem menschlichen Gehirn ausgewertete Darstellung des Audiosignals. Ferner wurde erkannt, dass für eine realitätsnahe Bestimmung des Nervenaktivitätsmusters eine Berechnung eines in Form von Vesikeln quantisierten Auftretens von Neurotransmittern vorteilhaft ist. Erst durch die Berücksichtigung der Quantisierung von Neurotransmittern in Form von Vesikeln können nämlich Zeitpunkte des Auftretens von Aktionspotenzialen auf den Nervenfasern, die das Nervenaktivitätsmuster bilden, mit ausreichender Genauigkeit bestimmt werden.

Somit unterscheidet sich das erfindungsgemäße Konzept zur Analyse eines Audiosignals wesentlich von bekannten Lösungen, die beispielsweise lediglich die Konzentration an Neurotransmitter-Substanzen für eine Analyse des Audiosignals verwenden. Die Berechnung der Konzentration an Neurotransmitter-Substanzen ist zwar einfacher als die Bestimmung des tatsächlichen Nervenaktivitätsmusters, das sich in Nervenfasern, die mit den Hörzellen gekoppelt sind, ergibt, eine Analyse des Audiosignals unter Verwendung des Nervenaktivitätsmusters ist allerdings wesentlich besser an das menschliche Gehör angepasst als eine Analyse aufgrund der Konzentration der Transmitter-Substanzen.

Im Übrigen ist anzumerken, dass das Nervenaktivitätsmuster wesentlich präzisere Zeitinformation trägt als bisher verwendete Analysedarstellungen (beispielsweise die Konzentration der Neurotransmitter-Substanz). Somit ermöglicht die Berechnung bzw. Bestimmung des Nervenaktivitätsmusters beispielsweise auch eine genaue Auswertung von Phasenverschiebungen zwischen benachbarten Nervenzellen, die für einen Menschen einen hohen Informationswert tragen.

Ferner erfolgt bei einer Berechnung des Nervenaktivitätsmusters aufgrund des Neurotransmitter-Vesikel-Auftretens auch eine Maskierung von Anregungen einer Hörzelle, die während einer Totzeit der Nervenfaser auftreten. Dies ist zweckmäßig, da somit das resultierende Datenaufkommen verringert werden kann. Andererseits geht bei dieser Maskierung trotz der Verringerung der Datenmenge keine für den Menschen relevante Information verloren, da auch in einem tatsächlichen menschlichen Gehörsystem eine derartige Maskierung stattfindet. Somit kann durch Verwendung einer erfindungsgemäßen Vorrichtung erreicht werden, dass eine Analysedarstellung des Audiosignals erzeugt wird, die im Einklang mit Vorgängen in dem menschlichen Gehörsystem ist.

Eine Analysedarstellung des Audiosignals, die in enger Anlehnung an das menschliche Gehör gebildet ist, ermöglicht aber eine besonders effiziente Auswertung des Audiosignals. Es kann nämlich bei einer Auswertung des Nervenaktivitätsmusters erreicht werden, dass all jene Anteile des Audiosignals durch eine nachgeschaltete Einrichtung zur Audiosignalerkennung erkannt werden, die auch von einem Menschen erkannt werden könnten. Umgekehrt werden in dem auf die erfindungsgemäße Weise bestimmten Nervenaktivitätsmuster diejenigen akustischen Ereignisse ausgeblendet, die auch für einen menschlichen Hörer nicht wahrnehmbar wären, wodurch in vorteilhafter Weise ein Datenaufkommen verringert werden kann.

Schließlich erlaubt die erfindungsgemäße Erzeugung eines Nervenaktivitätsmusters einen effizienten Einsatz von neuronalen Netzen bei einer Audiosignalanalyse aufgrund der Analysedarstellung, wobei die neuronalen Netze Teile eines menschlichen Gehirns nachbilden können. Die Verwendung des Nervenaktivitätsmusters als Eingangssignal für ein entsprechendes neuronales Netz ist besonders vorteilhaft, da es sich gezeigt hat, dass eben die Verwendung von natürlich vorkommenden Anregungsmustern (also beispielsweise des erfindungsgemäß erzeugten Nervenaktivitätsmusters) die Implementierung eines neuronalen Netzes in einer besonders einfachen Struktur erlaubt. Dies ergibt sich aus der evolutionären Optimierung des gesamten Gehörapparats.

Ferner wird darauf hingewiesen, dass das in der erfindungsgemäßen Weise unter Auswertung eines Neurotransmitter-Vesikel-Auftretens berechnete Nervenaktivitätsmuster auch für eine Anregung von Nervenfasern eines menschlichen Patienten dienen kann, um beispielsweise einem gehörgeschädigten Patienten zumindest einen Teil seines Gehörempfindens zurückzugeben. Hierbei ist wiederum die erfindungsgemäße Bestimmung eines Nervenaktivitätsmusters von besonderem Vorteil, da erfindungsgemäß Aktionspotenziale auf Nervenfasern in Form des Nervenaktivitätsmusters berechnet werden, die auch bei einem Menschen in ähnlicher Form auftreten würden. Somit ist eine Einkopplung des Nervenaktivitätsmusters in Nervenfasern des Gehörnervs ohne eine weitere Signalwandlung möglich. Damit können unter Verwendung der erfindungsgemäßen Vorrichtung auch Patienten behandelt werden, deren Hörzellen schwerwiegend geschädigt sind, da eine Anregung der Nervenfasern berechnet wird.

Es wird bevorzugt, dass die Einrichtung zum Berechnen des Nervenaktivitätsmusters eine Einrichtung zum Berechnen einer Spannung in einer Nervenzelle aufgrund des Neurotransmitter-Vesikel-Auftretens sowie eine Einrichtung zum Entscheiden über eine Freisetzung eines Aktionspotenzials, das eine einer Nervenfaser zugeordnete Spitze in dem Nervenaktivitätsmuster bildet, basierend auf der Spannung in der Nervenzelle, umfasst. Es hat sich nämlich gezeigt, dass die Spannung in der Nervenzelle, die ein post-synaptisches Potenzial beschreibt, ein wesentliches Kriterium für die Erzeugung einer Spitze in einer Nervenfaser ist. Eine Verwendung der Spannung in einer Nervenzelle, die ein post-synaptisches Potenzial beschreibt, ermöglicht daher eine besonders hohe Zeitgenauigkeit bei der Berechnung des Nervenaktivitätsmusters. Diese hohe Zeitgenauigkeit ist wiederum bei der Bestimmung von Phasenbeziehungen zwischen Aktivitäts-Impulsen auf benachbarten Nervenfasern vorteilhaft.

Ferner hat es sich gezeigt, dass es vorteilhaft ist, die Totzeit von Nervenfasern mit in eine Simulation einfließen zu lassen. Mit anderen Worten, es hat sich als vorteilhaft erwiesen, in dem Nervenaktivitätsmuster nur dann eine Freisetzung eines Aktionspotenzials auf einer Nervenfaser anzunehmen, wenn eine vorhergehende Freisetzung eines Aktionspotenzials auf der Nervenfaser länger als eine vorgegebene Refraktärzeit zurückliegt. Dadurch erfolgt eine Maskierung von zeitlich schnell aufeinander folgenden Neurotransmitter-Vesikel-Auftreten bei der Erzeugung des Nervenaktivitätsmusters, wodurch die zu verarbeitende Informationsmenge des Nervenaktivitätsmusters verringert wird und wodurch folglich das von der erfindungsgemäßen Vorrichtung bereitgestellt Nervenaktivitätsmuster eine effizientere Analyse des Audiosignals erlaubt als bisher bekannte Konzepte.

Ferner wird es bevorzugt, dass die Einrichtung zum Berechnen der Spannung in der Nervenzelle aufgrund des Neurotransmitter-Vesikel-Auftretens eine Einrichtung zum Bestimmen einer Neurotransmitter-Rest-Konzentration aufweist, die ausgelegt ist, um bei der Bestimmung der Neurotransmitter-Rest-Konzentration den Einfluss einer Diffusion von Neurotransmitter-Vesikeln zu berücksichtigen. Ferner wird es bevorzugt, dass die Einrichtung zum Berechnen der Spannung in der Nervenzelle ausgelegt ist, um die Spannung in der Nervenzelle in Abhängigkeit von der Neurotransmitter-Rest-Konzentration zu berechnen. Die Berücksichtigung einer Diffusion von Neurotransmitter-Vesikeln ermöglicht somit eine noch genauere Berechnung eines Zeitpunkts, in dem ein Aktionspotenzial in einer Nervenfaser ausgelöst wird. Somit kann die Präzision der erfindungsgemäßen Vorrichtung weiter gesteigert werden.

Im Übrigen wird es bevorzugt, dass die Einrichtung zum Umsetzen des Audiosignals eine Einrichtung zum Umsetzen des Audiosignals in eine Darstellung, die eine Bewegung einer Basilarmembran in dem Ohrmodell beschreibt, eine Einrichtung zum Umsetzen der Darstellung, die die Bewegung der Basilarmembran beschreibt, in eine Darstellung, die eine Auslenkung eines Stereoziliums einer mit der Basilarmembran gekoppelten Haarzelle beschreibt, und eine Einrichtung zum Umsetzen der Darstellung, die die Auslenkung des Stereoziliums beschreibt, in die Darstellung, die das Neurotransmitter-Vesikel-Auftreten beschreibt, umfasst. Eine solche Ausführungsform wird bevorzugt, da damit ein Neurotransmitter-Vesikel-Auftreten in einer physikalisch und chemisch sehr präzisen Weise berechnet werden kann.

Weiterhin wird es bevorzugt, dass die Einrichtung zum Umsetzen der Darstellung, die die Bewegung der Basilarmembran beschreibt, in eine Darstellung, die die Auslenkung des Stereoziliums beschreibt, ausgelegt ist, um das Stereozilium durch ein Modell eines harmonischen Oszillators zu beschreiben, der eine Feder-Rückstellkraft und eine Dämpfung aufgrund eines laminaren Strömungswiderstands aufweist. Damit kann im Gegensatz zu herkömmlichen Konzepten bei der Erzeugung eines Analysesignals aufgrund eines Audiosignals eine Eigenschwingung der Stereozilien mit berücksichtigt werden. Dadurch ergibt sich wiederum eine erhöhte Genauigkeit bei einer Berechnung der Analysedarstellung des Audiosignals und einer nachfolgenden Audiosignal-Analyse. Schwingungen der Stereozilien verändern nämlich eine Kaliumkonzentration in der Hörzelle und verändern somit den Zeitpunkt, zu dem ein Aktionspotenzial in dem Nervenaktivitätsmuster ausgelöst wird.

Ferner wird es bevorzugt, dass eine Anregung des Oszillatormodells, das das Stereozilium beschreibt, durch eine stochastische thermische Kraft erfolgt. Somit können auch thermische Fluktuationen, die in manchen besonders empfindlichen Hörzellen in einem Ruhezustand eine vergleichsweise hohe Anzahl von Aktionspotenzialen auf den zugeordneten Nervenfasern hervorrufen, bei der Herstellung der Analysedarstellung berücksichtigt werden. Dadurch ergibt sich wiederum eine besonders realitätsnahe Modellierung eines menschlichen Ohrs im Rahmen der Ermittlung der Analysedarstellung. Ferner hat sich gezeigt, dass gerade auch die stochastische Anregung der Stereozilien aufgrund einer stochastischen thermischen Kraft zu einer Herabsetzung der Wahrnehmungsschwelle führen kann. So kann ein extrem schwaches akustisches Signal, das für sich genommen durch die Hörzellen kaum detektierbar wäre, in Kombination mit der stochastisch über eine große Anzahl von Hörzellen verteilten stochastischen thermischen Anregung einen von den Hörzellen detektierbaren Effekt hervorrufen, da das sehr schwache Signal in einer leichten Verschiebung der Verteilung der stochastischen thermischen Anregung resultiert, die aufgrund der großen Anzahl von vorhandenen Hörzellen in dem Nervenaktivitätsmuster nachweisbar ist.

Ferner hat es sich als vorteilhaft erwiesen, für eine Konstante, die eine Feder-Rückstellkraft bei der Auslenkung eines Stereoziliums beschreibt, einen Wert heranzuziehen, der für eine Maus oder für eine junge Ratte gültig ist. Es hat sich nämlich gezeigt, dass eine Federkonstante zwischen $1\times10^{-3}$ Newton/Meter und $6\times10^{-3}$ Newton/Meter ein Analysesignal ergibt, das an ein menschliches Hörempfinden gut angepasst ist.

Weiterhin wird es bevorzugt, dass die Einrichtung zum Umsetzen des Audiosignals in eine Darstellung, die eine Bewegung der Basilarmembran beschreibt, eine Einrichtung zum Berechnen einer mechanischen Schallwandlung in einem Innenohr, die ausgelegt ist, um eine Darstellung einer Anregung eines Trommelfells des Ohrmodells zu erhalten, eine Einrichtung zum Berechnen einer Schallübertragung über Hörknöchelchen, die ausgelegt ist, eine Darstellung einer Anregung eines ovalen Fensters zwischen einem Mittelohr und einer Cochlea basierend auf der Darstellung der Anregung des Trommelfells zu erhalten, eine Einrichtung zum Berechnen einer Schwingungsanregung der Cochlea, die ausgelegt ist, um die Schwingungsanregung der Cochlea basierend auf der Darstellung der Anregung des ovalen Fensters zu bestimmen, und eine Einrichtung zur Berechnung der Bewegung der Basilarmembran aufgrund der Schwingungsanregung der Cochlea umfasst. Eine derartige Auslegung ist vorteilhaft, da somit alle wichtigen in einem menschlichen Ohr auftretenden Effekte in dem Ohrmodell modelliert werden können, so dass das Analysesignal eine Darstellung des Audiosignals ist, wie sie von einem menschlichen Gehirn empfangen werden könnte.

Ferner wird es bevorzugt, dass das in der erfindungsgemäßen Weise bestimmte Nervenaktivitätsmuster für eine Analyse eines Audiosignal-Inhalts des Audiosignals verwendet wird. Die Analyse des Audiosignal-Inhalts des Audiosignals kann beispielsweise durch Vergleichen des Nervenaktivitätsmusters mit einem in einer Datenbank gespeicherten Referenz-Nervenaktivitätsmuster erfolgen. Ein solcher Datenbankvergleich ist besonders einfach realisierbar, da das in der erfindungsgemäßen Weise erzeugte Nervenaktivitätsmuster nicht benötigte Informationen in einer wirkungsvollen Weise unterdrückt. So werden durch Berücksichtigung einer Totzeit einer Synapse während der Totzeit (Refraktärzeit) keine Aktionspotenziale generiert, so dass durch die erfindungsgemäße Erzeugung des Nervenaktivitätsmusters die Redundanz in dem erzeugten Nervenaktivitätsmuster besonders gering ist. Auf der anderen Seite können durch die erfindungsgemäße Bestimmung des Nervenaktivitätsmusters Zeitpunkte, zu denen Aktionspotenziale auf den Nervenfasern auftreten, besonders genau berechnet werden, was wiederum die Genauigkeit der Analyse des Audiosignal-Inhalts erhöht. Die entsprechenden Zeitpunkte sind nämlich charakteristisch für Reaktion des Ohrs auf das Audiosignals und tragen einen besonders hohen Informationsgehalt.

Auch kann eine Tonhöhe des Audiosignals basierend auf dem Nervenaktivitätsmuster leicht bestimmt werden, indem ermittelt wird, auf welcher Nervenfaser eine besonders hohe Aktivität vorliegt. Das erfindungsgemäß bestimmte Nervenaktivitätsmuster kann ferner zur Spracherkennung verwendet werden, wobei beispielsweise Vokale und/oder Konsonanten ermittelt werden können. Daneben wird es bevorzugt, das erfindungsgemäß berechnete Lärmaktivitätsmuster als einen Fingerabdruck des Audiosignals in einer Datenbank abzulegen. Dabei ist eine besonders effektive Informationsspeicherung möglich, da die Aktionspotenzial auf den Nervenfasern, die das Nervenaktivitätsmuster bilden, eine vergleichsweise geringe Informationsmenge darstellen, wobei Redundanzen, die durch ein herkömmliches Kompressionsverfahren nicht erkannt werden könnten, durch die Einrichtung zum Umsetzen des Neurotransmitter-Vesikel-Auftretens in das Nervenaktivitätsmuster bereits entfernt sind.

Schließlich ist es ebenfalls bevorzugt möglich, dass die Vorrichtung zur Analyse des Audiosignals eine Koppeleinrichtung umfasst, die ausgelegt ist, um das Nervenaktivitätsmuster an eine Mehrzahl von Nervenfasern eines menschlichen Patienten in Form von elektrischen und/oder chemischen Anregungen auszugeben. Somit kann ein menschlicher Patient, der beispielsweise einen Gehörschaden aufweist, mit dem Nervenaktivitätsmuster angeregt werden, so dass er eine Hörwahrnehmung hat, die einer natürlichen Hörwahrnehmung ähnlich ist. Die Koppeleinrichtung kann hierbei ein Cochlea-Implantat umfassen.

Ferner wird darauf hingewiesen, dass es besonders vorteilhaft ist, das in der erfindungsgemäßen Weise berechnete Nervenaktivitätsmuster einer Analyseeinrichtung zuzuführen, die eine Einrichtung zum Verarbeiten des Nervenaktivitätsmusters umfasst, wobei die Einrichtung zum Verarbeiten des Nervenaktivitätsmusters bevorzugt ausgelegt ist, um eine Darstellung zu gewinnen, die eine Folge von Zeitinformationen umfasst, die eine zeitliche Lage von aufeinander folgenden Trajektorien beschreibt, wobei eine Trajektorie Aktivitätsimpulse auf verschiedenen Nervenfasern aufgrund des gleichen Ereignisses in dem Audiosignal umfasst.

Dabei kann bei der Berechnung der Trajektorien wiederum die besonders hohe Genauigkeit des erfindungsgemäßen Nervenaktivitätsmusters vorteilhaft ausgenutzt werden, so dass auch die gewonnenen Zeitinformationen eine hohe Genauigkeit aufweisen. Durch diese hohe Genauigkeit können die Zeitinformationen für eine präzise Charakterisierung des Audiosignals verwendet werden.

Bevorzugte Ausführungsbeispiele der vorliegenden Erfindung werden nachfolgend bezugnehmend auf die beiliegenden Zeichnungen näher erläutert. Es zeigen:

Fig. 1 ein Flussdiagramm eines erfindungsgemäßen Verfahrens zum Berechnen einer Analysedarstellung eines Audiosignals aufgrund des Audiosignals gemäß einem ersten Ausführungsbeispiels der vorliegenden Erfindung;

Fig. 2 ein Flussdiagramm eines erfindungsgemäßen Verfahrens zum Berechnen eines Neurotransmitter-Vesikel-Auftretens aufgrund des Audiosignals gemäß dem ersten Ausführungsbeispiel der vorliegenden Erfindung;

Fig. 3 ein Flussdiagramm eines erfindungsgemäßen Verfahrens zum Berechnen einer Basilarmembranbewegung aufgrund des Audiosignals gemäß dem ersten Ausführungsbeispiel der vorliegenden Erfindung;

Fig. 4 ein Flussdiagramm eines erfindungsgemäßen Verfahrens zum Berechnen einer Auslenkung eines Stereoziliums aufgrund der Basilarmembranbewegung gemäß dem ersten Ausführungsbeispiel der vorliegenden Erfindung;

Fig. 5 ein Flussdiagramm eines erfindungsgemäßen Verfahrens zum Berechnen des Neurotransmitter-Vesikel-Auftretens aufgrund der Auslenkung eines Stereoziliums gemäß dem ersten Ausführungsbeispiel der vorliegenden Erfindung;

Fig. 6 ein Blockschaltbild eines erfindungsgemäßen Verfahrens zum Berechnen eines Aktionspotenzials einer Nervenfaser aufgrund des Neurotransmitter-Vesikel-Austretens gemäß dem ersten Ausführungsbeispiel der vorliegenden Erfindung;

Fig. 7 ein Flussdiagramm eines erfindungsgemäßen Verfahrens zum Berechnen einer Analysedarstellung eines Audiosignals gemäß einem zweiten Ausführungsbeispiel der vorliegenden Erfindung;

Fig. 8 eine graphische Darstellung von beispielhaften Nervenaktivitätsmustern;

Fig. 9 eine graphische Darstellung einer Verzögerung bei einer Ausbreitung von Audiosignalen verschiedener Frequenz auf einer Basilarmembran;

Fig. 10 eine graphische Darstellung eines Cochleograms für einen Vokal "i";

Fig. 11 eine graphische Darstellung eines Cochleograms, einer Transmitter-Freisetzungs-Wahrscheinlichkeit und einer Transmitter-Vesikel-Freisetzung für einen Vokal "A";

Fig. 12 eine graphische Darstellung einer Verarbeitungskette für eine erfindungsgemäße Analyse eines Audiosignals gemäß dem zweiten Ausführungsbeispiels der vorliegenden Erfindung;

Fig. 13 ein Blockschaltbild einer Vorrichtung zum erfindungsgemäßen Verarbeiten des Nervenaktivitätsmusters gemäß dem zweiten Ausführungsbeispiel der vorliegenden Erfindung;

Fig. 14 eine graphische Darstellung von Signalen in einer Vorrichtung zum erfindungsgemäßen Verarbeiten des Nervenaktivitätsmusters gemäß dem zweiten Beispiel der vorliegenden Erfindung;

Fig. 15 ein Schaltbild eines Hubel-Wiesel-Netzes zur erfindungsgemäßen Berechnung einer Analysedarstellung eines Audiosignals gemäß dem zweiten Ausführungsbeispiel der vorliegenden Erfindung;

Fig. 16 ein Schaltbild eines Hubel-Wiesel-Netzes zur erfindungsgemäßen Berechnung einer Analysedarstellung eines Audiosignals gemäß dem zweiten Ausführungsbeispiel der vorliegenden Erfindung;

Fig. 17 eine graphische Darstellung von Trainingsmustern zum Trainieren eines Hubel-Wiesel-Netzes;

Fig. 18 eine schematische Darstellung einer Einrichtung zum Identifizieren eines Audiosignals:

Fig. 19 eine schematische Darstellung einer Einrichtung zur Extraktion eines Audiosignal-Inhaltes;

Fig. 20 eine graphische Darstellung der auditorischen Peripherie;

Fig. 21 eine graphische Darstellung der Außenohrübertragungsfunktion;

Fig. 22 eine graphische Darstellung von Mittelohr und aufgerollter Cochlea;

Fig. 23 eine graphische Darstellung einer Hörfläche;

Fig. 24 eine graphische Darstellung eines Querschnitts durch eine Cochlea;

Fig. 25 eine schematische Darstellung einer Haarzelle;

Fig. 26 eine graphische Darstellung einer Anatomie der auditorischen Peripherie;

Fig. 27 eine graphische Darstellung eines Mechanismus der Signalübertragung in einem menschlichen Ohr;

Fig. 28 eine graphische Darstellung der Geometrie der Basilarmembran eines menschlichen Ohrs und einer Reaktion der Basilarmembran auf eine Anregung;

Fig. 29 eine graphische Darstellung eines erweiterten Zwicker[b2]-Modells zur Beschreibung eines Innenohrs;

Fig. 30 eine graphische Darstellung eines Cortischen Organs;

Fig. 31 eine graphische Darstellung eines Aufbaus von Haarzellen;

Fig. 32 eine graphische Darstellung von chemischen Abläufen in einer Haarzelle; und

Fig. 33 eine graphische Darstellung einer sensorischen Grube.

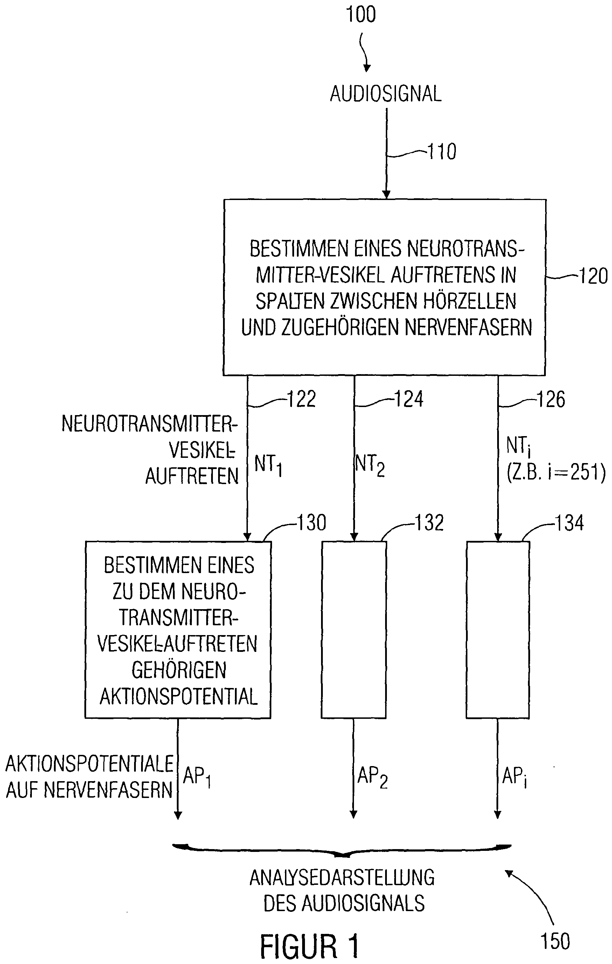

Fig. 1 zeigt ein Flussdiagramm eines erfindungsgemäßen Verfahrens zum Berechnen einer Analysedarstellung eines Audiosignals aufgrund des Audiosignals gemäß dem ersten Ausführungsbeispiel der vorliegenden Erfindung. Das Flussdiagramm der Fig. 1 ist in seiner Gesamtheit mit 100 bezeichnet.

Dabei wird für das Audiosignal 110 in einem ersten Schritt 120 ein Neurotransmitter-Vesikel-Auftreten in Spalten zwischen Hörzellen und zugehörigen Nervenfasern bestimmt. Das Auftreten von Neurotransmitter-Vesikeln, die typischerweise zwischen hundert und mehreren tausend Neurotransmitter-Molekülen umfassen, wird hierbei für eine Mehrzahl von Hörzellen bestimmt, wobei angenommen wird, dass die Hörzellen über ein Modell eines Ohrs räumlich verteilt sind. Es kann beispielsweise angenommen werden, dass die bei der entsprechenden Simulation des Neurotransmitter-Vesikel-Auftretens berücksichtigten Hörzellen äquidistant oder näherungsweise äquidistant über eine Cochlea eines Ohrmodells verteilt sind. Aufgrund der Anregung der Hörzellen durch das Audiosignal kann also für jede der betrachteten Hörzellen ein Auftreten von Neurotransmitter-Vesikeln bestimmt werden.

Basierend auf dem Neurotransmitter-Vesikel-Auftretens 122 kann dann in einem zweiten Schritt 130 in einer betrachteten Hörzelle ein Aktionspotenzial AP 1 auf einer Nervenfaser, die mit der betrachteten Hörzelle gekoppelt ist, bestimmt werden. Das gezeigte Vorgehen kann dann für alle Hörzellen wiederholt werden, die bei der Bestimmung des Neurotransmitter-Vesikel-Auftretens berücksichtigt wurden (Schritte 132 und 134). Es wird somit für alle bei der Bestimmung des Neurotransmitter-Vesikel-Auftretens beteiligten Hörzellen jeweils separat ein Aktionspotenzial AP1, AP2, AP3 auf einer der jeweiligen Hörzelle zugeordneten Nervenfaser aufgrund des der jeweiligen Hörzelle zugeordneten Neurotransmitter-Vesikel-Auftretens 122, 124, 126 berechnet.

Mit anderen Worten, werden i Hörzellen berücksichtigt, so wird für alle i Hörzellen ein zugehöriges Neurotransmitter-Vesikel-Auftreten 122, 124, 126 berechnet. Aufgrund des Neurotransmitter-Vesikel-Auftretens wird dann (für jede Nervenfaser separat) ein zugehöriges Aktionspotential AP1, AP2, AP3 berechnet. Somit liegen nach Abschluss der Berechnung i Aktionspotenziale auf Nervenfasern vor, die zusammen das Nervenaktivitätsmuster bilden.

Das Nervenaktivitätsmuster AP1, AP2, AP3 stellt somit eine Analysedarstellung des Audiosignals dar, die für eine weitere Verarbeitung, beispielsweise eine Audiosignalerkennung, verwendet werden kann.

Das erfindungsgemäße Verfahren bietet somit den Vorteil, dass eine besonders präzise und aussagekräftige Analysedarstellung des Audiosignals gebildet werden kann. Die Aktionspotenziale AP1, AP2, AP3 (die in ihrer Gesamtheit das Nervenaktivitätsmuster bilden) auf den Nervenfasern sind nämlich denjenigen Signalen sehr ähnlich, die von einem menschlichen Gehirn für eine Erkennung von akustischen Ereignissen verwendet werden.

Bei dem erfindungsgemäßen Verfahren, bei dem die Aktionspotenziale AP1, AP2, AP3 auf Nervenfasern von Neurotransmitter-Vesikel-Auftreten 122, 124, 126 abgeleitet werden, kann eine besonders hohe Genauigkeit bei einer nachfolgenden Analyse des Audiosignals erzielt werden. Die Aktionspotenziale auf den Nervenfasern tragen nämlich eine genaue zeitliche Information, da die Aktionspotenziale AP1, AP2, AP3 gequantelt auftreten. Ferner tritt bei der Bestimmung der Aktionspotenziale AP1, AP2, AP3 eine Totzeit (Refraktärzeit) auf, die bei bekannten Verfahren zur Bestimmung von Analysedarstellungen eines Audiosignals nicht berücksichtigt wird.

Im Übrigen ist festzuhalten, dass die Aktionspotenziale AP1, AP2, AP3 aufgrund ihrer Quantelung einfach dargestellt werden können, wobei nicht die Höhe eines Aktionspotenzials, sondern der Zeitpunkt des Auftretens eines Aktionspotenzials bzw. die Rate der nacheinander auftretenden Aktionspotenziale die Information trägt. Auch dadurch unterscheidet sich das erfindungsgemäße Verfahren wesentlich von bekannten Verfahren, bei denen beispielsweise eine Konzentration von Neurotransmittern in einem synaptischen Spalt ausgewertet wird, wobei die Konzentration eine kontinuierliche Kurve darstellt, die keine zeitlich scharf definierten Änderungen aufweist.

Im Übrigen ist festzuhalten, dass sich das Nervenaktivitätsmuster, das simulierte Aktionspotenziale AP1, AP2, AP3 auf einer Mehrzahl von Nervenfasern umfasst, auch verwendet werden kann, um Nervenfasern eines Hörnervs des menschlichen Patienten anzuregen, der beispielsweise einen Gehörschaden aufweist.

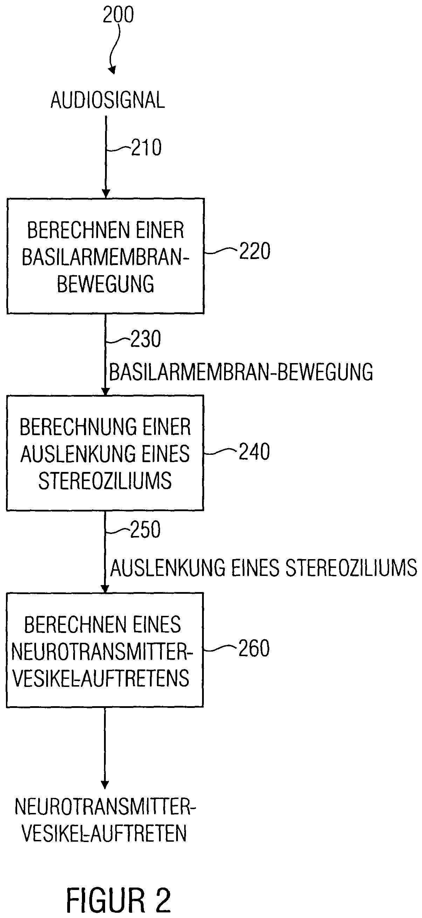

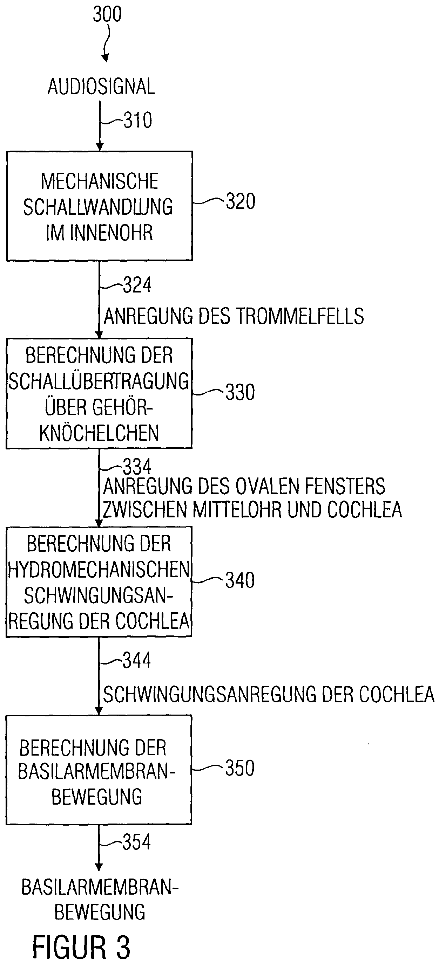

Fig. 2 zeigt ein Flussdiagramm eines erfindungsgemäßen Verfahrens zum Berechnen des Neurotransmitter-Vesikel-Auftretens aufgrund des Audiosignals gemäß dem ersten Ausführungsbeispiel der vorliegenden Erfindung. Das Flussdiagramm der Fig. 2 ist in seiner Gesamtheit mit 200 bezeichnet. Für ein Audiosignal 210 wird in einem ersten Schritt 220 eine zugehörige Basilarmembran-Bewegung 230 berechnet. Mit anderen Worten, basierend auf dem Audiosignal 210 wird eine Bewegung einer Basilarmembran unter Verwendung eines Ohrmodells berechnet. Die Bewegung kann beispielsweise durch Geschwindigkeit und/oder Auslenkung von verschiedenen Punkten der Basilarmembran beschrieben werden. Im Übrigen wird darauf hingewiesen, dass die Berechnung der Basilarmembran-Bewegung anhand von Fig. 3 näher erläutert wird.

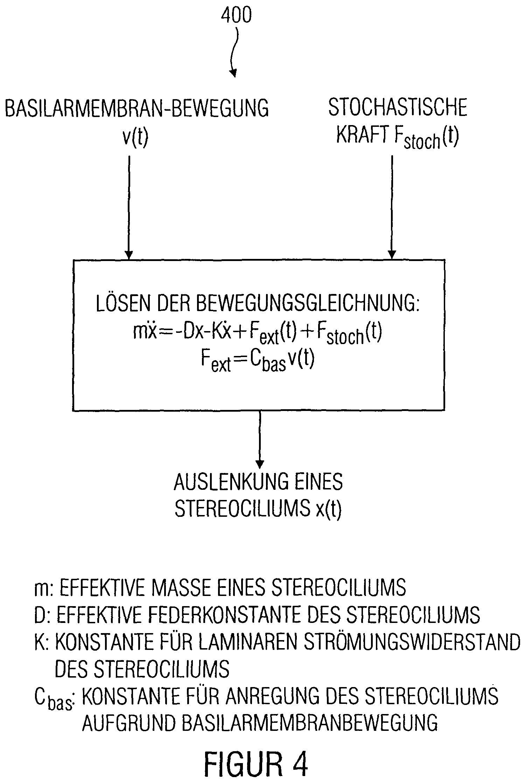

Basierend auf der Basilarmembran-Bewegung 230 wird dann in einem zweiten Schritt 240 eine Auslenkung 250 eines Stereoziliums, das mit der Basilarmembran gekoppelt ist, berechnet. Die Berechnung der Auslenkung des Stereoziliums wird im Übrigen anhand von Fig. 4 näher ausgeführt.

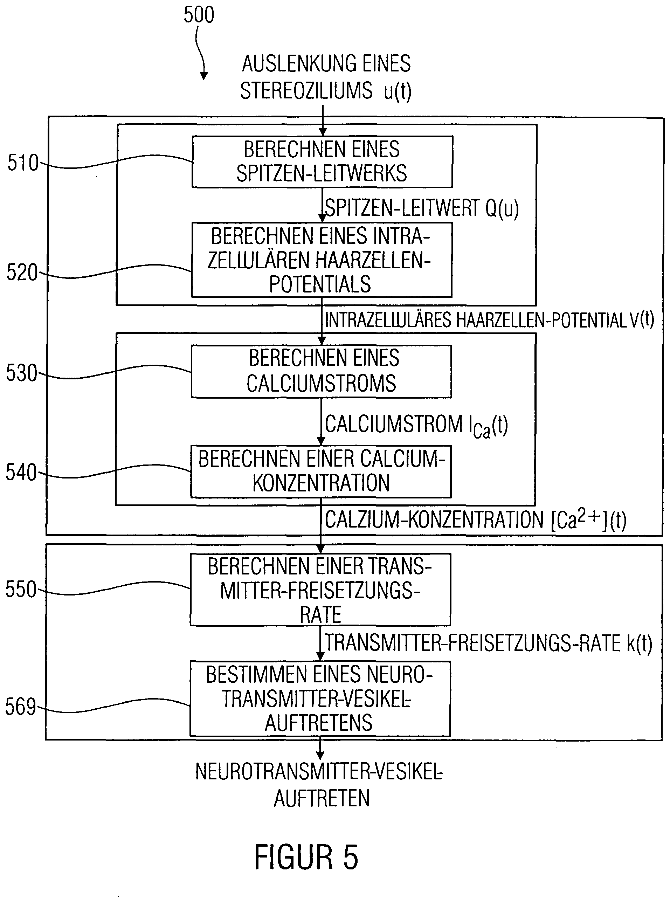

Aufgrund einer bekannten Auslenkung 250 des Stereoziliums kann in einem dritten Schritt 260 das Neurotransmitter-Vesikel-Auftreten berechnet bzw. bestimmt werden. Das Berechnen des Neurotransmitter-Vesikel-Auftretens wird im Übrigen anhand von Fig. 5 noch näher erläutert. Somit liefert das in Fig. 2 gezeigte Verfahren zu einem Audiosignal ein Neurotransmitter-Vesikel-Auftreten für eine oder mehrere Hörzellen. Es wird hierbei darauf hingewiesen, dass die Basilarmembran-Bewegung 230 bevorzugt an den Orten berechnet wird, an denen sich die betrachteten Hörzellen befinden. Es kann jedoch auch die gesamte Bewegung der Basilarmembran berechnet werden, soweit dies eine vorteilhafte Berechnung (beispielsweise unter Verwendung einer analytischen Lösung) ermöglicht.

Fig. 3 zeigt ein Flussdiagramm eines erfindungsgemäßen Verfahrens zum Berechnen einer Basilarmembran-Bewegung aufgrund eines Audiosignals gemäß dem ersten Ausführungsbeispiel der vorliegenden Erfindung. Die graphische Darstellung der Fig. 3 ist in ihrer Gesamtheit mit 300 bezeichnet.

Für ein Audiosignal 310 wird hierbei in einem ersten Schritt 320 eine mechanische Schallwandlung in einem Innenohr basierend auf einem Ohrmodell berechnet. Damit kann eine Anregung 324 des Trommelfells in dem Ohrmodells bestimmt werden. So kann beispielsweise von einem Audiosignal 310 ausgegangen werden, das auf dem Trommelfell eintrifft. Aufgrund mechanischer bzw. fluidischer Berechnungen kann damit die Anregung des Trommelfells ermittelt werden, woraufhin die Schwingung des Trommelfells bekannt ist.

In einem zweiten Schritt 330 kann dann auch die Schallübertragung über die Gehörknöchelchens des Mittelohrs in dem Ohrmodell berechnet werden. Hierbei kann eine detaillierte mechanische Analyse des Mittelohrs erfolgen. Es ist aber genauso gut möglich, lediglich Kraft- bzw. Amplituden-Übersetzungsverhältnisse der Gehörknöchelchen zu berücksichtigen, wodurch sich eine sehr einfache Berechnung ergibt. Weiterhin ist es möglich, die Trägheit der Gehörknöchelchen und/oder fluidische Einflüsse wie Dämpfung mit zu berücksichtigen. Ferner kann schließlich für eine Berechnung der Schallübertragung über die Gehörknöchelchen auch noch berücksichtigt werden, dass die Übertragungscharakteristik des Mittelohrs in Abhängigkeit von der Schallintensität variieren kann. Unabhängig von der Komplexität des Modells, das zur Berechndung der Schallübertragung über die Gehörknöchelchen in einem zweiten Schritt 330 verwendet wird, kann als Ergebnis der beschriebenen Berechnung eine Anregung 334 des ovalen Fensters zwischen Mittelohr und Cochlea ermittelt werden.

Aufgrund einer Kenntnis der Anregung 334 des ovalen Fensters zwischen Mittelohr und Cochlea kann dann in einem dritten Schritt 340 die hydromechanische Schwingungsanregung der Cochlea berechnet werden. Dies kann durch eine geeignete hydromechanische Simulation oder anhand eines vereinfachten analytischen Modells erfolgen. Somit sind als Ergebnis einer Analyse der Cochlea fluidische Strömungen in der Cochlea bekannt bzw. können ermittelt werden.

Basierend auf einer Kenntnis der Schwingungsanregung 344 der Cochlea kann schließlich in einem vierten Schritt 350 eine Bewegung 354 der Basilarmembran berechnet werden. Hierbei können wiederum lineare oder nichtlineare mechanische Modelle zum Einsatz kommen. Es wird darauf hingewiesen, dass eine Vielzahl von Möglichkeiten besteht, um die Basilarmembran-Bewegung an ausgewählten Orten, an denen die betrachteten Nervenzellen angeordnet sind, zu berechnen.

Es wird ferner darauf hingewiesen, dass es nicht notwendig ist, alle gezeigten Zwischengrößen (also die Anregung 324 des Trommelfells, die Anregung 334 des ovalen Fensters oder die Schwingungsanregung 344 der Cochlea) explizit zu berechnen. Vielmehr existieren auch Verfahren, um näherungsweise direkt von dem Audiosignal 310 auf eine Bewegung 354 der Basilarmembran zu schließen. Hierbei kann beispielsweise ein lineares oder ein nicht-lineares Filter zum Einsatz kommen, um eine Bewegung eines vorgegebenen Punkts einer Basilarmembran (an dem sich bevorzugterweise eine Hörzelle befindet) zu ermitteln. Für die Berechnung der Basilarmembran-Bewegung 354 an mehreren Orten kann dann eine Mehrzahl von verschiedenartig ausgelegten Filtern verwendet werden. Ein Filter beschreibt dabei eine Antwort eines Ortes der Cochlea als Reaktion auf das akustische Signal.

Im Übrigen wird darauf hingewiesen, dass von F. Baumgarte ein besonders vorteilhaftes Gehörmodell für eine Modellierung der Cochlea vorgeschlagen wurde (F. Baumgarte: "Ein psychophysiologisches Gehörmodell zur Nachbildung von Wahrnehmungsschwellen für die Audiocodierung", Dissertation, Universität Hannover, 2000). Das Modell von Baumgarte erlaubt eine besonders vorteilhafte Modellierung der Cochlea eines Ohrmodells, wobei wesentliche Effekte wie etwa eine Signalreflexion an dem Ende der Cochlea mit berücksichtigt werden können.

Fig. 4 zeigt ein Flussdiagramm eines erfindungsgemäßen Verfahrens zum Berechnen einer Auslenkung eines Stereoziliums aufgrund der Basilarmembran-Bewegung. Das Flussdiagramm der Fig. 4 ist in seiner Gesamtheit mit 400 bezeichnet.

Es wird hierbei davon ausgegangen, dass die Bewegung der Basilarmembran nach einer der oben genannten Methoden (oder auch auf andere Weise) berechnet wurde, so dass eine Geschwindigkeit der Bewegung der Basilarmembran für diejenigen Orte, an denen sich zu betrachtende Nervenzellen befinden, bekannt ist. Eine Auslenkung x(t) (zum Teil auch als u(t) bezeichnet) eines Stereozilium kann durch Lösen einer Bewegungsgleichung in Abhängigkeit von einer Basilarmembran-Bewegung bestimmt werden. Die Basilarmembran-Bewegung ist hierbei freilich als eine relative Bewegung aufzufassen, die bevorzugt durch eine Relativgeschwindigkeit v(t) beschrieben wird. Ferner wird es bevorzugt, als Anregung für das Stereozilium zusätzlich eine stochastische Kraft $F_{stoch}(t)$ zu berücksichtigen, die durch eine Stoßbewegung der Atome verursacht wird. Mit anderen Worten, es wird bevorzugt, bei der Anregung des Stereoziliums die Brownsche Bewegung mit zu berücksichtigen. Somit ergibt sich die Bewegungsgleichung eines Stereoziliums als inhomogene Bewegungsgleichung eines harmonischen Oszillators der Form

$$m\ddot{x} = -Dx - K\dot{x} + F_{ext}(t) + F_{stoch}(t)$$

Hierbei ist m die effektive Masse des betrachteten Stereoziliums, D eine effektive Federkonstante des Stereoziliums, und K eine Konstante für einen laminaren Strömungswiderstand des Stereoziliums, die die fluidische Dämpfung des Stereoziliums beschreibt. Die Anregung des Stereoziliums, die durch die externe Kraft $F_{ext}$ ausgedrückt wird, ist proportional zu der Relativgeschwindigkeit v(t) der Basilarmembran-Bewegung, so dass gilt:

$$F_{ext} = C_{Bas} v(t).$$

Dabei ist $C_{Bas}$ eine Konstante für eine Anregung des Stereoziliums aufgrund einer Bewegung der Basilarmembran. Wie schon erwähnt ergibt die Auswertung der gezeigten Bewegungsgleichung eine Auslenkung x(t) des Stereoziliums, die bisweilen auch mit u(t) bezeichnet wird.

Es wird im Übrigen darauf hingewiesen, dass auch andere Verfahren zur Berechnung einer Auslenkung der Stereozilien verwendet werden können. So ist es beispielsweise möglich, eine Bewegungsgleichung erster Ordnung zu verwenden, die ein Tiefpassverhalten des Stereoziliums modelliert. Mit anderen Worten, es kann bei einer alternativen Beschreibung davon ausgegangen werden, dass ein Stereozilium die Tiefpasssystem darstellt, das durch eine Bewegungsgleichung der Form eine Gleichung

$$\tau_c \dot{u}(t) + u(t) = \tau_c C_{cilia} v(t).$$

Im Übrigen sei darauf hingewiesen, dass die Berücksichtigung der durch die Brownsche Bewegung hervorgerufenen Kraft $F_{stoch}(t)$ optional ist.

Fig. 5 zeigt ein Flussdiagramm eines erfindungsgemäßen Verfahrens zum Berechnen eines Neurotransmitter-Vesikel-Auftretens aufgrund einer Auslenkung eines Stereoziliums gemäß dem ersten Ausführungsbeispiel der vorliegenden Erfindung. Das in Fig. 5 gezeigte Flussdiagramm ist in seiner Gesamtheit mit 500 bezeichnet.

Aufgrund einer Auslenkung u(t) eines Stereozilium kann beispielsweise in einem ersten Schritt 510 ein Spitzen-Leitwert (apical conductance) G(u) berechnet werden. Eine Auslenkung eines Stereoziliums verändert nämlich eine Anzahl von offenen Ionen-Kanälen, wodurch sich ein Leitwert einer Membran einer Hörzelle ändert.

Aufgrund des aus dem ersten Schritts 510 bekannten Spitzen-Leitwerts G(u) kann somit in einem zweiten Schritt 520 ein intrazelluläres Haarzellen-Potenzial V(t) berechnet werden. Bei der Berechnung dieses Membranpotenzials des Zellkörpers kann beispielsweise ein Modell einer passiven elektrischen Schaltung verwendet werden, was die Berücksichtigung einer Zellkapazität ermöglicht. Weiterhin können die Leitfähigkeiten verschiedener Membranen der Hörzelle sowie die durch verschiedene Ionen bedingten Potenziale berücksichtigt werden.

Nach einer Berechnung des intrazellulären Haarzellen-Potenzials V(t) in dem zweiten Schritt 520 kann in einem dritten Schritt 530 ein Kalziumstrom $I_{Ca}$(t)berechnet werden. Die Freisetzung von Neurotransmittern in den synaptischen Spalt wird nämlich durch Kalzium-Ionen vermittelt. Hierbei kann beispielsweise aufgrund des intrazellulären Haarzellen-Potenzials V(t) ein Anteil von offenen Kalziumkanälen bestimmt werden. Der Kalziumstrom an sich ist wiederum abhängig von der Anzahl der offenen Kalziumkanäle sowie einer Potenzialdifferenz zwischen dem intrazellulären Haarzellen-Potenzial V(t) und einem entgegengerichteten Potenzial für das Kalzium. Im Übrigen sei angemerkt, dass die Anzahl der offenen Kalziumkanäle einer Trägheit unterliegt, die ebenfalls berücksichtigt werden kann.

Aufgrund des Kalziumstroms $I_{Ca}$(t) kann dann in einem vierten Schritt 540 eine Kalziumkonzentration [$Ca^{2+}$] (t) bestimmt werden. Hierbei kann wiederum beispielsweise ein Tiefpass-Charakter berücksichtigt werden, wobei angenommen werden kann, dass die Kalziumkonzentration in einem Gleichgewichtszustand einen konstanten Wert annimmt.

Aufgrund der Kalziumkonzentration [$Ca^{2+}$] (t) kann ein in einem fünften Schritt 550 eine Transmitter-Freisetzungs-Rate k(t) ermittelt werden. Hierbei kann bevorzugt die Speicherung der Transmitter-Substanzen in einem Reservoir, in dem synaptischen Spalt und in einem Verarbeitungsspeicher berücksichtigt werden. Aufgrund der Transmitter-Freisetzungs-Rate k(t) kann in einem damit gekoppelten sechsten Schritt 560 ein Auftreten bzw. eine Freisetzung von Neurotransmitter-Vesikeln bestimmt werden. Es wird hierbei bevorzugt, von einer gequantelten und stochastischen Freisetzung von Neurotransmitter-Vesikeln auszugehen, wobei ein stochastischer Transport durch eine Funktion N(n,p) beschrieben werden kann. Dabei kann beispielsweise davon ausgegangen werden, dass jedes von n Neurotransmittern-Quanten in einem Simulationsintervall eine gleiche Freisetzungswahrscheinlichkeit hat. Im Übrigen wird darauf hingewiesen, dass auch die Rückführung von Neurotransmittern bzw. Neurotransmitter-Vesikeln in einem Reservoir bei einer Modellierung mit eingeschlossen werden kann.

Es wird ferner darauf hingewiesen, dass ein vorteilhaft einsetzbares Modell einer inneren Haarzelle in dem Artikel "A revised model of the inner hair cell and auditory nerve complex" von C.J. Sumner, E.A. Lopez-Poveda, L.P. O'Mard und R. Meddis (J. Acoust. Soc. Am., Vol. 111, No. 5, Pt. 1, May 2002) beschrieben ist.

Es wird allerdings ferner darauf hingewiesen, dass auch andere Modelle für eine Berechnung eines Neurotransmitter-Vesikel-Auftretens aufgrund einer Auslenkung u(t) eines Stereoziliums verwendet werden können. Allerdings hat es sich gezeigt, dass es besonders empfehlenswert ist, ein Modell zu verwenden, das eine stochastische Freisetzung von Neurotransmitter-Vesikeln berücksichtigt, da eine solche stochastische Freisetzung ein zu berechnendes Nervenaktivitätsmuster deutlich besser beschreibt als Modelle, die von einer kontinuierlichen bzw. nichtgequantelten Freisetzung von Neurotransmitter-Vesikeln ausgehen. Wie schon erwähnt kann im Übrigen durch eine Auswertung der Neurotransmitter-Vesikel-Freisetzung eine genauere Information über ein Audiosignal erhalten werden als bei Verwendung kontinuierlicher Freisetzungs-Modelle, bei denen keine scharf definierten zeitlichen Übergänge stattfinden.

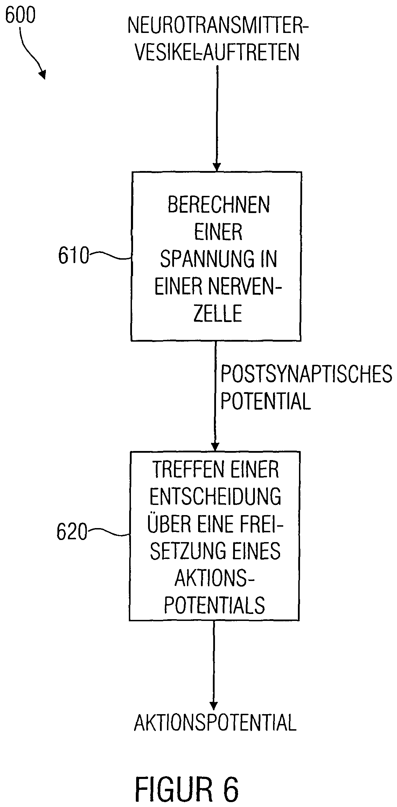

Fig. 6 zeigt ein Blockschaltbild eines erfindungsgemäßen Verfahrens zum Berechnen eines Aktionspotenzials einer Nervenfaser aufgrund eines Neurotransmitter-Vesikel-Auftretens gemäß dem ersten Ausführungsbeispiel der vorliegenden Erfindung. Das Flussdiagramm der Fig. 6 ist in seiner Gesamtheit mit 600 bezeichnet.

Bei dem gezeigten Verfahren zum Berechnen eines Aktionspotenzials aufgrund eines Neurotransmitter-Vesikel-Auftretens wird in einem ersten Schritt 610 eine Spannung in einer Nervenzelle aufgrund des Neurotransmitter-Vesikel-Auftretens berechnet. Bei dieser Spannung handelt es sich beispielsweise um ein post-synaptisches Potenzial bzw. eine Depolarisation der post-synaptischen Nervenzelle. Für eine Berechnung der Spannung in der Nervenzelle kann beispielsweise eine Diffusion von Neurotransmittern berücksichtigt werden. Mit anderen Worten, eine durchschnittliche Neurotransmitter-Konzentration kann durch Auswertung eines Faltungsintegrals bestimmt werden, wobei in dem Faltungsintegral ein Diffusions-Typ-Kern ausgewertet werden kann. Eine solche Berücksichtigung des Diffusionsverhaltens wird beispielsweise in dem Artikel "Estimating Transmitter Release Rates from Postsynaptic Current Fluctuations" von Erwin Neher und Takeshi Sakaba (The Journal of Neuroscience, December 15, 2001, 21(24): 9638-9654) beschrieben. Bei der Berechnung des post-synaptischen Potenzials können ferner eine Kapazität der Zellmembran sowie Membranpotenzial-abhängige Leitfähigkeiten von Membran-Ionenkanälen berücksichtigt werden. In ein entsprechendes Modell zur Berechnung einer Spannung in der Nervenzelle fließt somit ein Ionenaustausch durch eine Zellmembran sowie eine Anregung durch die Neurotransmitter ein.

Aufgrund des in dem ersten Schritt 610 berechneten post-synaptischen Potenzials kann dann in einem zweiten Schritt 620 eine Entscheidung über eine Freisetzung eines Aktionspotenzials getroffen werden. Hierbei wird davon ausgegangen, dass sich durch die Freisetzung der Neurotransmitter das post-synaptische Potenzial erhöht.

Zusammenfassend lässt sich also festhalten: Die inneren Haarzellen sind die Verbindung zwischen der Basilarmembran und den auditiven Nervenfasern (ANF), die Impulse in Richtung Gehirn senden. Bevor jedoch eine am postsynaptischen Ende befindliche ANF ein Aktionspotenzial in Richtung Gehirn senden kann muss sich Ihre Spannung ausreichend erhöhen und einen Schwellenwert überschreiten. Dazu ist eine Verkettung von Ereignissen in der pre-synaptischen IHC nötig, die die mechanischen Schwingungen in elektrische Signale umwandelt.

Am oberen Ende einer IHC befinden sich Haarbündel- drei Reihen von Stereozilien - die durch sogenannte Tip-Links miteinander verbunden sind. Sie folgen der schwingenden Bewegung der Basilarmembran - gedämpft durch die Flüssigkeit, in der sie sich befinden und pendeln durch ihre Steifigkeit wieder in die Ausgangslage zurück. Die Stereozilienbewegung lässt sich als ein System gekoppelter harmonischer Oszillatoren auffassen. Vereinfacht wird sie durch folgende Gleichung beschrieben

$$\tau_c \dot{x}(t) + x(t) = \tau_c C_{cilia} v(t)$$ .

wobei x(t) für die Auslenkung der Stereozilien und v(t) für die Geschwindigkeit der BM steht. $\tau c$ ist eine Zeitkonstante, Ccilia ein Gain-Faktor. Bei niedrigen Frequenzen bewegen sich die Stereozilien phasengleich mit der BM-Geschwindigkeit, bei hohen Frequenzen phasenverschoben.

Im Inneren der IHC Zellmembran liegt ein erheblich niedrigeres Potenzial vor als außerhalb. Dieses ist im Wesentlichen beeinflusst durch die vorliegenden Konzentrationen an (positiv geladenen) Kalium-Ionen. Es herrscht ein stetiger Ionenfluß zwischen innen und außen.

Im ruhenden Zustand stellen sich Gleichgewichtskonzentrationen von [K+] = 130mmol/l (innen) sowie [K+] = 155mmol/l außen ein. Es wird davon ausgegangen, dass auch im Ruhezustand etwa 15% der, an den Stereozilienenden befindlichen Ionenkanäle geöffnet sind. Bewegen sich die Stereozilien nun in medialer Richtung führt das zu einer Öffnung von zusätzlichen Kanälen. Die Leitfähigkeit zwischen Innerem und Äußerem der IHC wird erhöht. Dadurch fließen vermehrt postiv geladene Kaliumionen von außen nach innen und die Spannung im Inneren der IHC steigt an. Andersherum hemmt eine Auslenkung der Stereozilien in entgegengesetzter (lateraler) Richtung den Ionenflusses und führt damit zu einem Absinken des Membranpotenzials.

Die Stereozilien reagieren hochgradig sensibel auf Bewegung. Bereits sinusförmige Auslenkung um +/-0.003 Grad wird als hörbares Signal wahrgenommen. Aber auch ohne externe Anregung durch die schwingende Basilarmembran bewegen sich die Stereozilien. Sie unterliegen Brownscher Bewegung, so dass auch ganz ohne eingehende Schallwelle - je nach Art der Nervenfaser - bis zu über 100 Aktionspotenziale je Sekunde emittiert werden.

Depolarisierung der Haarzelle öffnet spannungsabhängige Calciumkanäle in der Zellmembran, die in der Nähe sogenannter aktiver Zonen liegen. Das sind Regionen am afferenten Ende des synaptischen Spalts, in denen sich Neurotransmitter befinden. Von außen können so verstärkt Ca++-Ionen in die IHC eintreten. Dieser Zustrom von Calcium-Ionen führt zur Freisetzung von Neurotransmittern. Eine hohe Geschwindigkeit bei der Signalübertragung wird dadurch gewährleistet dass die Botenstoffe bereits in sogenannten Vesikeln etwa gleicher Anzahl von einigen 1000 Neurotransmittermolekülen "verpackt" und abrufbereit sind. Modelliert werden kann dies durch sogenannte Pools von verfügbaren Vesikeln.

Die Freisetzung der Transmittermoleküle in den synaptischen Spalt geschieht, in dem das Vesikel an dafür vorgesehenen Stellen der pre-synaptischen Membran, den Aktiven Zonen, diffundiert, sich mit der Membran verbindet, und seinen Inhalt - die Neurotransmitter - in den Spalt freigibt. Leert sich der Pool zur Verfügung stehender Vesikel, so wird er nach und nach wieder aufgefüllt. Ferner empfiehlt sich die Verwendung eines Reprocessing Pool, in dem Transmitter aus dem Spalt mit einer gewissen Rate wieder zu Vesikeln verpackt werden und von dort aus direkt wieder in den freien Pool gelangen. Ein Teil der Transmitter geht im synaptischen Spalt verloren.

Die Ausschüttung von Neurotransmittern aus dem Pool von Vesikeln wird im Allgemeinen als binomialverteilt beschrieben. Die Emissionswahrscheinlichkeit ist dabei von der Calciumkonzentration im inneren der IHC-Zellmembran in der Nähe der aktiven Zonen abhängig.

Die ursprüngliche Modellierung des Spannnungsverlaufs in Nervenzellen geht auf Hodgkin und Huxley aus dem Jahr 1952 zurück. Dabei sind im Wesentlichen der Austausch von Kalium- [K] und Natrium-Ionen [Na] sowie ein spezifisches "Leck" [L] (im Wesentlichen von CL⁻-Ionen) von Bedeutung, die sich durch unterschiedliche maximale Leitfähigkeiten (gNa, gK, gL) und den zeitlichen Verlauf der Membrandurchlässigkeit, in Abhängigkeit vom vorliegenden Potenzial (modelliert durch die von u(t) abhängigen Variablen m, h, n) auszeichnen. Die Membranspannung u(t) verhält sich gemäß

$$\mathtt{u'C \ = \ -\Sigma I}_k \mathtt{(t) \ + \ I(t)}$$

$$\mathtt{\Sigma \ Ik \ = \ (u{-}VNa)\,m^3hgNa{+}(u{-}VK)\,n^4gK \ +(u{-}VL)\,gL}$$

wobei u' die Ableitung nach der Zeit bezeichnet. VNa, VK und VL sind Nernst-Gleichgewichtspotenziale, die die Richtung des Ionenaustauschs determinieren. Ik(t) stellt den Ionenaustausch durch die Zellmembran dar, I(t) dagegen ist ein externer Strom - hier z.B. durch die von den IHC emittierten Neurotransmitter - und C der Kondensator, den die Zellmembran bildet. Wenn ein externer Strom in die Zelle strömt, lädt sich der Kondensator und es entsteht Leck durch die Membran-Ionenkanäle. Das zeitliche Verhalten der Leitfähigkeiten in Abhängigkeit vom Membranpotenzial wird durch ein System dreier Differentialgleichungen beschrieben:

$$m' = \alpha m(u)(1 - m) - \beta m(u)m,$$

$$n' = \alpha n(u)(1 - n) - \beta m(u)n,$$

$$h' = \alpha h(u)(1 - h) - \beta h(u)h.$$

Die Funktionen $\alpha i(u)$ und $\beta i(u)$ in Abhängigkeit von der Spannung u sind von Hodgkin und Huxley für i = {m, n, h} angepasst worden. Es ergeben sich unterschiedlich schnelle Reaktionen der verschiedenen Ionenflüsse auf eine externe Spannungsänderung, die den Spannungsverlauf in der Membran charakterisieren. Diffundiert nun ein Vesikel aus der presynaptischen IHC in den Spalt, so bindet es an einem Rezeptorprotein der post-synaptischen Membran und setzt Ladung frei. Durch die Moleküle eines Vesikels erhöht sich das post-synaptische Potenzial um ein so bezeichnetes mEPP (miniature end-plate potenzial) von etwa 0.5- 1 mV.

Überschreitet die Depolarisation der post-synaptischen Nervenzelle, die durch das post-synaptische Potential beschrieben wird, einen bestimmten Schwellwert v, so kommt es beispielsweise zur Freisetzung eines Aktionspotentials.

Aktionspotenziale kennzeichnen sich durch ihren stets nahezu identischen zeitlichen Verlauf. Die Membranspannung depolarisiert zunächst für eine sehr kurze Dauer von weniger als einer Millisekunde extrem stark, danach hyperpolarisiert sie und ist für einen Zeitraum blockiert, in dem keine weiteren Aktionspotenziale auftreten können.

Das Verhalten einer auditiven Nervenfaser i lässt sich durch die Zeitpunkte gefeuerter Spikes $T = \{t_i^k \mid 1 \leq k \leq n_i\} = \{t \mid u_i(t) = v\}$ beschreiben, wobei $u_i(t)$ die Spannung der postsynaptischen Membran i und v der zur AP-Auslösung nötige Schwellenwert sind. Im allgemeinen wird v = v(t) als dynamisch angenommen, da Neuronen unmittelbar nach Emission eines Spikes eine kurze Weile blockiert sind. Diese Spikeunterdrückung wird in der Regel modelliert mit Hilfe einer kurzen absoluten Refraktärzeit $\tau$ abs von in etwa 0.8 ms gefolgt von einem anschließenden exponentiellen Abklingen mit einer Zeitkonstante $\tau$ von etwa 0.25 ms, so dass die Schwellwertspannung modelliert werden kann durch

$$v(t) = v_0 + \sum \eta o e^{\left(\frac{-(t-t_i^{(k)}-\tau_{abs})}{\tau}\right)} * I_{[\tau_{abs},\infty]}\left(t - t_i^{(k)}\right),$$

wenn das Neuron sich nicht im absolut refraktären Zustand befindet und v(t) = $\infty$, falls $t - t_i^{(k)} \in [0, \tau_{abs}]$ für ein k $\in$ {1,...,ni}, mit I(.) als Indikatorfunktion. Aus Recheneffizenzgründen wird die Summe meist auf den Einfluss der letzten 1 oder 2 Spikes vereinfacht.

Mit anderen Worten, ein Aktionspotenzial wird ausgelöst, wenn das post-synaptische Potenzial den vorgegebenen Schwellenwert überschreitet. Dieser Schwellenwert kann zeitlich variieren. Im Übrigen wird darauf hingewiesen, dass bei einer Erzeugung eines Aktionspotenzials bevorzugterweise sowohl eine absolute Refraktärzeit, d.h. eine Totzeit, während der eine Auslösung eines weiteren Aktionspotenzials nicht mehr möglich ist, als auch eine relative Refraktärzeit, während der eine Schwelle für eine Auslösung eines Aktionspotenzials höher ist als in einem Ruhezustand, berücksichtigt werden können. Die absolute Refraktärzeit kann dabei beispielsweise durch einen unendlich hohen Schwellwert v modelliert werden, aber auch anderweitig einbezogen werden. Die relative Refraktärzeit wird bevorzugt durch einen zeitlich variablen Schwellenwert v für die Auslösung eines Aktionspotenzials beschrieben.

Somit kann gewährleistet werden, dass Totzeiten der Nervenfasern bei einer Erzeugung von Aktionspotenzialen bzw. einer Erzeugung des Nervenaktivitätsmusters, das Aktionspotenziale auf einer Mehrzahl von Nervenfasern beschreibt, berücksichtigt werden. Dadurch kann die Informationsmenge des Nervenaktivitätsmusters verringert werden, was eine weitere Verarbeitung des Nervenaktivitätsmusters und eine Speicherung des Nervenaktivitätsmusters erleichtert. Im Übringen kann durch eine beschriebene Anordnung auch eine spontane Aktivität der Nervenfasern mit berücksichtigt werden.

Weiterhin sei darauf hingewiesen, dass während der absoluten Refraktärzeit auch das zeitlich überlappte Auftreten einer Mehrzahl von Neurotransmitter-Vesikeln nicht zu einer Auslösung eines Aktionspotenzials führt. In der relativen Refraktärzeit hingegen löst zwar das Auftreten eines einzigen Neurotransmitter-Vesikels noch kein Aktionspotenzial aus, während hingegen ein überlappendes Auftreten mehrerer Neurotransmitter-Versikel in der relativen Refraktärzeit durchaus in einer Auslösung eines Aktionspotenzials resultieren kann.

Gemäß dem anhand der Fig. 1 bis 6 beschriebenen Verfahren kann also eine besonders aussagekräftige Analysedarstellung eines Audiosignals erzeugt werden, die ein Nervenaktivitätsmuster umfasst. In der erfindungsgemäßen Weise wird also das Nervenaktivitätsmuster basierend auf einem Neurotransmitter-Vesikel-Auftreten berechnet, wobei die stochastische Natur des Neurotransmitter-Vesikel-Auftretens berücksichtigt werden kann. Im Übrigen können auch bei der Bestimmung von Aktionspotenzialen auf den Nervenfasern alle relevanten Effekte (wie beispielsweise die Diffusion von Neurotransmittern und die Potenzialabhängigkeit bei der Auslösung eines Aktionspotenzials auf einer Nervenfaser) berücksichtigt werden. Dadurch weist das in der erfindungsgemäßen Weise berechnete Nervenaktivitätsmuster eine besonders hohe zeitliche Genauigkeit auf, die für eine aussagekräftige Analyse benötigt wird. Anhand eines erfindungsgemäß bestimmten Nervenaktivitätsmuster kann nämlich beispielsweise auch eine Phasenbeziehung zwischen den Aktionspotenzialen benachbarter Nervenzellen ausgewertet werden, die herkömmlicherweise aufgrund einer zu unpräzisen Modellierung nur wenig aussagekräftig ist.

Ferner wird darauf hingewiesen, dass es vorteilhaft ist, Neurotransmitter-Vesikel 1-zu-1 in elektrische Impulse einer Höhe von 1mV umzusetzen. Eine berechnete Freisetzung eines Neurotransmitter-Vesikels kann beispielsweise verwendet werden, um in einer Cochlea-Implantat-Ansteuervorrichtung einen Rechteck-Impuls zu erzeugen die entstehenden elektrischen Impulse dann einem Cochlea-Implantat zuzuführen. Durch die Impulse können dann Hörnervenfasern getriggert werden. Hierbei werden beispielsweise 251 Nervensignale für 251 Hörnerven auf die in einem Cochlea-Implantat typischerweise verfügbaren 22 Kanäle skaliert.

Weiterhin wird es bevorzugt, das Nervenaktivitätsmuster weiter zu verarbeiten, um eine noch vorteilhaftere Analysedarstellung des Audiosignals zu erhalten. Die Berechnung des Nervenaktivitätsmusters kann hierbei vorteilhafter Weise nach dem oben beschriebenen Verfahren erfolgen, jedoch sind auch andere Verfahren, die ein Nervenaktivitätsmuster liefern, für die Gewinnung einer verbesserten Analysedarstellung verwendbar.

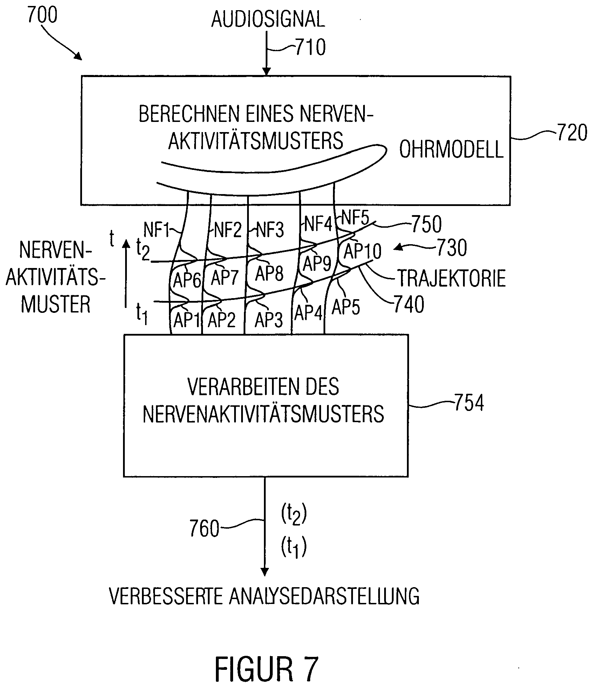

Fig. 7 zeigt ein Flussdiagramm eines erfindungsgemäßen Verfahrens zur Berechnung einer verbesserten Analysedarstellung eines Audiosignals gemäß einem weiteren Ausführungsbeispiel der vorliegenden Erfindung. Das Flussdiagramm der Fig. 7 ist in seiner Gesamtheit mit 700 bezeichnet. Dabei wird basierend auf einem Audiosignal 710 in einem ersten Schritt 720 ein Nervenaktivitätsmuster 730 berechnet. Bei der Ermittlung des Nervenaktivitätsmusters 730 in dem ersten Schritt 720 kann beispielsweise ein Ohrmodell verwendet werden, wie dies schon oben ausführlich erläutert wurde. Ergebnis der Berechnung des Nervenaktivitätsmusters 730 sind somit zeitliche Verläufe von Aktionspotenzialen auf einer Mehrzahl von Nervenfasern NF1, NF2, NF3, NF4, NF5.

Es wird hierbei darauf hingewiesen, dass das Nervenaktivitätsmuster 730 als eine Antwort auf das Audiosignal 710 charakteristische Trajektorien 740, 750 aufweist. Mit anderen Worten ausgedrückt: tritt ein akustisches Ereignis in dem Audiosignal 710 auf, so resultiert dieses akustische Ereignis in einer Serie von Aktionspotenzialen Ap1, AP2, AP3, AP4, AP5, die mit leichtem zeitlichem Versatz auf einer Mehrzahl von Nervenfasern NF1, NF2, NF3, NF4, NF5 auftreten. Eine Trajektorie 740, 750 umfasst somit eine Mehrzahl von Aktionspotenzialen AP1, AP2, AP3, AP4, AP5 bzw. Spitzen auf mehreren verschiedenen Nervenfasern NF1, NF2, NF3, NF4, NF5 ("pulse spiking trains").

Eine Trajektorie 740, 750 ist u.a. dadurch gekennzeichnet, dass sie in einer zweidimensionalen Darstellung des Nervenaktivitätsmusters 730 über der Zeit als eine Linie erkennbar ist. Die Trajektorie 740, 750 kann hierbei entweder gerade oder gekrümmt sein, wobei verschiedene Arten der Krümmung (z.B. parabolisch oder hyperbelförmig) auftreten können. Im Übrigen ist zu erwähnen, dass es in manchen Fällen auch möglich ist, eine Trajektorie 740, 750 durch einen Ausschnitt aus einer Sinusfunktion anzunähern. Ferner ist festzuhalten, dass eine Trajektorie 740, 750 definitionsgemäß eine Mindestlänge aufweist. Eine Trajektorie 740, 750 liegt also nur dann vor, wenn Aktionspotenziale AP1, AP2, AP3, AP4, AP5 auf einer Mindestanzahl von Nervenfasern auftreten. Dabei bilden dann die Aktionspotentiale in einer zweidimensionalen Darstellung des Nervenaktivitätsmusters als Funktion der Zeit ein Liniensegment.

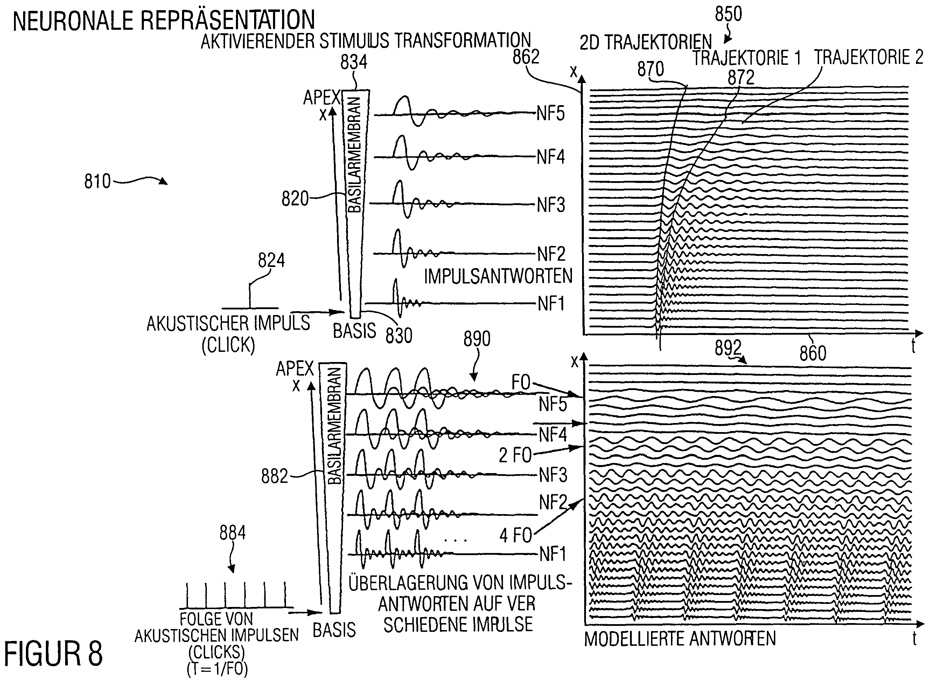

Im Übrigen ist festzuhalten, dass eine zweidimensionale Darstellung eines Nervenaktivitätsmusters 730 als Funktion der Zeit hier eine Darstellung beschreibt, in der die Aktionspotenziale auf mehreren Nervenfasern parallel als Funktion der Zeit angetragen sind, wobei die Zeit in Richtung einer Abszisse angetragen ist, und wobei Verläufe der Aktionspotenziale auf mehreren Nervenfasern durch Linienzüge, die im Wesentlichen parallel zu der Abszisse verlaufen, angetragen sind (vgl. auch Fig. 8). Eine Trajektorie 740, 750 liegt somit vor, wenn zusammengehörige Maxima oder Minima der Aktionspotenziale AP1, AP2, AP3, AP4, AP5 in der zweidimensionalen Darstellung des Nervenaktivitätsmusters 730 durch eine gerade oder gebogene Linie (die bevorzugterweise glatt bzw. knickfrei ist) verbunden werden können. Eine Trajektorie kann somit als eine Verbindungslinie von zusammengehörigen Maxima bzw. Minima von Aktionspotenzialen benachbarter Nervenfasern angesehen werden.

Das in dem Flussdiagramm 700 gezeigte erfindungsgemäße Verfahren umfasst in einem zweiten Schritt 754 das Verarbeiten des Nervenaktivitätsmusters, das in dem ersten Schritt 720 gebildet wird. Dabei wird das Nervenaktivitätsmuster 730 bevorzugt in einer zweidimensionalen Darstellung als Funktion der Zeit und als Funktion der Position der Nervenfasern betrachtet. In dem zweiten Schritt 754 wird somit das Nervenaktivitätsmuster 730 verarbeitet, um als Analysedarstellung eine Folge von Zeitinformationen t1, t2 zu erhalten, die die zeitliche Lage von aufeinander folgenden Trajektorien 740, 750 beschreiben. Dabei wird davon ausgegangen, dass eine Trajektorie 740, 750 Aktivitätsimpulse AP1, AP2, AP3, AP4, AP5 auf verschiedenen Nervenfasern NF1, NF2, NF3, NF4, NF5 aufgrund des gleichen Ereignisses in dem Audiosignal umfasst bzw. verbindet. Die ermittelten Zeitinformationen t1, t2 beschreiben hierbei den Zeitpunkt des Auftretens der Trajektorien 740, 750. Es wird ferner darauf hingewiesen, dass eine Trajektorie 740, 750 nur dann erkannt wird, wenn eine Mindestanzahl an Nervenfasern NF1, NF2, NF3, NF4, NF5 ein Aktionspotenzial AP1, AP2, AP3, AP4, AP5 aufweisen, wodurch verhindert wird, dass einzelne Aktivitätspotenziale in dem Nervenaktivitätsmuster fälschlich als Trajektorie identifiziert bzw. erfasst werden.

Ein akustisches Ereignis, das eine Trajektorie mit sich bringt, kann beispielsweise ein Beginn eines Lauts sein. Auch Vokale erzeugen mindestens eine charakteristische Trajektorie, die bei dem erfindungsgemäßen Verfahren erkannt werden kann, und der daraufhin eine Zeitinformation zugeordnet werden kann. Üblicherweise umfasst allerdings ein Laut sogar mehrere Trajektorien, wobei bevorzugterweise für jede der aufeinander folgenden Trajektorien eine zugehörige Zeitinformation erzeugt werden kann. Die Zeitinformation kann dabei beispielsweise einen Anfangszeitpunkt der Trajektorie kennzeichnen, also einen Zeitpunkt, an dem die erste zu der Trajektorie 740, 750 gehörige Nervenfaser NF1, NF2, NF3, NF4, NF5 ein Aktionspotenzial AP1, AP2, AP3, AP4, AP5 aufweist.

Die Verarbeitung des Nervenaktivitätsmusters 730 kann beispielsweise so ausgelegt werden, dass solche Muster in dem Nervenaktivitätsmuster als Trajektorie erkannt werden, die eine Wanderwelle auf einer Basilarmembran eines Ohrmodells zugeordnet sind. Mit anderen Worten, eine Trajektorie kann dann erkannt werden, wenn auf den einzelnen Nervenfasern ein Anregungsmuster anliegt, das auf einer Ausbreitung einer Wanderwelle basiert. Eine Wanderwelle löst dabei typischerweise nacheinander und mit einem geringen aber nicht verschwindenden zeitlichen Versatz Aktionspotenziale auf der Mehrzahl von Nervenfasern aus.

Weiterhin ist bei obigen Überlegungen zu berücksichtigen, dass üblicherweise Nervenfasern, die gemäß des Ohrmodells bevorzugt auf niedrige Frequenzen ansprechen, erst später aktiviert werden bzw. ein Aktionspotenzial aufweisen, als Nervenfasern, die bevorzugt hohe Frequenzen detektieren. Dies liegt darin begründet, dass Hörnerven, die auf hohe Frequenzen ansprechen, an dem Anfang der Cochlea bzw. der Basilarmembran angeordnet sind, während hingegen Hörnerven, die niedrige Frequenzen erfassen, an dem Ende der Cochlea angeordnet sind.

Mit der Ausbreitung eines Schallereignisses über die Cochlea bzw. über die Basilarmembran ergibt sich hierbei eine frequenzabhängige Verzögerung, sodass hohe Frequenzanteile früher als tiefe Frequenzanteile ein Aktivitätspotential auf den jeweils zugeordneten Nervenfasern erzeugen.

So zeigt auch die Darstellung der Figur 7 Aktionspotentiale AP1, AP2, AP3, AP4, AP5 auf eine Mehrzahl von Nervenfasern NF 1, NF 2, NF 3, NF 4, NF 5. Es wird hierbei davon ausgegangen, dass eine erste Nervenfaser NF 1 einer hohen Frequenz zugeordnet ist (z. B. 20 kHz), während eine fünfte Nervenfaser (NF 5) einer niedrigeren Frequenz (z. B. 20 Hz) zugeordnet ist. Eine dazwischenliegende zweite Nervenfaser NF 2, eine dritte Nervenfaser NF 3 und eine vierte Nervenfaser NF 4 sind dazwischenliegende Frequenzen zugeordnet.

Zum besseren Verständnis wird hier auch auf eine Zeitachse t hingewiesen. Es ist erkennbar, dass das Aktionspotential AP1 auf der ersten Nervenfaser NF 1 früher auftritt als Aktionspotentiale auf den übrigen Nervenfasern NF 2, NF 3, NF 4 und NF 5. Die zusammengehörigen Aktionspotentiale auf den fünf Nervenfasern bilden somit bei einer zeitlichen Betrachtung eine erste Trajektorie 740, der ein Anfangszeitpunkt t1 zugeordnet werden kann. Ein später folgendes zweites akustisches Ereignis erzeugt wiederum Aktionspotentiale AP6, AP7, AP8, AP9, AP10 auf den Nervenfasern NF1, NF2, NF3, NF4, NF5 , die die zweite Trajektorie 750 bilden. Der zweite Trajektorie 750 kann dann ein zweiter Zeitpunkt t2 zugeordnet werden.

Aufgabe der Verarbeitung des Nervenaktivitätsmusters in dem zweiten Schritt 730 ist es nunmehr, die ersten Trajektorie 740 und die zweite Trajektorie750 zu erkennen und entsprechende Zeitinformationen (t1), (t2), die den Trajektorien 740, 750 zugeordnet sind, zu liefern. Die Zeitinformationen (t1) und (t2) bilden somit eine verbesserte Analysedarstellung des Audiosignals 710, die eine noch vorteilhaftere Verarbeitung als das Nervenaktivitätsmuster selbst ermöglicht. Die verbesserte Analysedarstellung 760 kann insbesondere auch zur Zwecke einer Spracherkennung eingesetzt werden. Daneben eignet sich die verbesserte Analysedarstellung 760 auch sehr gut für eine Rhythmuserkennung eines Signals.

Fig. 8 zeigt zum besseren Verständnis eine Darstellung von beispielhaften Nervenaktivitätsmustern. Eine erste graphische Darstellung 810 zeigt, wie eine Basilarmembran 820 durch einen akustischen Impuls 824 (CLICK) angeregt wird. Der akustische Impuls 824 wird über das ovale Fenster der Cochlea eingekoppelt und erreicht somit zuerst eine Basis 830 der Basilarmembran 820. Der akustische Impuls breitet sich dann über die Basilarmembran 820 zu deren Apex 834 aus. Entlang der Basilarmembran sind beispielsweise fünf (Hör-)Nerven angeordnet, wobei eine grafische Darstellung 840 Pulsantworten der Basilarmembran 820 bei einer Anregung durch den akustischen Impuls 824 an verschiedenen Orten der Basilarmembran zeigt. Es ist hierbei ersichtlich, dass in der Nähe der Basis 830 der Basilarmembran 820 eine kurze Impulsantwort auftritt, die im wesentlichen hohe Frequenzanteile umfasst, während hingegen in der Nähe des Apex 834 der Basilarmembran 820 eine lange Impulsantwort auftritt, die im wesentlichen niedrige Frequenzen umfasst.

Eine weitere grafische Darstellung 850 zeigt Impulsantworten für eine Vielzahl von Orten entlang der Basilarmembran. Eine Abszisse 860 beschreibt hierbei die Zeit, während eine Ordinate 862 einen Ort entlang der Basilarmembran 820 kennzeichnet. Aus der grafischen Darstellung 850 ist ersichtlich, dass die Impulsantworten an verschieden Orten entlang der Basilarmembran 820 bei einer Anregung durch den akustischen Impuls 824 eine Mehrzahl von Trajektorien aufweisen, von denen zwei Trajektorien 870, 872 beispielhaft in der grafischen Darstellung 850 eingezeichnet sind. Trajektorien, die in der grafischen Darstellung 850 der Impulsantwort der Basilarmembran gezeigt sind, können im übrigen in ähnlicher weise auch in einem Nervenaktivitätsmuster identifiziert werden, das hier nicht explizit gezeigt ist.

Es wird im übrigen auch noch darauf hingewiesen, dass die in der graphischen Darstellung 850 gezeigten Trajektorien 870, 872 ein unterschiedliches Krümmungsverhalten aufweisen. Das unterschiedliche Krümmungsverhalten resultiert aus der Tatsache, dass an verschiedenen Orten der Basilarmembran 820 verschiedene Frequenzen dominant sind (beispielsweise an der Basis der Basilarmembran hohe Frequenzen und an dem Apex der Basilarmembran niedrige Frequenzen).

Eine weitere grafische Darstellung 880 zeigt analog zu der grafischen Darstellung 810 eine Anregung einer Basilarmembran 882 durch eine Folge von akustischen Impulsen (CLICKS) die mit 884 bezeichnet ist. Entsprechend zeigt eine grafische Darstellung 890 eine Überlagerung von Impulsantworten auf verschiedene akustische Impulse. Eine grafische Darstellung 892 zeigt wiederum die entsprechende Impulsantwort für eine große Anzahl von Orten x auf der Basilarmembran 881.

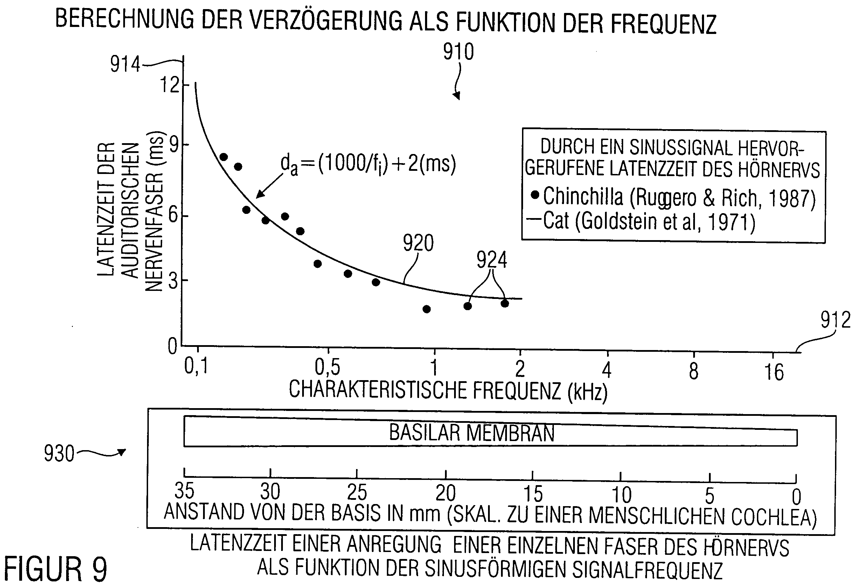

Zur weiteren Verdeutlichung zeigt die Figur 9 eine grafische Darstellung einer Verzögerung bei einer Ausbreitung von Signalen verschiedener Frequenz auf der Basilarmembran. Hierbei beschreibt eine erste grafische Darstellung 910 eine Latenzzeit von auditorischen Nervenfasern als Funktion einer charakteristischen Frequenz.

Eine Frequenz ist hierbei an einer Abszesse 912 in einem Bereich zwischen 0,1 kHz und 16 kHz angetragen. Eine Ordinate von 914 beschreibt eine Latenzzeit der auditorischen Nervenfaser in einem Bereich zwischen 0 und 12 Millisekunden. Eine Kurve 920 beschreibt einen Verlauf einer durch ein Sinussignal hervorgerufenen Latenzzeit des Hörnervs über der Frequenz für eine Katze. Messpunkte 924 beschreiben einen ähnlichen Verlauf für einen Chinchilla. Es wurde herausgefunden, dass die Latenzzeit auf auditorischen Nervenfasern als Funktion einer charakteristischen Frequenz $f_i$ für die betreffende Nervenfaser durch die folgende Gleichung beschrieben werden kann:

$$d_a = (1000/f_i) + 2ms.$$

Der entsprechende Zusammenhang ist im Detail beispielsweise in dem Artikel "A Space-Time Theory of Pitch and Timbre Based on Cortical Expansion of the Cochelar Travelling Wave Delay" von S. Greenberg, D. Poeppel und T. Roberts, vorgestellt auf dem XIth International Symposium on Hearing, Grantham, ausführlich erläutert. Für Einzelheiten wird daher auf den entsprechenden Artikel verwiesen.

Es wird hierbei ferner darauf hingewiesen, dass eine zweite grafische Darstellung 930 einen Abstand von zu einer charakteristischen Frequenz gehörigen Hörzellen von einer Basis einer menschlichen Cochlea zeigt. Hieraus wird wiederum deutlich, dass zu hohen Frequenzen gehörige Hörzellen nahe bei der Basis der menschlichen Cochlea angeordnet sind, während zu niedrigen Frequenzen gehörige Hörzellen entfernt von der Basis der menschlichen Cochlea angeordnet sind.

Die vorliegende Darstellung bestehend aus der ersten grafischen Darstellung 910 und der zweiten grafischen Darstellung 930 zeigt allerdings auch, dass eine Latenzzeit des auditorischen Nervs für tiefe Frequenzen (unter ca. 0,5 kHz) stark ansteigt. Daraus kann gefolgert werden, dass eine Ausbreitungszeit in der Nähe des Endes der Basilarmembran (also entfernt von der Basis der Cochlea) deutlich abnimmt.

Die beschriebene Latenzzeit der auditorischen Nervenfasern resultiert, wie auch schon anhand von Figur 8 beschrieben, in einer Krümmung von Trajektorien in dem Nervenaktivitätsmuster, das Aktionspotenziale auf einer Mehrzahl von Nervenfasern, die mit Hörzellen gekoppelt sind, beschreibt.

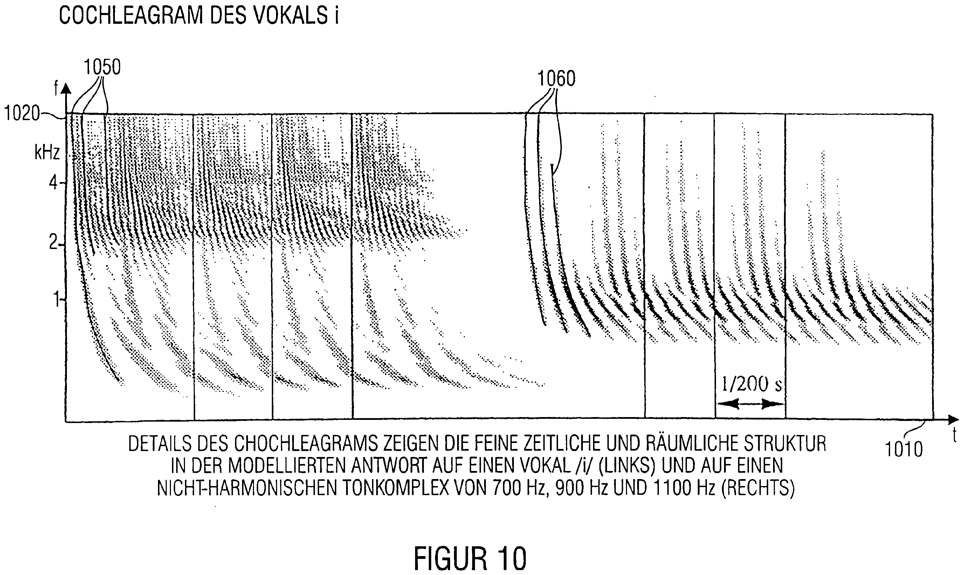

Zur weiteren Verdeutlichung zeigt die Figur 10 eine grafische Darstellung eines Cochleograms für einen Vokal "i" und für einen nicht-harmonischen Tonkomplex von 700 Hz, 900 Hz und 1100 Hz. Eine Abszisse 1010 beschreibt hierbei die Zeit, während eine Ordinate 1020 eine Frequenz beschreibt. In dem entsprechenden Cochleogram des Vokals "i" sind wiederum deutlich Trajektorien erkennbar, von denen einige ausgewählte Trajektorien gekennzeichnet und mit 1050 bezeichnet sind. In ähnlicher Weise zeigt auch das Cochleogram des nicht-harmonischen Tonkomplexes Trajektorien, die mit 1060 bezeichnet sind.

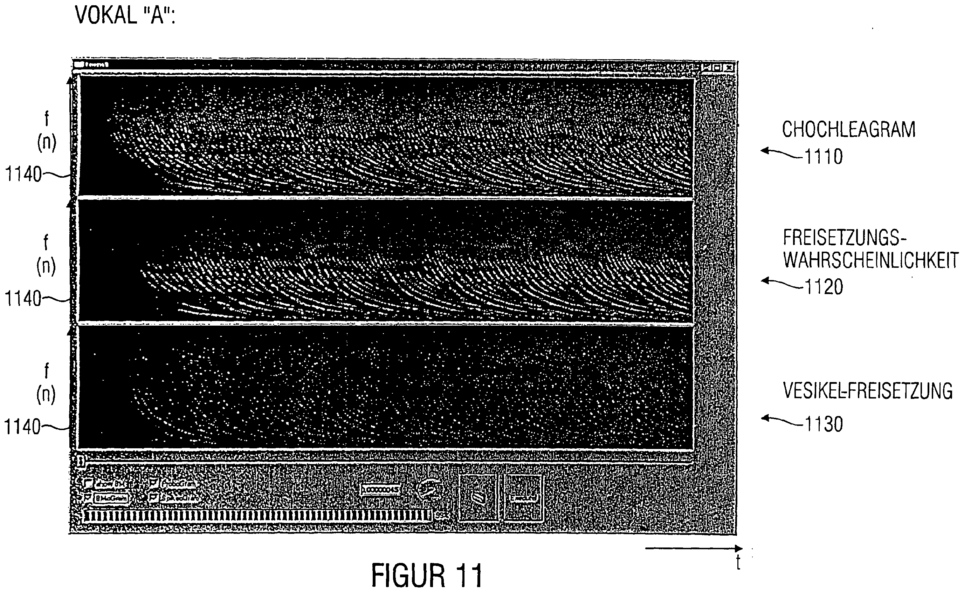

Figur 11 zeigt eine weitere grafische Darstellung eines Cochleograms sowie eine Transmitter-Freisetzungs-

18

Wahrscheinlichkeit und eine Transmitter-Vesikel-Freisetzung für einen Vokal "A". Das Cochleogram ist hierbei in einer ersten grafischen Darstellung 1110 gezeigt. Eine zweite grafische Darstellung 1120 beschreibt eine auf dem Cochleogram basierende Transmitter-Freisetzungs-Wahrscheinlichkeit. Es wird hierbei darauf hingewiesen, dass das Cochleogram eine Anregung der Basilarmembran über der Zeit und Frequenz beschreibt. Basierend darauf, kann, wie schon oben beschrieben, eine Freisetzungs-Wahrscheinlichkeit für Neurotransmitter-Vesikel durch Analyse der mechanischen, chemischen und elektrischen Abläufe in einer Hörzelle berechnet werden. Die Freisetzungs-Wahrscheinlichkeit wurde beispielsweise als k(t) bezeichnet. Basierend auf der Freisetzungswahrscheinlichkeit k(t) kann dann eine Neurotransmitter-Vesikel-Freisetzung durch eine stochastische Auswertung berechnet werden. Ein Beispiel für eine entstehende Neurotransmitter-Vesikel-Freisetzung ist in der dritten grafischen Darstellung 1130 gezeigt. Es ist hierbei ersichtlich, dass auch die Vesikel-Freisetzung über Zeit und Frequenz charakteristische Trajektorien aufweist. Diese Trajektorien werden dann durch eine Modellierung des synaptischen Spalts auf Trajektorien von Aktionspotenzialen (also Trajektorien in dem Nervenaktivitätsmuster) abgebildet.

Es wird hierbei schließlich darauf hingewiesen, dass die an Ordinaten 1140 eingetragene Frequenz jeweils zugehörigen Hörzellen bzw. Nervenfasern (n) zugeordnet werden kann. Somit treten die gezeigten Trajektorien in sehr ähnlicher Form auch in dem Nervenaktivitätsmuster auf.

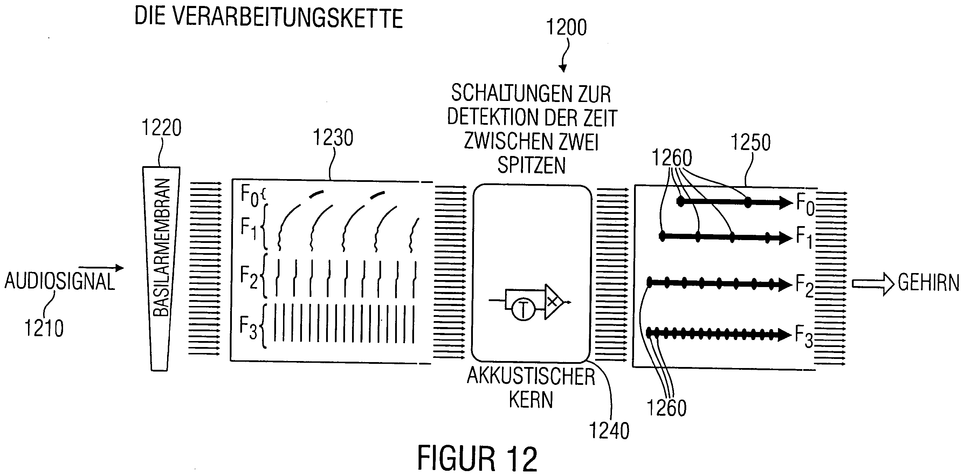

Figur 12 zeigt eine grafische Darstellung einer Verarbeitungskette für eine erfindungsgemäße Analyse eines Audiosignals gemäß dem zweiten Ausführungsbeispiel der vorliegenden Erfindung. Die grafische Darstellung der Figur 12 ist in der Gesamtheit mit 1200 bezeichnet.

Gemäß der Fig. 12 wird aufgrund eines Audiosignals 1210 die Bewegung einer Basilarmembran 1220 an einer Mehrzahl von Punkten (bzw. Orten oder Regionen) berechnet. Ein Anregungsmuster der Basilarmembran eine Abhängigkeit von einer Position entlang der Basilarmembran wird dann entweder direkt einer Einrichtung zur Erkennung von Trajektorien zugeführt, oder es wird basierend auf dem Anregungsmuster der Basilarmembran ein Nervenaktivitätsmuster erzeugt, dass für eine weitere Verarbeitung herangezogen wird. Das Anregungsmuster der Basilarmembran weist, ebenso wie ein daraus abgeleitetes Nervenaktivitätsmuster, bei einer Betrachtung über der Zeit eine Mehrzahl von Trajektorien auf, die charakteristisch für ein Audiosignal sind. Die Trajektorien sind dabei aufgrund der anhand der Figuren 8 und 9 beschriebenen Laufzeitunterschiede zwischen hohen und niedrigen Frequenzen typischerweise gebogen. Im übrigen ist anzumerken, dass die Trajektorien beispielsweise auch unterbrochen sein können. Ferner können in einzelnen Frequenzbereichen bzw. räumlichen Bereichen der Basilarmembran unterschiedliche Anregungsmuster und Trajektorien auftritt. Unterbrochene Trajektorien bzw. Teil-Trajektorien, die nur in einem bestimmten Frequenzbereich angeregt werden, sind beispielsweise für Sprachsignale charakteristisch.

Das gezeigte Nervenaktivitätsmuster 1230 bzw. ein Basilarmembran-Anregungsmuster, das dem Nervenaktivitätsmuster ähnlich ist, wird daraufhin einem akustischen Kern 1240 zugeführt. Der akustische Kern ist ausgelegt, um eine Zeit zwischen zwei Spitzen zu detektieren. Im übrigen wird darauf hingewiesen, dass es die Aufgabe des akustischen Kerns ist, Trajektorien in dem Nervenaktivitätsmuster (bzw. Basilarmembran-Anregungsmuster) zu identifizieren. Der akustische Kern kann ausgelegt sein, um den gesamten Frequenzbereich, also alle Nervenfasern, die das Nervenaktivitätsmuster bilden, oder nur einen ausgewählten Teilbereich zu analysieren. Ferner ist es auch möglich, verschiedene Kombinationen von Teilbereichen sowie den gesamten Frequenzbereich parallel zu analysieren.

Der akustische Kern 1240 ist ausgelegt, um eine Trajektorie zu erkennen, wenn eine Mindestanzahl von betrachteten Nervenfasern ein Aktivitätsmuster 1230 aufweist, was in einer Zeit-Nervenfaser-Nummer-Darstellung einem linienförmigen Verlauf in Form einer typischerweise gekrümmten Linie entspricht. Der akustische Kern 1240 liefert dann als Ausgangssignal eine Folge von Zeitinformationen, die eine zeitliche Lage der Trajektorien beschreiben. Dies kann beispielsweise der Anfangszeitpunkt einer Trajektorie oder auch ein mittlerer Zeitpunkt innerhalb einer Trajektorie sein. Im übrigen wird darauf hingewiesen, dass es bevorzugt wird, eine parallele Auswertung für eine Mehrzahl von Frequenzbändern bzw. für eine Mehrzahl von Gruppen von Nervenfasern durchzuführen, um die Teilinformationen zu einer Mehrzahl von Frequenzbändern für eine weitere Verarbeitung, z. B. eine Spracherkennung, zur Verfügung zu stellen. Ein beispielhaftes Ausgangssignals eines akustischen Kerns 1240 ist in der grafischen Darstellung 1250 gezeigt. Das Ausgangssignal des akustischen Kerns 1240 stellt hierbei ein verbessertes Analysesignal dar, in dem die Datenmenge gegenüber dem Nervenaktivitätsmuster 1230 deutlich verringert ist. Das Ausgangssignal 1250 des akustischen Kerns beschreibt nämlich nur diskrete Zeitpunkte für ein oder mehrere Frequenzbänder, die Trajektorien zugeordnet sind. Die diskreten Zeitpunkte sind hier mit 1260 bezeichnet. Die nach Frequenzbändern aufgeteilten Informationen, die die zeitliche Lage von Trajektorien beschreiben, eignen sich im übrigen besonders gut für eine Spracherkennung.

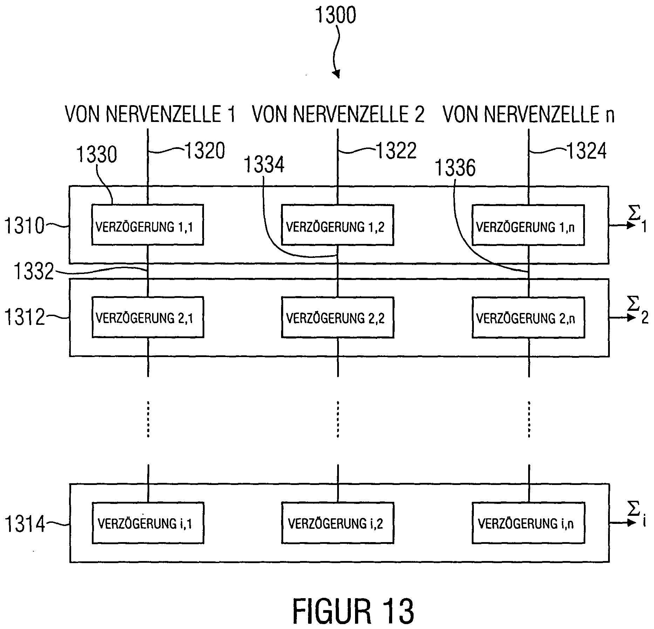

Figur 13 zeigt ein Blockschaltbild einer Vorrichtung zum erfindungsgemäßen Verarbeiten des Nervenaktivitätsmusters. Die in der Figur 13 gezeigten Vorrichtung ist in ihrer Gesamtheit mit 1300 bezeichnet. Die gezeigte Vorrichtung 1300 weist eine Mehrzahl von Stufen 1310, 1312, 1314 auf, wobei die erste Stufe 1310 parallel Signal 1320, 1322, 1324 von Nervenzellen empfängt. Die Signale 1320, 1322, 1324 beschreiben bevorzugt Aktionspotentiale auf Nervenfasern, die mit den entsprechenden Nervenzellen gekoppelt sind, und beschreiben somit das Nervenaktivitätsmuster.

In einer ersten Stufe 1310 wird dann beispielsweise das erste Nervensignal 1320 in einer ersten Verzögerungseinrichtung 1330 einer Verzögerung unterworfen und dann als verzögertes Nervensignal 1332 an eine zweite Stufe 1312 weitergeleitet. In ähnlicher weise wird auch das zweite Nervensignal 1322 in der ersten Stufe 1310 verzögert und als verzögertes Nervensignal an die zweite Stufe 1312 weitergeleitet. In der gleichen Weise werden auch die übrigen Nervensignale in der ersten Stufe 1310 verarbeitet (also beispielsweise auch das n-te Nervensignal 1324).

Die zweite Stufe 1312 ist parallel zu der ersten Stufe 1310 ausgelegt, ermöglicht also wiederum die verzögerte Weiterleitung der verzögerten Nervensignale 1332, 1334, 1336, wodurch zwei Mal verzögerte Nervensignale entstehen. Eine Vorrichtung zum erfindungsgemäßen Verarbeitung des Nervenaktivitätsmusters umfasst nun eine Mehrzahl von hintereinander geschalteten Stufen, die gleich wie die erste Stufe 1310 bzw. die zweite Stufe 1312 aufgebaut sind. Die Nervensignale 1320, 1322, 1324 werden also parallel durch die Mehrzahl von Stufen 1310, 1312, 1314 weitergeleitet, wobei jede Stufe eine einstellbare Verzögerung zu den Nervensignalen hinzufügt.

Weiterhin ist jede der Stufen 1310, 1312, 1314 ausgelegt, um eine Summe der an ihr einlaufenden bzw. der aus ihr auslaufenden Nervensignale (bzw. m-mal verzögerten Nervensignale) zu bilden. Ferner sind die Stufen 1310, 1312, 1314 bevorzugt ausgelegt, um diese Summe mit einem einstellbaren Schwellwert zu vergleichen, um festzustellen, ob zu einem gegebenem Zeitpunkt mindestens eine vorgegebene Anzahl von Nervensignalen bzw. verzögerten Nervensignalen (also einlaufende Nervensignale oder auslaufende Nervensignale) aktiv sind (bzw. ein Aktionspotential aufweisen).

Es wird ferner bevorzugt, dass die Verzögerungen der in den Stufen 1310, 1312, 1314 vorhandenen Verzögerungseinrichtungen unterschiedlich eingestellt sind, sodass beispielsweise ein erstes Nervensignal 1320 bei einem Durchlaufen der Stufen 1310, 1312, 1314 einer anderen Verzögerung unterliegt als das zweite Nervensignal 1322. Verzögerungen können beispielsweise so eingestellt sein, dass sich für die Nervensignale 1320, 1322, 1324 unterschiedliche Gesamtverzögerungen beim Durchlaufen durch die Stufen 1310, 1312, 1314 ergeben (wobei es freilich zulässig ist, das beispielsweise zwei Nervensignale in der gleichen Weise verzögert werden). In anderen Worten, die Einrichtung 1300 ist bevorzugt so ausgelegt, das sich nicht für alle Nervensignale die gleichen Verzögerungen ergeben. Außerdem ist es vorteilhaft, dass bei Vorhandensein von j Stufen 1310, 1312, 1314 mindestens (j-1) Stufen 1310, 1312 so ausgelegt sind, dass die in einer Stufe enthaltenen Verzögerungseinrichtungen für die Mehrzahl von Nervensignalen nicht alle die gleiche Verzögerung aufweisen. Dadurch kann erreicht werden, dass ein in eine erfindungsgemäße Einrichtung 1300 einlaufendes Nervenaktivitätsmuster über der Zeit beim Durchlauf durch die beschriebene Einrichtung zeitlich verzerrt wird, dass also einzelne Nervensignale gegenüber anderen Nervenssignalen zeitlich verschoben werden. Durch die Verzerrung können in einer zeitlichen Darstellung gebogene linienartige Muster, also Trajektorien, in dem Nervenaktivitätsmuster gerade gebogen werden.

Ferner wird darauf hingewiesen, dass durch die Summenbildung innerhalb einer Stufe erkannt werden kann, wenn eine ursprünglich gebogene Trajektorie in dem Nervenaktivitätsmuster zu einer geraden Linie gebogen wurde (die dadurch beschrieben wird, dass eine vorgegebene Anzahl der verzögerten Nervensignale nahezu gleichzeitig bzw. zeitlich überlappend ein Aktionspotential aufweisen).

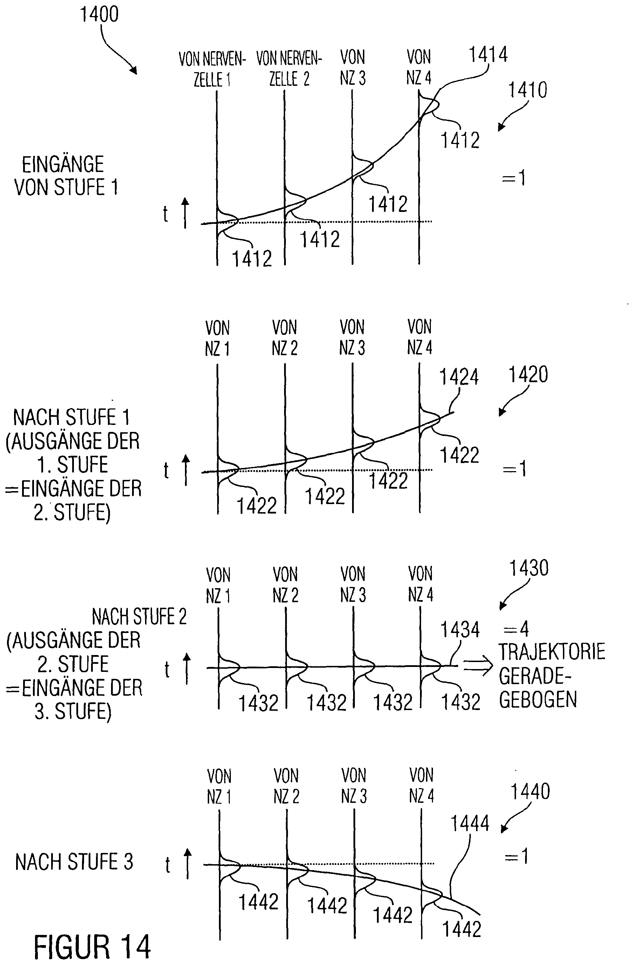

Die Funktionsweise der Einrichtung 1300 soll anhand der Figur 14 veranschaulicht werden. Figur 14 zeigt eine beispielhafte grafische Darstellung der Signale in einer Vorrichtung 1300 zum erfindungsgemäßen Verarbeiten des Nervenaktivitätsmusters. Die grafische Darstellung der Figur 14 ist in ihrer Gesamtheit mit 1400 bezeichnet.

Eine erste graphische Darstellung 1410 beschreibt hierbei ein beispielhaftes Nervenaktivitätsmuster an Eingängen der Vorrichtung 1300. Gezeigt sind hierbei beispielhaft die Signale von vier Nervenzellen (bzw. auf vier Nervenfasern) in einem zeitlichen Verlauf. Im übrigen wird darauf hingewiesen, dass die Aktionspotentiale 1412 eine Trajektorie 1314 bilden. Wie gezeigt, weist die Trajektorie 1414 in der zeitlichen Darstellung eine starke Krümmung auf, da die Aktionspotentiale 1412 von den verschiedenen Nervenfasern an den Eingängen der ersten Stufe 1310 einen deutlichen zeitlichen Versatz aufweisen. Somit liegt in der ersten Stufe 1310 zu einem festen Zeitpunkt nur jeweils ein Aktionspotential vor, sodass ein Schwellwert für eine Summe der an der ersten Stufe anliegenden Aktionspotentiale, der beispielsweise zu zwei gesetzt ist, nicht überschritten wird. Folglich liefert die erste Stufe kein Ausgangssignal an einem Schwellwertausgang.

Eine zweite grafische Darstellung 1420 beschreibt die Verhältnisse an einem Ausgang der ersten Stufe 1310. Hierbei wird davon ausgegangen, dass in der ersten Stufe 1310 das von der erste Nervenzelle NZ 1 gelieferte Nervensignal stärker verzögert wird als das von den anderen Stufen gelieferte Nervensignal. Im übrigen wird davon ausgegangen, dass bei dem gegebenem Beispiel das von der vierten Nervenzelle NZ4 gelieferte Nervensignal am wenigsten verzögert wird, während das Nervensignal von der dritten Nervenzelle NZ3 etwas mehr verzögert wird, und wobei die Verzögerung für Nervensignalen von den Nervensignalen NZ2 und NZ1 immer stärker ansteigen. Allgemein gesprochen werden Signale, die zu Nervenzellen gehören, die auf eine niedrigere Frequenz ansprechen, weniger stark verzögert, als Nervensignale von Nervenzellen, die höhere Frequenzen detektieren.

Die zweite grafische Darstellung zeigt somit wiederum Aktionspotentiale 1424 als Funktion der Zeit, wobei die Aktionspotentiale 1422 eine Trajektorie 1424 bilden. Wie aus der zweiten grafischen Darstellung 1420 ersichtlich ist, ist die Krümmung der Trajektorie 1424 an den Ausgängen der ersten Stufe geringer als eine (zeitlich-örtliche bzw. zeitlich-frequenzmäßige) Krümmung der Trajektorie 1414 an den Eingängen der ersten Stufe. Dies resultiert aus der unterschiedlichen Verzögerung der zu verschiedenen Nervenzellen gehörigen Nervensignale in den Verzögerungseinrichtungen (z.B. 1330) der ersten Stufe. Dadurch wird also eine gekrümmte Trajektorie gleichsam geradegebogen. Wie aus der zweiten grafischen Darstellung 1420 ersichtlich, weist die zweite Trajektorie 1424 allerdings noch eine Restkrümmung auf, so dass die von verschiedenen Nervenzellen bzw. Nervenfasern stammenden Aktionspotentiale 1422 nicht alle gleichzeitig an den Ausgängen der ersten Stufe 1310 bzw. Eingängen der zweiten Stufe 1312 anliegen.

Auch die zweite Stufe 1312 bewirkt eine weitere Verzögerung, wobei wiederum Signale von Nervenzellen, die für niedrige Frequenzen empfindlich sind, weniger verzögert werden als Signale von Nervenzellen, die für höhere Frequenzen empfindlich sind. Eine dritte grafische Darstellung 1430 zeigt die in der zweiten Stufe 1312 nochmals verzögerten Nervensignale an Ausgängen der zweiten Stufe. Es ist aus der dritten graphischen Darstellung 1430 ersichtlich, dass bei dem vorliegenden Beispiel die Nervensignale an den Ausgängen der zweiten Stufe jeweils so verzögert sind, dass Aktionspotentiale 1432 von mehreren Nervenzellen an den Ausgängen der zweiten Stufe gleichzeitig anliegen. In anderen Worten, eine Trajektorie 1434, die durch die Aktionspotentiale 1432 beschrieben wird, ist zumindest näherungsweise gerade gebogen. Die Aktionspotentiale 1432 treten also gleichzeitig bzw. näherungsweise gleichzeitig (zumindest aber zeitlich überlappend) auf, sodass das gleichzeitige Auftreten durch eine Summation der an den Ausgängen der zweiten Stufe (bzw. Eingängen der dritten Stufe) anliegenden Signale einen deutlichen Peak aufweist, der groß genug ist, um einen vorgegebenen Schwellenwert (z. B. zwei oder drei) zu überschreiten.

In anderen Worten, es kann durch eine geeignete Summiereinrichtung (oder eine andere geeignete Einrichtung) erkannt werden, wann eine gekrümmte Trajektorie gerade gebogen wurde. Die entsprechende Information ermöglicht einen Rückschluss sowohl auf den Anfangszeitpunkt der Trajektorie als auch auf die Form der Trajektorie. Es kann nämlich festgestellt werden, nach Durchlaufen von wie vielen Stufe eine Trajektorie gerade gebogen wurde. Dadurch kann bei Kenntnis der Verzögerungen für die einzelnen Nervensignale in den Stufen der Einrichtung 1300 auch auf eine ursprüngliche Form der Trajektorie zurückgeschlossen werden. Ferner ist die Durchlaufzeit für die Stufen bevorzugterweise bekannt, sodass auch der Zeitpunkt, zudem eine Trajektorie in die Einrichtung 1300 eingelaufen ist, bestimmt werden kann. Somit kann die Analysedarstellung sowohl charakteristische Zeitinformationen der Trajektorien als auch Informationen über Form bzw. Krümmung der Trajektorien umfassen.

Die zusätzliche Information über die Form der Trajektorien kann dann bei einer Weiterverarbeitung der Analysedarstellung vorteilhaft ausgenutzt werden, um z. B. eine Spracherkennung zu erleichtern oder eine Erkennungsqualität bei der Spracherkennung zu verbessern.

Im übrigen wird noch darauf hingewiesen, dass eine vierte grafische Darstellung 1440 zur Verbesserung des Verständnisses noch Ausgangssignale an Ausgängen einer dritten Stufe zeigt. Aktionspotentiale 1442 beschreiben eine Trajektorie 1444, die allerdings durch ein weiteres Verbiegen der Trajektorie wieder gekrümmt ist.

Es wird darauf hingewiesen, dass die Verzögerungen in den Stufen 1310, 1312, 1314 auf verschiedenem Weg erzielt werden können. Die Verzögerungseinrichtungen (z.B. 1330) können beispielsweise getaktet sein, und/oder es kann sich um kontinuierlich oder diskret einstellbare Verzögerungseinrichtungen handeln. Im übrigen ist es auch möglich, dass eine oder mehrere Verzögerungseinrichtungen in einer vorgegebenen Stufe für eine oder mehrere Nervensignale deaktiviert sind, sodass einige Nervensignale durch eine Stufe mit geringst möglicher Verzögerung weitergeleitet werden. Im übrigen ist festzuhalten, dass die Einrichtung 1300 insgesamt als eine analoge oder digitale Schaltung implementiert sein kann.

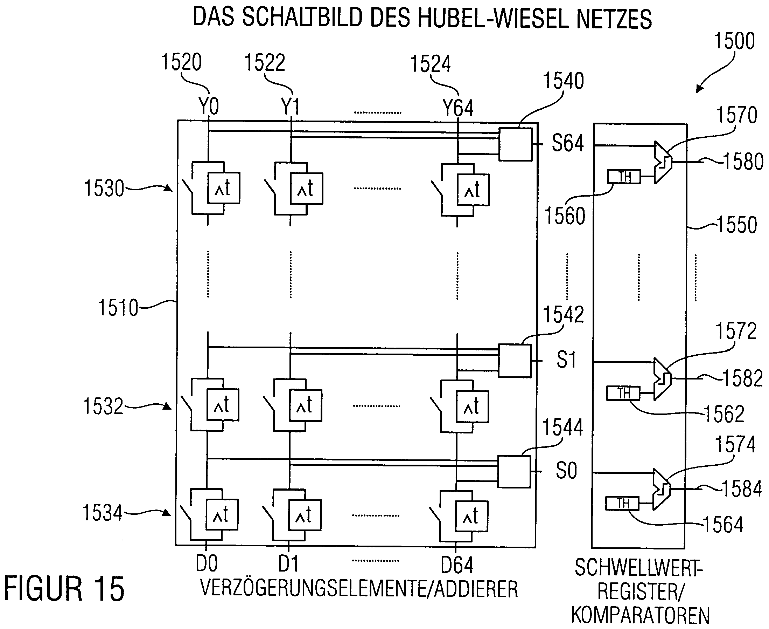

Figur 15 zeigt ein Schaltbild eines beispielhaften Hubel-Wiesel-Netzes zur erfindungsgemäßen Berechnung einer Analysedarstellung eines Audiosignals gemäß dem zweiten Ausführungsbeispiel der vorliegenden Erfindung. Das Schaltbild der Figur 15 ist in seiner Gesamtheit mit 1500 bezeichnet. Erster Schaltungsblock 1510 empfängt Eingangssignale 1520, 1522, 1524, die beispielsweise ein Nervenaktivitätsmuster oder ein Anregungsmuster einer Basilarmembran repräsentieren können. Die Eingangssignale 1520, 1522, 1524 werden dann durch eine Mehrzahl von Stufen 1530, 1532, 1534 geleitet. Ein Eingangssignal 1520 durchläuft somit eine Mehrzahl von Stufen 1530, 1532, 1534, wobei ein Eingangssignal 1520 in einer Stufe 1530, 1532, 1534 entweder eine Verzögerungseinrichtung durchläuft oder direkt zu einer darauffolgenden Stufe weitergeleitet wird. In anderen Worten, die Verzögerungseinrichtungen können auch überbrückt werden.

In anderen Worten, jede Stufe umfasst für jedes Signal eine schaltbare Verzögerungseinrichtung, wobei die Verzögerungseinrichtung in einen Signalpfad, der von einem Eingangssignal durchlaufen wird, eingeschaltet werden kann oder überbrückt werden kann. Signale an den Eingängen jeder Stufe werden abgegriffen und Summierern 1540, 1542, 1544 zugeführt, wobei jeweils die an den Eingängen einer Stufe anliegenden Signale aufsummiert werden. Der erste Schaltungsblock 1510 bildet somit ein Gitter von Verzögerungselementen und Addieren, die in der gezeigten Weise verschaltet sind.

Das Hubel-Wiesel-Netz 1500 weist ferner eine Schwellwerteinrichtung 1550 auf, wobei jeweils ein Wert aus einem Schwellwertregister 1560, 1562, 1564 sowie ein Ausgang eines Summierers 1540, 1543, 1544 einem Komparator 1570, 1572, 1572 zugeführt wird. Ausgangsignale 1580, 1582, 1584 der Komparatoren 1570, 1572, 1574 liefern dabei eine Aussage darüber, ob an den Eingängen einer vorgegebenen Stufe 1530, 1532, 1534 eine Anzahl von Signalen gleichzeitig aktiv sind, wobei eine Mindestanzahl, bei der ein aktives Ausgangssignal 1580, 1582, 1584 ausgegeben wird, durch die Schwellwertregister 1560, 1562, 1564 festgelegt ist. In anderen Worten, durch die Komparatoren 1570, 1572, 1574 kann in Verbindung mit den Summierern 1540, 1542, 1544 und den Schwellwertregistern 1560, 1562, 1564 festgestellt werden, wenn (bzw. nach Durchlaufen wie vieler der Stufen 1530, 1532, 1534) eine Trajektorie, die über die Eingänge 1520, 1522, 1524 des ersten Blocks 1510 eingelesen wurde, gerade gebogen ist.

Die Verzögerungen der einzelnen Stufen 1530, 1532, 1534 können dabei geeignet vorgegeben werden, um eine Erkennung einer möglichst großen Anzahl von Trajektorien (bzw. Trajektorien-Formen) zu ermöglichen.

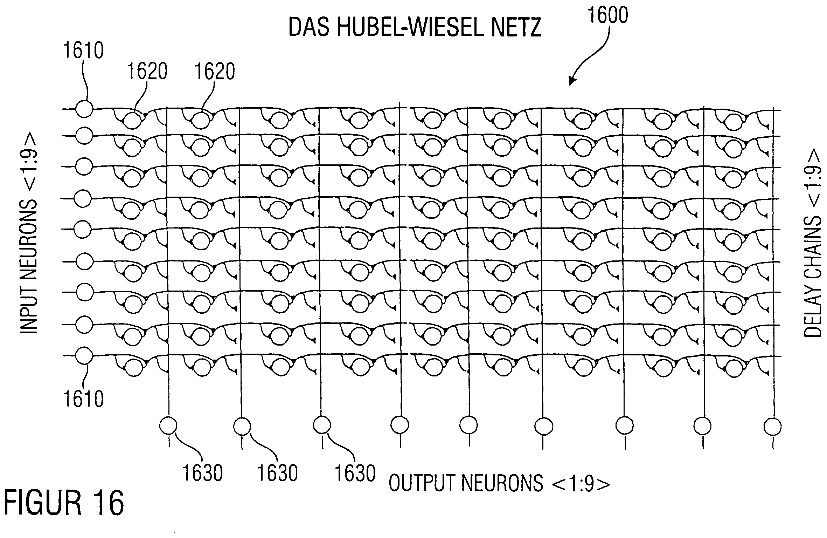

Figur 16 zeigt ein Blockschaltbild eines Hubel-Wiesel-Netzes zur erfindungsgemäßen Berechung einer Analysedarstellung eines Audiosignals gemäß dem zweiten Ausführungsbeispiel der vorliegenden Erfindung. Das gezeigte Hubel-Wiesel-Netzwerk ist in seiner Gesamtheit mit 1600 bezeichnet.

Eingangsneuronen 1610 sind ausgelegt, um ein Nervenaktivitätsmuster oder ein Anregungsmuster einer Basilarmembran eines Ohrmodells in Form von parallel bereitstehenden Zeitsignalen zu empfangen. Das Nervenaktivitätsmuster (bzw. das Anregungsmuster der Basilarmembran) wird dabei unter optionaler Einbeziehung von Verzögerungsneuronen durch mehrere Stufen des neuronalen Netzes weitergeleitet. Hierbei ist anzumerken, dass die Verzögerungsneuronen 1620 auch überbrückt werden können, sodass in einer Stufe keine Verzögerung eines von einem Eingangsneuron 1610 gelieferten Signals stattfindet. Das neuronale Netz weist ferner Ausgangsneuronen 1630 auf. Die Verschaltung des neuronalen Netzes 1600 kann dabei der Figur 16 entnommen werden. Es wird darauf hingewiesen, dass das gezeigte neuronale Netz in der Lage ist, eine gekrümmte Kurve bzw. Trajektorie in einem Nervenaktivitätsmuster (bzw. Basilarmembran-Anregungsmuster), das über die Eingangsneuronen 1610 das neuronale Netz 1600 eingegeben wird, zu erkennen. Das neuronale Netz ist dabei (nach einem Training) in der Lage, sowohl Zeitpunkt als auch Form einer Trajektorie in einem über die Eingangsneuronen 1610 eingegebenen Nervenaktivitätsmuster zu bestimmen, wobei ein aktives Ausgangsneuron diese Informationen beschreibt. Die Informationen über die Form und Zeit einer Trajektorie sind hierbei dadurch codiert, welches Ausgangsneuron wann aktiviert wird.

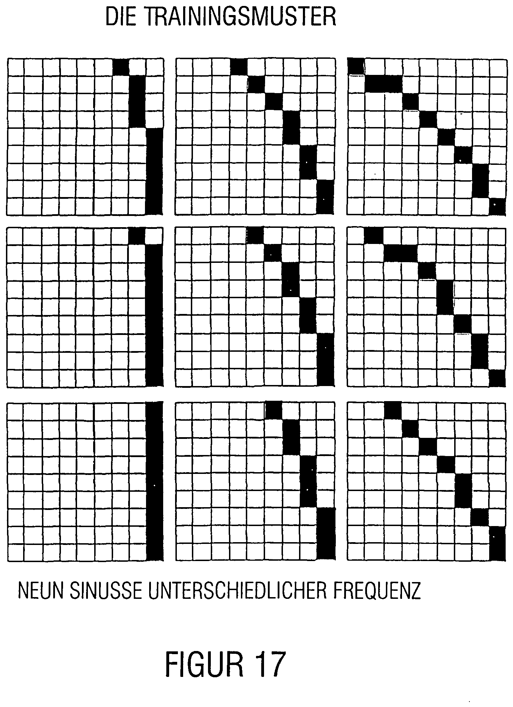

Figur 17 zeigt schließlich eine grafische Darstellung von beispielhaften Trennungsmustern, die zum Trainieren eines neuronalen Netzwerks 1600 verwendet werden können. Nach einem Training ist das neuronale Netzwerk 1600 dann in der Lage, entsprechende gerade oder gekrümmte Verläufe als Trajektorien zu identifizieren.

Somit ist festzuhalten, dass ein Hubel-Wiesel-Netz 1600 sich sehr gut dazu eignet, um Trajektorien in einem Nervenaktivitätsmuster zu erkennen. Hierzu ist lediglich das Nervenaktivitätsmuster an Eingänge 1520, 1522, 1524 des Hubel-Wiesel-Netzes anzulegen. An Ausgängen 1580, 1582, 1584 von Komparatoren 1570, 1572, 1574 stehen dann Signale an, die eine Aussage über Form und zeitliche Lage einer Trajektorie umfassen. Die Ausgangssignale 1580, 1582, 1584 können freilich bei Bedarf noch in eine leichter interpretierbare Form gebracht werden, aus der beispielsweise direkt eine Zeitinformation über eine Trajektorie hervorgeht. Die Zeitinformation bildet dann die vorteilhafte Analysedarstellung.

Es wird im übrigen darauf hingewiesen, dass das in Figur 17 bezeichnete neuronale Netz 1700, das sich sehr gut für eine Erkennung von Trajektorien in einem Nervenaktivitätsmuster eignet, im Detail in dem Artikel "A neural net for 2D-slope and sinusoidal shape detection" von A. Brückmann, S. Klefenz und A. Wünsche (veröffentlicht in dem CIST International Scientific Journal of Computing, ISSN 1727-6209) im Detail beschrieben wurde.

Ferner wird darauf hingewiesen, dass das Verarbeiten des Nervenaktivitätsmusters bevorzugt durch Anwendung einer sogenannten Hough-Transformation erfolgt (Vergleiche US Patent US 3,069,654). Eine Hough-Transformation ist nämlich in der Lage, in effektiver Weise aufeinanderfolgende Trajektorien in eine räumlich-zeitlichen Muster zu erkennen. Damit eignet sich die Hough-Transformation hervorragend zum Extrahieren einer Analysedarstellung aus einem Audiosignal, da im Rahmen der vorliegenden Erfindung erkannt wurde, dass eben Trajektorien in einem Nervenaktivitätsmuster eines Ohrmodells eine charakteristische Information des Audiosignals darstellen, die für eine weitere Analyse vorteilhaft eingesetzt werden kann.

Weiterhin wird darauf hingewiesen, dass auch andere bekannte Verfahren zur Mustererkennung eingesetzt werden können, um Trajektorien in den Nervenaktivitätsmuster zu erkennen. Hierbei können besonders vorteilhaft solche Verfahren eingesetzt werden, die eine Erkennung von gebogenen Linien ermöglichen, da erkannt wurde, dass Trajektorien in den Nervenaktivitätsmuster typischerweise eine hyperbolische Form aufweisen. Hierbei ist zu berücksichtigen, dass das Nervenaktivitätsmuster für eine Mehrzahl von Nerven über der Zeit ein zweidimensionales Muster ergibt, wobei entlang einer ersten Richtung Signale auf mehreren Nervenfasern beispielsweise durch eine Intensitätsverteilung bzw. durch numerische Werte dargestellt werden können, während hingegen in einer zweiten Richtung die zeitliche Entwicklung angetragen ist. Eine typische Darstellungsform für einen zeitlichen Verlauf eines Nervenaktivitätsmusters kann somit beispielsweise ähnlich zu den gezeigten Cochlea-Diagrammen (Cochleogrammen) sein.

Somit ist es möglich, einen beliebigen Mustererkennungsalgorithmus einzusetzen, der in der Lage ist, gekrümmte Linien zu erkennen. Allerdings hat sich gezeigt, dass die Anwendung einer Hough-Transformation besonders vorteilhaft ist, weil eben die Hough-Transformation sich besonders gut für eine Erkennung von gekrümmten Linien eignet. Im übrigen wird darauf hingewiesen, dass bei Durchführen einer Hough-Transformation in einer Anordnung 1600 bzw. 1700 auch bei Vorliegen mehrerer eng benachbarten Trajektorien ein gutes Erkennungsergebnis erzielt werden kann, wenn nur die Schwellenwerte bzw. Ansprechempfindlichkeiten der Ausgangsneuronen (bzw. der Komparatoren) passend eingestellt sind.

Ferner ist es möglich, im Rahmen einer Hough-Transformation (oder einer anderen Mustererkennungsoperation) auch eine Länge einer Trajektorie zu erkennen, sodass neben den Informationen über Zeitpunkt und Form der Trajektorie noch eine dritte Information für eine anschließende Analyse zur Verfügung steht.

Im übrigen wird darauf hingewiesen, dass auch ein zweidimensionaler Mustervergleich über der zeitlichen Darstellung des Nervenaktivitätsmuster durchgeführt werden kann, um eine Folge von Zeitinformationen, die eine zeitliche Lage von aufeinanderfolgenden Trajektorien beschreiben, zu gewinnen.

Ferner wird darauf hingewiesen, dass es vorteilhaft ist, aufgrund einer Analysedarstellung eines Audiosignals eine Mustererkennung durchzuführen. Gemäß der vorliegenden Erfindung existieren zwei besonders vorteilhafte Analysedarstellungen. Es hat sich nämlich gezeigt, dass ein Nervenaktivitätsmuster besonders vorteilhaft für eine Analyse eines Audiosignals eingesetzt werden kann. Ferner eignet sich auch eine Darstellung, die Zeitinformationen über in dem Nervenaktivitätsmuster enthaltene Trajektorien umfasst, besonders gut für eine Analyse des Audiosignals. Sowohl das Nervenaktivitätsmuster als auch die zeitliche Darstellung mit Informationen über in dem Nervenaktivitätsmuster enthaltene Trajektorien werden im folgenden als Audiosignal-Repräsentanten bezeichnet.

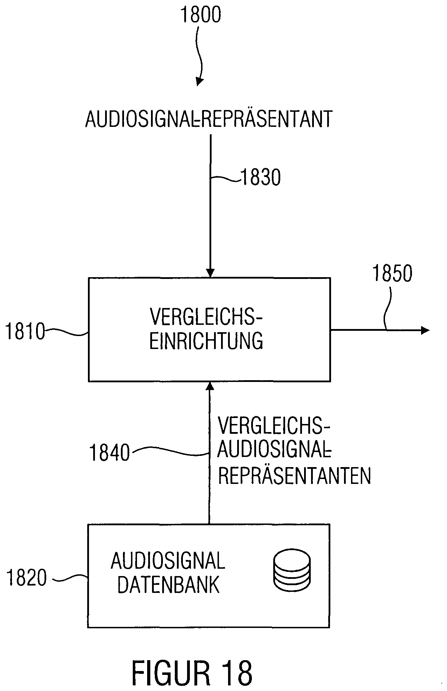

Figur 18 zeigt eine schematische Darstellung einer Einrichtung zur Identifizierung eines Audiosignals. Die in der Figur 18 gezeigte Einrichtung ist in Ihrer Gesamtheit mit 1800 bezeichnet. Die Einrichtung umfasst eine Vergleichseinrichtung 1810, die mit einer Audiosignal-Datenbank 1820 gekoppelt ist. Der Vergleichseinrichtung 1810 wird ferner ein Audiosignal-Repräsentant 1830 zugeführt. Basierend auf dem Audiosignal-Repräsentant 1830 und in der Audiosignal-Datenbank enthaltenen Vergleichs-Audiosignal-Repräsentanten erzeugt die Vergleichseinrichtung 1810 ein Vergleichsergebnis 1850, das eine Aussage darüber umfasst, ob der Audiosignal-Repräsentant 1830 eine Ähnlichkeit zu mindestens einem in der Audiosignal-Datenbank gespeicherten Vergleichs-Audiosignal-Repräsentanten 1840 aufweist. Das Vergleichsergebnis 1850 kann freilich auch eine Aussage darüber umfassen, zu welchen Vergleichs-Audiosignal-Repräsentanten 1840 der Audiosignal-Repräsentant 1830 die größte Ähnlichkeit aufweist.

Die Vergleichseinrichtung 1810 kann eine beliebige Vorrichtung zum Vergleich zweier Audiosignal-Repräsentanten umfassen. Beispielsweise ist es möglich, eine Einrichtung zu verwenden, die basierend auf mathematischen Methoden einen mathematischen Abstand zwischen dem Audiosignal-Repräsentant und dem Vergleichs-Audiosignal-Repräsentant ermitteln kann. Ferner kann auch ein neuronales Netz verwendet werden, um den Audiosignal-Repräsentanten 1830 mit den Vergleichs-Audiosignal-Repräsentanten 1840 zu vergleichen. Ein neuronales Netz kann jeweils beispielsweise mit einer Mehrzahl von Audiosignal-Repräsentanten trainiert werden.

Weiterhin wird darauf hingewiesen, dass die Audiosignal-Datenbank 1820 beispielsweise eine Mehrzahl von Vergleichs-Audiosignal-Repräsentanten umfassen kann, die Musikstücke beschreiben. Weiterhin ist es genau so gut möglich, dass die Audiosignal-Datenbank Vergleichs-Audiosignal-Repräsentanten umfasst, die lediglich einzelne Laute, beispielsweise einen Vokal oder einen Konsonanten beschreiben. Somit kann die gezeigte Einrichtung 1800 zum Identifizieren eines Audiosignals auch für eine Spracherkennung effektiv eingesetzt werden, wobei die gewählten Audiosignal-Repräsentanten sich für eine solche Anwendung besonders gut eignen.

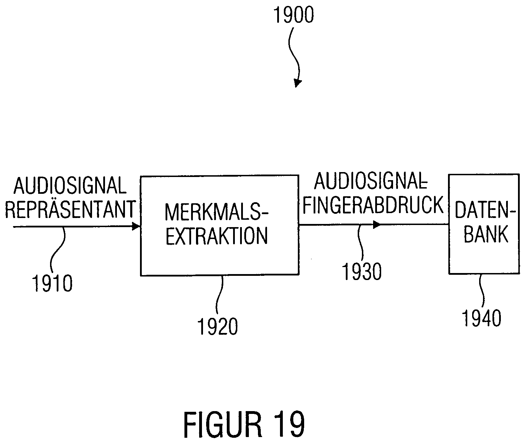

Figur 19 zeigt eine schematische Darstellung einer Einrichtung zum Extrahieren eines Audiosignal-Fingerabdrucks aus dem Audiosignal-Repräsentanten. Die in Figur 19 gezeigte Einrichtung zum Extrahieren des Audiosignal-Fingerabdrucks ist in ihrer Gesamtheit mit 1900 bezeichnet. Hierbei wird ein Audiosignal-Repräsentant 1910 einer Einrichtung 1920 zur Merkmalsextraktion zugeführt. Die Einrichtung 1920 zur Merkmalsextraktion erzeugt basierend auf dem Audiosignal-Repräsentant 1910 einen Audiosignal-Fingerabdruck 1930. Der Audiosignal-Fingerabdruck 1930 wird dann bevorzugt einer Audiosignal-Datenbank 1940 zugeführt.

Die Einrichtung 1920 zur Merkmalsextraktion kann beispielsweise ausgelegt sein, um eine Tonhöhe und/oder ein Rhythmus aus dem Audiosignal-Repräsentant 1910 zu extrahieren. Ist der Audiosignal-Repräsentant ein Nervenaktivitätsmuster, so kann beispielsweise eine Tonhöhe extrahiert werden, indem diejenigen Nervenfasern identifiziert werden, die eine maximale Aktivität in dem Nervenaktivitätsmuster aufweisen. Die Nervenfaser mit höchsten Aktivität in den Nervenaktivitätsmuster ist nämlich ein gutes Maß für eine Tonhöhe, da die Nervenfasern typischerweise eine bevorzugte Frequenz aufweisen. Ferner können Veränderungen (bzw. die entsprechenden Zeitpunkte der Veränderungen) in den Nervenaktivitätsmuster verwendet werden, um einen Rhythmus des Audiosignals zu bestimmen. Eine Erkennung von Veränderungen in den Nervenaktivitätsmuster ist vergleichsweise einfach möglich, indem zwischen aufeinanderfolgenden Momentanwerten des Nervenaktivitätsmusters ein Abstandsmaß, z. B. eine mathematische Norm, gebildet wird. Signifikante Veränderungen können dann erkannt werden, wenn das Abstandsmaß einen vorgegebenen Wert überschreitet.

Auch Verfahren zur Mustererkennung können auf den Audiosignal-Repräsentanten 1910 angewendet werden, um einen Audiosignal-Fingerabdruck zu erzeugen. Der Audiosignal-Fingerabdruck kann so somit beispielsweise eine kombinierte Information über eine Tonhöhe und/oder Rhythmus des Audiosignals umfassen.

Ist der Audiosignal-Repräsentant 1910 ferner eine Darstellung, die Zeitinformationen zu Trajektorien, die in den Nervenaktivitätsmuster enthalten sind, umfasst, so ist eine Merkmalsextraktion in einer besonders einfachen Weise möglich. Es können nämlich beispielsweise Zeitabstände zwischen den aufeinanderfolgenden Trajektorien berechnet werden, die dann für das Audiosignal charakteristisch sind.

Ferner hat sich auch gezeigt, dass eine Form der Trajektorien ein besonders wichtiges Merkmal ist. Somit ist es möglich, in einem Audiosignal-Fingerabdruck nur Informationen zu Trajektorien einer besonderen Form zu speichern, wodurch eine Datenmenge des Audiosignal-Fingerabdrucks 1930 verringert werden kann. Dies wiederum ermöglicht eine effiziente Ablage des Audiosignal-Fingerabdrucks in einer Datenbank.

Ferner hat sich gezeigt, dass einem Schallereignis typischerweise mehrere Trajektorien zugeordnet sind, die eine charakteristische Form und einen charakteristischen Abstand aufweisen. Somit kann beispielsweise bei der Merkmalsextraktion 1920 eine Gruppe von einer Mehrzahl von Trajektorien ausgewertet werden, woraufhin jeweils einer Gruppe von Trajektorien ein Symbol zugeordnet wird. Eine Folge von Symbolen, die zu aufeinanderfolgenden Gruppen von Trajektorien gehören, bilden dann den Audiosignal-Fingerabdruck und können in einer Datenbank abgelegt werden. Basierend auf einer Analyse einer Gruppe von Trajektorien kann ferner eine Spracherkennung realisiert werden, da Vokalen und Konsonanten jeweils charakteristische Trajektorien-Muster (im Hinblick auf Abstand und Form der Trajektorien) aufweisen, die erkannt werden können.

Zusammenfassend lässt sich also festhalten, dass die vorliegende Erfindung ein neurophysiologisch parametrisiertes Modell der ersten Stufen des Gehörsystems (first stages of the auditory system model) schafft. Das Modell setzt sich aus einer Cochlea-Modellierung, einem harmonischen Oszillatormodell der Stereozilienbewegungsgleichung sowie einem Modell für eine Neurotransmitter-Vesikel-Freisetzung der inneren Hörzellen (IHC Neurotransmitter Vesikel Release) zusammen. Ferner umfasst das erfindungsgemäße Modell eine Beschreibung für die Generierung von postsynaptischen Aktionspotentialen. Es wird hierbei darauf hingewiesen, dass bevorzugt ein Federkonstantenwert für die Beschreibung der Stereozilien verwendet wird, der mit Hilfe eines Rasterkraftelektronenmikroskops ermittelt ist. Das erfindungsgemäße Modell kann beispielsweise zur Optimierung einer Simulation von Cochlea-Implantaten dienen, da Cochlea-Implantate direkt den auditorischen Nerv anregen.

Ein wesentlicher Vorteil der vorliegenden Erfindung besteht darin, dass die Vorrichtung in einer neurophysiologischen Weise angepasst ist. Ferner wird in einer vorteilhaften Weise eine stochastische Ausschüttung von Neurotransmitter-Vesikeln berücksichtigt. Für eine Ankoppelung der Basilarmembrangeschwindigkeit an die Stereozilienbewegung wird ein erfindungsgemäßes Oszillatormodell verwendet, wobei die Bewegungsgleichungen für die Stereozilienbewegung optimiert ist. Eine Kräftegleichung bzw. Bewegungsgleichung für die Stereozilienbewegung lautet:

$$m\ddot{x} = -Dx - k\dot{x} + F_{ext} + F_{brown}\ .$$

Dabei beschreibt Dx die Federrückstellkraft, $k\dot{x}$ einen laminaren Strömungswiderstand, $F_{ext}$ (Proportionalitätskonstante x Basilarmembrangeschwindigkeit v) eine Anregung und $F_{brown}$ eine stochastische thermische Kraft durch Stoßbewegung der Atome.

Eine post-synaptische Generierung von Aktionspotentialen wird ebenso modelliert (vgl. E. Neher und T. Sakaba: "Quantal release parameters estimated from noise" J. Neuroscience, December 15, 2001, 21 (24):9638-9654).

In anderen Worten, die vorliegende Erfindung zeigt eine Vorrichtung zur Analyse eines Audiosignals, wobei die ersten Stufen des Gehörsystems von der mechanischen Schallwandlung im Innenohr, der Übertragung durch die Gehörknöchelchen, der hydromechanischen Schwingungsanregung der Cochlea, der mechano-elektrischen Wandlung an den inneren Haarzellen bis hin der Erzeugung der Puls-Spitzen (Puls-Spike-Generierung) der Spiralganglienzellen des auditorischen Nervs modelliert sind.

Ein besonderer Vorteil der vorliegenden Erfindung liegt in der Verwendung des zur Basilaranregung verwendeten Modells. Ferner wird bei der vorliegenden Erfindung eine besonders vorteilhafte Modellierung der inneren Haarzellen verwendet. Eine Freisetzung von Neurotransmittern in einem synaptischen Spalt erfolgt gemäß der vorliegenden Erfindung in Vesikel verpackt. Die vorliegende Erfindung umfasst ferner ein besonders vorteilhaftes Modell einer Kopplung zwischen der Basilarmembran und einer Bewegung der Stereozilien, wobei ein harmonisches Oszillatormodell verwendet wurde. Ein weiterer Vorteil der vorliegenden Erfindung liegt auch in einer neurophysiologischen Parametrisierung. Für eine Federkonstante (spring stiffness konstante) wurde ein IHC-Tabellenwert für die Maus angenommen. Eine post-synaptische Spitzenerzeugung (Post-synaptische Spike-Generierung) erfolgt erfindungsgemäß unter Verwendung eines Diffusions-/Refraktärmodells). Die vorliegende Erfindung ist somit in der Lage, Gehörvorgänge sehr realistisch nachzubilden, also zu erzeugen und zu analysieren.

Im Übrigen sei darauf hingewiesen, dass die vorliegende Erfindung auch eine Vorrichtung zur Expansion von Wanderwellen auf einer Cochlea (Cochlea travelling wave expansion) schafft. Die entsprechende Vorrichtung basiert auf einer frequenzabhängigen Berechnungsformel für Verzögerungstrajektorien (Delay-Trajektorien) längs einer Basilarmembran. Diese Formel beruht auf den Arbeiten von Greenberg. Die Verzögerung der Trajektorien entsteht durch die Wellengruppengeschwindigkeit bei einer Ausbreitung eines Impulses auf der Basilarmembran eines Ohrmodells. Tiefe Frequenzen ergeben einen zeitverzögerten Ausschlag auf der Basilarmembran, da die tiefen Frequenzen an dem Ende der Basilarmembran registriert werden. Somit ist für die tiefen Frequenzen die Laufzeitverzögerung der Basilarmembran wirksam.

Gekrümmte Bahnen bzw. Trajektorien können mit einer Hough-Transformation vorteilhaft bestimmt werden. So eignet sich die Hough-Transformation sehr gut, um Kreise, Ellipsen oder Geraden zu erkennen, aber auch andere linienförmige Kurven können mit Hilfe einer Hough-Transformation erkannt werden. Im Übrigen wird darauf hingewiesen, dass ein Zeit-Weiterleitungs-Netzwerk (timing feed forward network) gemäß der Hubel-Wiesel-Theorie Muster wie Balken oder Sinusaudiosignale unterschiedlicher Frequenz selbst lernen kann. In anderen Worten, ein Hubel-Wiesel-Netzwerk kann selbststrukturierend und selbstorganisierend eine Hough-Transformation nach einer beschriebenen Verzögerungsleitungs-Methode lernen.

Eine besondere Erkenntnis der vorliegenden Erfindung liegt darin, dass die Hough-Transformation zur Informationsgewinnung aus den parallelen Puls-Spitzen-Zügen (Pulse spiking trains) direkt mit dem auditorischen Nerv eines Ohrmodells gekoppelt wird. In anderen Worten, die Hough-Transformation wird direkt an den auditorischen Nerv angeflanscht, um die parallelen "Puls spiking trains" zu verarbeiten. Die Hough-Transformation biegt hierbei Verzögerungs-Trajektorien gerade und detektiert sowohl die Signalform (Krümmung) als auch den Auftrittzeitpunkt der Trajektorien. Bei der Hough-Transformation durchlaufen die Daten kontinuierlich mehrere Stufen. Eine Zeitfensterung ist hierbei, anders als bei bekannten Analysemethoden, nicht erforderlich.

In anderen Worten, die vorliegende Erfindung modelliert die ersten Stufen des Gehörsystems, von der mechanischen Schallwandlung im Innenohr, der Übertragung durch die Gehörknöchelchen, der hydromechanischen Schwingungsanregung der Cochlea, der mechano-elektrischen Wandlung an den inneren Haarzellen bis hin zur Erzeugung von Pulsspitzen (Puls spike Generierung) in den Spiralganglienzellen des auditorischen Nervs. Jedes Audiosignal generiert ein zweidimensionales Basilarmembrangeschwindigkeitsprofil aufgetragen gegen die Zeit, wobei die Basilarmembran bevorzugt in n-Sektionen unterteilt ist. Ein Klick-Impuls erzeugt dabei eine Wanderwellenbewegung auf der Basilarmembran. Eine Verzögerungs-Trajektorie der Faserlatenzzeit des auditorischen Nervs (AN-fiber latency) eines sinusförmigen Anregungssignals ist frequenzabhängig und berechnet sich nach der Formel von Greenberg zu $d_a = 1000/f_i + 2$ ms. Ein Audiosignal, das beispielsweise Vokale umfasst, ist durch ein Bündel von Verzögerungs-Trajektorien gegeben, wobei die Verzögerungs-Trajektorien eine jeweils frequenzabhängige Form aufweisen.

Die vorliegende Erfindung ermöglicht es hierbei, die Verzögerungs-Trajektorien zu detektieren. Hierbei kann bevorzugterweise ein erfindungsgemäßer Hubel-Wiesel-Neurosimulator eingesetzt werden, der die Detektion von sinusförmigen Mustern oder Geraden selbst erlernen kann. Ein entsprechender Hubel-Wiesel-Neurosimulator kann bevorzugt eine parallele Hough-Transformation lernen, die vorteilhaft in einer erfindungsgemäßen Vorrichtung zur Erzeugung eines Analysesignals aufgrund eines Audiosignals eingesetzt werden kann.

Die Verzögerungs-Trajektorien werden erfindungsgemäßer Weise durch eine parallele Hough-Transformation bestimmt, wobei vermerkt wird, zu welchem Zeitpunkt welche Verzögerungs-Trajektorie gefunden wird, um dadurch die vorliegende Signalform zu identifizieren. Bei einem erfindungsgemäßen Einsatz einer Hough-Transformation kann aus einem Nervenaktivitätsmuster (neural activity pattern) direkt ein Größen-Form-Abbild (size-shape-image) in einem Schritt erzeugt werden. Eine weitere Analyse des Audiosignals kann erfindungsgemäßer Weise in einem Hough-Raum (hough-space) erfolgen.

Es wird ferner darauf hingewiesen, dass die erfindungsgemäße Vorrichtung auch ein erfindungsgemäßes Verfahren definiert. Das Verfahren kann in beliebiger Weise durchgeführt werden, wobei sich eine elektronische Recheneinrichtung besonders gut für die Durchführung des erfindungsgemäßen Verfahrens eignet.

In anderen Worten, die erfindungsgemäße Vorrichtung und das erfindungsgemäße Verfahren kann in Hardware oder in Software implementiert werden. Die Implementation kann auf einem digitalen Speichermedium, beispielsweise einer Diskette, einer CD, einer DVD oder einem FLASH-Speicher mit elektronisch auslesbaren Steuersignalen erfolgen, die so mit einem programmierbaren Computersystem zusammenwirken können, dass das entsprechende Verfahren ausgeführt wird. Allgemein besteht die vorliegende Erfindung somit auch in einem Computer-Programm-Produkt mit auf einem maschinenlesbaren Träger gespeicherten Programmcode zur Durchführung des erfindungsgemäßen Verfahrens, wenn das Computerprogramm-Produkt auf einem Rechner abläuft. In anderen Worten ausgedrückt, kann die Erfindung somit als ein Computer-Programm mit einem Programmcode zur Durchführung des Verfahrens realisiert werden, wenn das Computer-Programm auf einem Computer abläuft.

Die vorliegende Erfindung zeigt also die Bearbeitung eines Basilarmembran-Schwingungsmusters mit Hilfe der Hough-Transformation.

**Patentansprüche**

1. Vorrichtung (100) zur Analyse eines Audiosignals (110), um eine Analysedarstellung (150) des Audiosignals (110) zu erhalten, mit folgenden Merkmalen:

    einer Einrichtung (120) zum Umsetzen des Audiosignals (110) in eine Darstellung, die ein Neurotransmitter-Vesikel-Auftreten (122,124,126) in Spalten zwischen einer Mehrzahl von Nervenfasern und inneren Hörzellen eines Ohrmodells wiedergibt; und

einer Einrichtung (130, 132, 134) zum Berechnen eines Nervenaktivitätsmusters über die Zeit auf der Mehrzahl von Nervenfasern, das sich aufgrund des Neurotransmitter-Vesikel-Auftretens (122,124,126) ergibt, wobei das Nervenaktivitätsmuster die Analysedarstellung (150) des Audiosignals ist;

wobei die Einrichtung zum Umsetzen des Audiosignals ausgelegt ist, um ein in Form von Vesikeln quantisiertes Auftreten von Neurotransmittern zu berechnen,

wobei die Einrichtung zum Umsetzen des Audiosignals ausgelegt ist, um eine Freisetzungs-Wahrscheinlichkeit für Neurotransmitter-Vesikel durch Analyse von mechanischen, chemischen und elektrischen Abläufen in einer Hörzelle zu berechnen, und um basierend auf der Freisetzungs-Wahrscheinlichkeit eine Neurotransmitter-Vesikel-Freisetzung durch eine stochastische Auswertung zu berechnen;

wobei die Vorrichtung ausgelegt ist, um Aktionspotentiale auf Nervenfasern von Neurotransmitter-Vesikel-Auftreten abzuleiten,

wobei eine Vesikel-Freisetzung über Zeit und Frequenz Trajektorien aufweist, und wobei die Trajektorien durch eine Modellierung eines synaptischen Spalts auf Trajektorien von Aktionspotentialen abgebildet werden;

wobei die Einrichtung (130,132,134) zum Berechnen des Nervenaktivitätsmusters eine Einrichtung (610) zum Berechnen einer Spannung in einer Nervenzelle aufgrund des Neurotransmitter-Vesikel-Auftretens (122,124,126) sowie eine Einrichtung (620) zum Entscheiden über eine Freisetzung eines Aktionspotentials, das eine einer Nervenfaser zugeordneten Spitze in dem Nervenaktivitätsmuster bildet, basierend auf der Spannung der Nervenzelle, aufweist;

wobei die Einrichtung (120) zum Umsetzen des Audiosignals

eine Einrichtung (220) zum Umsetzen des Audiosignals in eine Darstellung, die eine Bewegung (230) einer Basilarmembran in dem Ohrmodell beschreibt,

eine Einrichtung (240) zum Umsetzen der Darstellung (230), die die Bewegung der Basilarmembran beschreibt, in eine Darstellung(250), die eine Auslenkung eines Stereoziliums einer mit der Basilarmembran gekoppelten Haarzelle beschreibt, und

eine Einrichtung (260) zum Umsetzen der Darstellung (250), die die Auslenkung des Stereoziliums beschreibt, in eine Darstellung, die das Neurotransmitter-Vesikel-Auftreten beschreibt, umfasst;

wobei die Einrichtung (240) zum Umsetzen der Darstellung (330), die die Bewegung der Basilarmembran beschreibt, in die Darstellung (250), die die Auslenkung eines Stereoziliums beschreibt, ausgelegt ist, um das Stereozilium durch ein Modell eines harmonischen Oszillators zu beschreiben, der eine Feder-Rückstellkraft und eine Dämpfung aufgrund eines laminaren Strömungswiederstands aufweist;

wobei das Modell (400) des harmonischen Oszillators so ausgelegt ist, dass eine Anregung des harmonischen Oszillators durch eine zweite Kraftkomponente $F_{stoch}$ erfolgt, die eine stochastische thermische Kraft beschreibt.

2. Vorrichtung gemäß Anspruch 1, bei der die Spannung in der Nervenzelle ein post-synaptisches Potential beschreibt, das an einer post-synaptischen Membran anliegt.

3. Vorrichtung gemäß Anspruch 1 oder 2, bei der die Einrichtung (610) zum Berechnen der Spannung in der Nervenzelle ausgelegt ist, um die Spannung zu erhöhen, wenn ein Neurotransmitter-Vesikel auftritt, und bei der die Einrichtung (620) zum Entscheiden über eine Freisetzung eines Aktionspotentials ausgelegt ist, um ein Aktionspotential zu erzeugen, wenn ein Neurotransmitter-Vesikel auftritt und wenn eine vorhergehende Freisetzung eines Neurotransmitter-Vesikels länger als eine vorgegebene Refraktärzeit zurückliegt.

4. Vorrichtung gemäß Anspruch 1 oder 2, bei der die Einrichtung (610) zum Berechnen der Spannung in der Nervenzelle ausgelegt ist, um die Spannung in der Nervenzelle unter Verwendung eines externen Stroms, der durch das Neurotransmitter-Vesikel-Auftreten bestimmt ist, und eines Ionenaustauschstroms, der durch einen Ionenaustausch durch eine Membran bestimmt ist, unter Berücksichtigung einer elektrischen Kapazität der Nervenzelle zu berechnen.

5. Vorrichtung gemäß einem der Ansprüche 1 bis 4, bei der die Einrichtung (620) zum Entscheiden über eine Freisetzung eines Aktionspotentials ausgelegt ist, um ein Aktionspotential freizusetzen, wenn die Spannung in der Nervenzelle größer als ein Schwellenwert ist, wobei ein zeitlicher Verlauf des Schwellenwerts von zumindest einer zurückliegenden Freisetzung eines Aktionspotentials abhängig ist.

6. Vorrichtung (100) gemäß einem der Ansprüche 1 bis 5, bei der die Einrichtung (610) zum Berechnen der Spannung in der Nervenzelle aufgrund des Neurotransmitter-Vesikel-Auftretens (122,124,126) eine Einrichtung zum Bestimmen einer Neurotransmitter-Rest-Konzentration aufweist, die ausgelegt ist, um bei der Bestimmung der Neurotransmitter-Rest-Konzentration einen Einfluss einer Diffusion von Neurotransmitter-Vesikeln zu berücksichtigen, und wobei die Einrichtung zum Berechen der Spannung in der Nervenzelle ferner ausgelegt ist, um die Spannung in der Nervenzelle in Abhängigkeit von der Neurotransmitter-Restkonzentration zu berechnen.

7. Vorrichtung gemäß Anspruch 6, bei der die Einrichtung zum Bestimmen der Neurotransmitter-Restkonzentration ausgelegt ist, um die Neurotransmitter-Restkonzentration durch Auswerten eines Faltungsintegrals über ein Produkt aus einem zeitabhängigen Neurotransmitter-Vesikel-Auftreten und einem Diffusionsterm zu bestimmen.

8. Vorrichtung gemäß einem der Ansprüche 1 bis 7, bei der das Modell (400) des harmonischen Oszillators so ausgelegt ist, dass eine Anregung des harmonischen Oszillators durch eine erste Kraftkomponente erfolgt, die proportional einer Geschwindigkeit v(t), die die Bewegung der Basilarmembran beschreibt, ist.

9. Vorrichtung gemäß einem der Ansprüche 1 bis 8, bei der die stochastische thermische Kraft $F_{stoch}$ eine Brownsche Bewegung beschreibt.

10. Vorrichtung gemäß einem der Ansprüche 1 bis 9, bei der das Modell (400) des harmonischen Oszillators durch eine Bewegungsgleichung der Form

$$m\ddot{x} = -Dx - K\dot{x} + F_{ext}(t) + F_{stoch}(t)$$

beschrieben ist,

> wobei -Dx eine Federrückstellkraft darstellt;
> wobei $-k\dot{x}$ einen laminaren Strömungswiderstand darstellt;
> wobei $F_{ext}$ eine externe Kraft darstellt, die proportional zu einer Geschwindigkeit v(t) der Basilarmembran ist;
> und wobei $F_{brown}$ eine stochastische thermische Kraft durch eine Stoßbewegung von Atomen darstellt.

11. Vorrichtung gemäß einem der Ansprüche 1 bis 10, bei der eine Federkonstante D, die einen Zusammenhang zwischen der Feder-Rückstellkraft und der Auslenkung des Stereoziliums beschreibt, in einem Bereich zwischen 1 x $10^{-3}$ N/m und 6 x $10^{-3}$ N/m liegt.

12. Vorrichtung gemäß einem der Ansprüche 1 bis 11, bei der die Einrichtung (340) zum Umsetzen der Darstellung (230), die die Bewegung der Basilarmembran beschreibt, in die Darstellung (250), die die Auslenkung des Stereoziliums beschreibt, ausgelegt ist, um die Auslenkung des Stereoziliums durch Anwendung einer Tiefpassfilterung auf eine Geschwindigkeit v(t), die die Bewegung der Basilarmembran beschreibt, zu bestimmen.

13. Vorrichtung gemäß einem der Ansprüche 1 bis 12, bei der die Einrichtung (260) zum Umsetzen der Darstellung (250), die die Auslenkung des Stereoziliums beschreibt, in die Darstellung, die das Neurotransmitter-Vesikel-Auftreten beschreibt, eine Einrichtung (510,520,530,540) zum Berechnen einer Kalzium-Konzentration $[Ca^{2+}](t)$ in der Nervenzelle aufgrund der Auslenkung u(t) des Stereoziliums und eine Einrichtung (550,560) zum Bestimmen eines Neurotransmitter-Vesikel-Auftretens in Abhängigkeit von der Kalziumkonzentration $[Ca^{2+}](t)$ in der Nervenzelle umfasst.

14. Vorrichtung gemäß Anspruch 13, bei der die Einrichtung (510,520,530,540) zum Berechnen einer Kalziumkonzentration $[Ca^{2+}](t)$ in der Nervenzelle eine Einrichtung zum Berechnen eines intrazellulären Potentials V(t) der Nervenzelle und eine Einrichtung (530,540) zum Berechnen der Kalziumkonzentration $[Ca^{2+}](t)$ in der Nervenzelle aufgrund des intrazellulären Potentials V(t) umfasst.

15. Vorrichtung gemäß Anspruch 14, bei der die Einrichtung (510,520) zum Berechnen des Potentials V(t) in der Nervenzelle eine Einrichtung (510) zum Berechnen eines Spitzen-Leitwerts G(u) in Abhängigkeit von einer Auslenkung u(t) des Stereoziliums und eine Einrichtung (520) zum Berechnen des intrazellulären Potentials V(t) der Nervenzelle aufgrund des Spitzenleitwerts G(u) umfasst, wobei der Spitzenleitwert G(u) beschreibt, wie durchlässig Ionenkanäle der Nervenzelle für Ionen sind.

16. Vorrichtung gemäß einem der Ansprüche 13 bis 15, bei der die Einrichtung (510, 520, 530, 540) zum Berechnen der Kalziumkonzentration $[Ca^{2+}](t)$ in der Nervenzelle eine Einrichtung (530) zum Berechnen eines Kalziumstroms $I_{ca}(t)$ aufgrund des intrazellulären Potentials V(t) und eine Einrichtung zum Berechnen der Kalziumkonzentration

$[Ca^{2+}](t)$ umfasst.

17. Vorrichtung gemäß einem der Ansprüche 13 bis 16, bei der die Einrichtung (260) zum Bestimmen des Neurotransmitter-Vesikel-Auftretens einer Einrichtung (550) zum Berechnen einer Transmitter-Freisetzungs-Rate k(t) aufgrund der Kalziumkonzentration $[Ca^{2+}](t)$ und eine Einrichtung zum Bestimmen des Neurotransmitter-Vesikel-Auftretens aufgrund der Transmitter-Freisetzungs-Rate k(t) umfasst.

18. Vorrichtung gemäß Anspruch 17, bei der die Einrichtung (560) zum Berechnen des Neurotransmitter-Vesikel-Auftretens aufgrund der Transmitter-Freisetzungs-Rate k(t) ausgelegt ist, um eine quantisierte Freisetzung eines Neurotransmitters in Form von Neurotransmitter-Vesikeln zu beschreiben.

19. Vorrichtung gemäß Anspruch 17 oder 18, bei der die Einrichtung (560) zum Bestimmen des Neurotransmitter-Vesikel-Auftretens aufgrund der Transmitter-Freisetzungs-Rate k(t) ausgelegt ist, um die Freisetzung des Neurotransmitters in Form von Neurotransmitter-Vesikeln unter Verwendung einer stochastischen Methode zu beschreiben.

20. Vorrichtung gemäß einem der Ansprüche 1 bis 19, bei der die Einrichtung (220) zum Umsetzen des Audiosignals in eine Darstellung, die eine Bewegung der Basilarmembran beschreibt, eine Einrichtung zum Berechnen einer mechanischen Schallwandlung in einem Innenohr, die ausgelegt ist, um eine Darstellung einer Anregung des Trommelfells des Ohrmodells zu erhalten,
eine Einrichtung zum Berechnen einer Schallübertragung über Gehörknöchelchen, die ausgelegt ist, um eine Darstellung einer Anregung eines ovalen Fensters zwischen einem Mittelohr und einer Cochlea, basierend auf einer Darstellung der Anregung des Trommelfells zu erhalten,
eine Einrichtung zum Berechnen einer Schwingungsanregung der Cochlea, die ausgelegt ist, um die Schwingungsanregung der Cochlea basierend auf einer Darstellung der Anregung des ovalen Fensters zu bestimmen, und
eine Einrichtung zur Berechnung der Bewegung der Basilarmembran aufgrund der Schwingungsanregung der Cochlea umfasst.

21. Vorrichtung gemäß einem der Ansprüche 1 bis 20, der das Nervenaktivitätsmuster eine Darstellung von Aktionspotentialen auf einer Mehrzahl von Nervenfasern, die unterschiedlichen Hörzellen des Ohrmodells zugeordnet sind, bildet.

22. Vorrichtung gemäß einem der Ansprüche 1 bis 21, wobei die Vorrichtung zur Analyse ferner eine Einrichtung zum Analysieren eines Audiosignalinhalts auf der Basis des Nervenaktivitätsmusters umfasst.

23. Vorrichtung gemäß Anspruch 22, bei der die Einrichtung (1800) zum Analysieren des Audiosignalinhalts ausgelegt ist, um das Nervenaktivitätsmuster (1830) mit einem in einer Datenbank (1820) gespeicherten Referenznervenaktivitätsmuster (1840) zu vergleichen.

24. Vorrichtung gemäß Anspruch 22 oder 23, die ferner eine Vorrichtung (1920) zur Merkmalsextraktion umfasst, die ausgelegt ist, um Nervenfasern zu identifizieren, auf denen eine überdurchschnittlich hohe Aktivität vorliegt, und um basierend auf den identifizierten Nervenfasern ein Maß für eine Tonhöhe des Audiosignals zu ermitteln.

25. Vorrichtung gemäß einem der Ansprüche 22 bis 24, die ferner eine Vorrichtung (1920) zur Merkmalsextraktion umfasst, die ausgelegt ist, um Veränderungen der Aktivität von Nervenfasern in die Nervenaktivitätsmuster zu bestimmen, um aufgrund der Veränderungen der Aktivität einen Rhythmus des Audiosignals zu erkennen.

26. Vorrichtung gemäß einem der Ansprüche 22 bis 25, bei der die Einrichtung (1800) zum Analysieren des Audiosignalinhalts ausgelegt ist, und basierend auf dem Nervenaktivitätsmuster Vokale und/oder Konsonanten zu erkennen.

27. Vorrichtung gemäß einem der Ansprüche 22 bis 26, die ferner eine Einrichtung (1920) zur Morkmalsextraktion umfasst, die ausgelegt ist, um das Nervenaktivitätsmuster (1910) als einen Fingerabdruck (1930) des Audisignals in einer Datenbank (1940) abzulegen.

28. Vorrichtung gemäß einem der Ansprüche 1 bis 27, wobei die Vorrichtung zum Analysieren des Audiosignals ferner eine Koppeleinrichtung umfasst, die ausgelegt ist, um das Nervenaktivitätsmuster an eine Mehrzahl von Nervenfasern eines menschlichen Patienten in Form von elektrischen und/oder chemischen Anregungen auszugeben.

29. Vorrichtung gemäß Anspruch 28, bei der die Koppeleinrichtung ein Cochlea-Implantat ist.

30. Vorrichtung gemäß einem der Ansprüche 1 bis 29, die ferner eine Einrichtung zum Verarbeiten des Nervenaktivitätsmusters umfasst, der ausgelegt ist, um als eine verbesserte Analysedarstellung eine Folge von Zeitinformationen zu erhalten, die eine zeitliche Lage von aufeinanderfolgenden Trajektorie beschreiben, wobei eine Trajektorie Aktivitätsimpulse auf verschiedenen Nervenfasern aufgrund des gleichen Ereignisses in dem Audiosignal umfasst.

31. Vorrichtung gemäß Anspruch 30, bei der die Einrichtung zum Verarbeiten des Nervenaktivitätsmusters eine Einrichtung zur Mustererkennung umfasst, die ausgelegt ist, um in einer zweidimensionalen Darstellung, die durch das Nervenaktivitätsmuster über der Zeit gebildet wird, ein gerades oder gekrümmtes linienförmiges Muster als eine Trajektorie zu erkennen, um eine zeitliche Lage der Trajektorie zu ermitteln und um eine zu der Trajektorie gehörige Zeitinformation als die verbesserte Analysedarstellung des Audiosignals zu liefern.

32. Vorrichtung gemäß Anspruch 31, die die Einrichtung zur Mustererkennung ferner ausgelegt ist, um eine Information über eine Form der Trajektorie als einen Teil der verbesserten Analysedarstellung zu liefern.

33. Vorrichtung gemäß einem der Ansprüche 30 bis 32, bei der die Einrichtung zum Verarbeiten des Nervenaktivitätsmusters ausgelegt ist, um eine zweidimensionale Darstellung der Nervenaktivitätsmusters schrittweise zu verzerren, um eine verzerrte zweidimensionale Darstellung des Nervenaktivitätsmusters zu erhalten, und um zu erkennen, wenn in einer verzerrten zweidimensionalen Darstellung des Nervenaktivitätsmusters eine näherungsweise gerade Linie enthalten ist, um die gerade Linie als Trajektorie zu erkennen, um eine zeitliche Lage der Trajektorie zu bestimmen und um eine zu der erkannten Trajektorie gehörige Zeitinformation zu liefern.

34. Vorrichtung gemäß einem der Ansprüche 30 bis 33, der die Vorrichtung zur Verarbeitung des Nervenaktivitätsmusters eine Vorrichtung zur Durchführung einer Hough-Transformation ist.

35. Computerimplementiertes Verfahren zur Analyse eines Audiosignals, um eine Analysedarstellung des Audiosignals zu erhalten, mit folgenden Schritten:

Umsetzen des Audiosignals in eine Darstellung, die ein Neurotransmitter-Vesikel-Auftreten in Spalten zwischen einer Mehrzahl von Nervenfasern und inneren Hörzellen eines Ohrmodells wiedergibt; und
Berechnen eines Nervenaktivitätsmusters über der Zeit, auf der Mehrzahl von Nervenfasern, die sich aufgrund des Neurotransmitter-Vesikel-Auftretens ergibt, wobei das Nervenaktivitätsmuster die Analysedarstellung des Audiosignals ist;
wobei das Verfahren ein Berechnen eines in Form von Vesikeln quantisierten Auftretens von Neurotransmittern umfasst,
wobei eine Freisetzungs-Wahrscheinlichkeit für Neurotransmitter-Vesikel durch Analyse von mechanischen, chemischen und elektrischen Abläufen in einer Hörzelle berechnet wird, und wobei basierend auf der Freisetzungs-Wahrscheinlichkeit eine Neurotransmitter-Vesikel-Freisetzung durch eine stochastische Auswertung berechnet wird;
wobei Aktionspotentiale auf Nervenfasern von Neurotransmitter-Vesikel-Auftreten abgeleitet werden,
wobei eine Vesikel-Freisetzung über Zeit und Frequenz Trajektorien aufweist, und wobei die Trajektorien durch eine Modellierung eines synaptischen Spalts auf Trajektorien von Aktionspotentialen abgebildet werden;
wobei das Berechnen des Nervenaktivitätsmusters ein Berechnen einer Spannung in einer Nervenzelle aufgrund des Neurotransmitter-Vesikel-Auftretens (122,124,126) sowie ein Entscheiden über eine Freisetzung eines Aktionspotentials, das eine einer Nervenfaser zugeordneten Spitze in dem Nervenaktivitätsmuster bildet, basierend auf der Spannung der Nervenzelle umfasst;
wobei das Umsetzen des Audiosignals
ein Umsetzen des Audiosignals in eine Darstellung, die eine Bewegung (230) einer Basilarmembran in dem Ohrmodell beschreibt,
ein Umsetzen der Darstellung (230), die die Bewegung der Basilarmembran beschreibt, in eine Darstellung (250), die eine Auslenkung eines Stereoziliums einer mit der Basilarmembran gekoppelten Haarzelle beschreibt, und ein Umsetzen der Darstellung (250), die die Auslenkung des Stereoziliums beschreibt, in eine Darstellung, die das Neurotransmitter-Vesikel-Auftreten beschreibt, umfasst;
wobei das Umsetzen der Darstellung (330), die die Bewegung der Basilarmembran beschreibt, in die Darstellung (250), die die Auslenkung eines Stereoziliums beschreibt, ein Beschreiben des Stereoziliums durch ein Modell eines harmonischen Oszillators, der eine Feder-Rückstellkraft und eine Dämpfung aufgrund eines laminaren Strömungswiderstands aufweist, umfasst;

wobei das Modell (400) des harmonischen Oszillators so ausgelegt ist, dass eine Anregung des harmonischen Oszillators durch eine zweite Kraftkomponente $F_{stoch}$ erfolgt, die eine stochastische thermische Kraft beschreibt.

**36.** Verfahren gemäß Anspruch 35 wobei das Modell (400) des harmonischen Oszillators durch eine Bewegungsgleichung der Form

$$m\ddot{x} = -Dx - K\dot{x} + F_{ext}(t) + F_{stoch}(t)$$

beschrieben ist,

wobei -Dx eine Federrückstellkraft darstellt;
wobei -$k\dot{x}$ einen laminaren Strömungswiderstand darstellt;
wobei $F_{ext}$ eine externe Kraft darstellt, die proportional zu einer Geschwindigkeit v(t) der Basilarmembran ist; und wobei $F_{brown}$ eine stochastische thermische Kraft durch eine Stoßbewegung von Atomen darstellt.

**37.** Computer-Programm mit Programmcode zur Durchführung des Verfahrens gemäß Anspruch 35 oder 36, wenn das Computer-Programm auf einem Computer abläuft.

**Claims**

**1.** A device (100) for analyzing an audio signal (110) to obtain an analysis representation (150) of the audio signal (110), comprising:

a means (120) for converting the audio signal (110) to a representation rendering a neurotransmitter vesicle occurrence (122, 124, 126) in clefts between a plurality of nerve fibers and inner auditory cells of an ear model; and a means (130, 132, 134) for calculating a neural activity pattern over time on the plurality of nerve fibers which results from the neurotransmitter vesicle occurrence (122, 124, 126), wherein the neural activity pattern is the analysis representation (150) of the audio signal;
wherein the means for converting the audio signal is configured to calculate an occurrence of neurotransmitters that is quantized in the form of vesicles,
wherein the means for converting the audio signal is configured to calculate a release probability for neurotransmitter vesicles by analyzing mechanical, chemical and electrical processes within an auditory cell, and to calculate neurotransmitter vesicle release on the basis of said release probability by means of a stochastic evaluation;
wherein the device is configured to derive action potentials on nerve fibers from neurotransmitter vesicle occurrences,
wherein vesicle release over time and frequency comprises trajectories, and wherein said trajectories are mapped onto trajectories of action potentials by a modeling a synaptic cleft;
wherein the means (130, 132, 134) for calculating the neural activity pattern comprises a means (610) for calculating a voltage in a nerve cell which results from the neurotransmitter vesicle occurrence (122, 124, 126), and a means (620) for deciding about a release of an action potential, which forms a peak associated with a nerve fiber in the neural activity pattern, on the basis of the voltage of the nerve cell;
wherein the means (120) for converting the audio signal includes
a means (220) for converting the audio signal to a representation describing a movement (230) of a basilar membrane in the ear model,
a means (240) for converting the representation (230) describing the movement of the basilar membrane to a representation (250) describing a deflection of a stereocilium of a hair cell coupled to the basilar membrane, and
a means (260) for converting the representation (250) describing the deflection of the stereocilium to a representation describing the neurotransmitter vesicle occurrence;
wherein the means (240) for converting the representation (330) describing the movement of the basilar membrane to the representation (250) describing the deflection of a stereocilium is implemented to describe the stereocilium by a model of a harmonic oscillator which comprises a spring return force and an attenuation due to a laminar flow resistance;
wherein the model (400) of the harmonic oscillator is implemented so that excitation of the harmonic oscillator is performed by a second force component $F_{stoch}$, which describes a stochastic thermal force.

2. The device as claimed in claim 1, wherein the voltage in the nerve cell describes a postsynaptic potential applied to a postsynaptic membrane.

3. The device as claimed in claim 1 or 2, wherein the means (610) for calculating the voltage in the nerve cell is implemented to increase the voltage when a neurotransmitter vesicle occurs, and wherein the means (620) for deciding about a release of an action potential is implemented to generate an action potential when a neurotransmitter vesicle occurs and when a preceding release of a neurotransmitter vesicle took place earlier than a predetermined refractory time.

4. The device as claimed in claim 1 or 2, wherein the means (610) for calculating the voltage in the nerve cell is implemented to calculate, while considering an electric capacity of the nerve cell, the voltage in the nerve cell using an external current determined by the neurotransmitter vesicle occurrence, and using an ion exchange current determined by an ion exchange through a membrane.

5. The device as claimed in any of claims 1 to 4, wherein the means (620) for deciding about a release of an action potential is implemented to release an action potential when the voltage in the nerve cell is higher than a threshold value, wherein a time course of the threshold value is dependent on at least one past release of an action potential.

6. The device (100) as claimed in any of claims 1 to 5, wherein the means (610) for calculating the voltage in the nerve cell that is due to the neurotransmitter vesicle occurrence (122, 124, 126) comprises a means for determining a neurotransmitter residual concentration which is implemented to take into account an influence of a diffusion of neurotransmitter vesicles in determining the neurotransmitter residual concentration,
and wherein the means for calculating the voltage in the nerve cell is further implemented to calculate the voltage in the nerve cell as a function of the neurotransmitter residual concentration.

7. The device as claimed in claim 6, wherein the means for determining the neurotransmitter residual concentration is implemented to determine the neurotransmitter residual concentration by evaluating a convolution integral of a product of a timedependent neurotransmitter vesicle occurrence and a diffusion term.

8. The device as claimed in any of claims 1 to 7, wherein the model (400) of the harmonic oscillator is implemented so that excitation of the harmonic oscillator is performed by a first force component, which is proportional to a velocity v(t) which describes the movement of the basilar membrane.

9. The device as claimed in any of claims 1 to 8, wherein the stochastic thermal force $F_{stoch}$ describes a Brownian movement.

10. The device as claimed in any of claims 1 to 9, wherein the model (400) of the harmonic oscillator is described by a movement equation of the following form

$$m\ddot{x} = -Dx - K\dot{x} + F_{ext}(t) + F_{stoch}(t)$$

wherein -Dx represents a spring return force;
wherein $-K\dot{x}$ represents a laminar flow resistance;
wherein $F_{ext}$ represents an external force proportional to a velocity v(t) of the basilar membrane; and
wherein $F_{brown}$ represents a stochastic thermal force by an impact movement of atoms.

11. The device as claimed in any of claims 1 to 10, wherein a spring constant D which describes a connection between the spring return force and the deflection of the stereocilium lies within a range between $1 \times 10^{-3}$ N/m and $6 \times 10^{-3}$ N/m.

12. The device as claimed in any of claims 1 to 11, wherein the means (340) for converting the representation (230) describing the movement of the basilar membrane to the representation (250) describing the deflection of the stereocilium is implemented to determine the deflection of the stereocilium by applying a low-pass filtering to a velocity v(t) describing the movement of the basilar membrane.

13. The device as claimed in any of claims 1 to 12, wherein the means (260) for converting the representation (250) describing the deflection of the stereocilium to the representation describing the neurotransmitter vesicle occurrence includes a means (510, 520, 530, 540) for calculating a calcium concentration $[Ca^{2+}](t)$ in the nerve cell based on the deflection u(t) of the stereocilium and a means (550, 560) for determining a neurotransmitter vesicle occurrence depending on the calcium concentration $[Ca^{2+}](t)$ in the nerve cell.

14. The device as claimed in claim 13, wherein the means (510, 520, 530, 540) for calculating a calcium concentration $[Ca^{2+}](t)$ in the nerve cell includes a means for calculating an intracellular potential V(t) of the nerve cell and a means (530, 540) for calculating the calcium concentration $[Ca^{2+}](t)$ in the nerve cell based on the intracellular potential V(t).

15. The device as claimed in claim 14, wherein the means (510, 520) for calculating the potential V(t) in the nerve cell includes a means (510) for calculating a peak conductance G(u) depending on a deflection u(t) of the stereocilium and a means (520) for calculating the intracellular potential V(t) of the nerve cell based on the peak conductance G(u), wherein the peak conductance G(u) describes how transmissive ion channels of the nerve cell are for ions.

16. The device as claimed in any of claims 13 to 15, wherein the means (510, 520, 530, 540) for calculating the calcium concentration $[Ca^{2+}](t)$ in the nerve cell includes a means (530) for calculating a calcium current $I_{ca}(t)$ based on the intracellular potential V(t) and a means for calculating the calcium concentration $[Ca^{2+}](t)$.

17. The device as claimed in any of claims 13 to 16, wherein the means (260) for determining the neurotransmitter vesicle occurrence includes a means (550) for calculating a transmitter release rate k(t) based on the calcium concentration $[Ca^{2+}](t)$ and a means for determining the neurotransmitter vesicle occurrence based on the transmitter release rate k(t).

18. The device as claimed in claim 17, wherein the means (560) for calculating the neurotransmitter vesicle occurrence based on the transmitter release rate k(t) is implemented to describe a quantized release of a neurotransmitter in the form of neurotransmitter vesicles.

19. The device as claimed in claim 17 or 18, wherein the means (560) for determining the neurotransmitter vesicle occurrence based on the transmitter release rate k(t)is implemented to describe the release of the neurotransmitter in the form of neurotransmitter vesicles using a stochastic method.

20. The device as claimed in any of claims 1 to 19, wherein the means (220) for converting the audio signal to a representation describing a movement of the basilar membrane includes a means for calculating a mechanical sound conversion in an inner ear, which is implemented to obtain a representation of excitation of the eardrum of the ear model,
a means for calculating a sound transmission via auditory ossicles, which is implemented to obtain a representation of excitation of an oval window between a middle ear and a cochlea based on a representation of the excitation of the eardrum,
a means for calculating an oscillation excitation of the cochlea, which is implemented to determine the oscillation excitation of the cochlea based on a representation of the excitation of the oval window, and
a means for calculating the movement of the basilar membrane based on the oscillation excitation of the cochlea.

21. The device as claimed in any of claims 1 to 20, wherein the neural activity pattern forms a representation of action potentials on a plurality of nerve fibers associated with different auditory cells of the ear model.

22. The device as claimed in any of claims 1 to 21, wherein the device for analyzing further includes a means for analyzing an audio signal content on the basis of a neural activity pattern.

23. The device as claimed in claim 22, wherein the means (1800) for analyzing the audio signal content is implemented to compare the neural activity pattern (1830) to a reference numeral activity pattern (1840) stored in a database (1820).

24. The device as claimed in claim 22 or 23, further including a device (1920) for a feature extraction, which is implemented to identify nerve fibers on which a higher than average activity is present, and to determine a measure for a pitch of the audio signal based on the identified nerve fibers.

25. The device as claimed in any of claims 22 to 24, further including a device (1920) for a feature extraction, which is implemented to detect changes of the activity of nerve fibers in the neural activity pattern to detect a rhythm of the audio signal based on the changes of the activity.

26. The device as claimed in any of claims 22 to 25, wherein the means (1800) is implemented for analyzing the audio signal content and to detect vocals and/or consonants based on the neural activity pattern.

27. The device as claimed in any of claims 22 to 26, further including a means (1920) for a feature extraction, which is implemented to store the neural activity pattern (1910) as a fingerprint (1930) of the audio signal in a database (1940).

28. The device as claimed in any of claims 1 to 27, wherein the device for analyzing the audio signal further includes a coupling means, which is implemented to output the neural activity pattern to a plurality of nerve fibers of a human patient in the form of electrical and/or chemical excitations.

29. The device as claimed in claim 28, wherein the coupling means is a cochlea implant.

30. The device as claimed in any of claims 1 to 29, further including a means for processing the neural activity pattern, which is implemented to obtain a sequence of pieces of time information as an improved analysis representation, which describe a temporal position of consecutive trajectories, wherein a trajectory includes activity impulses on different nerve fibers based on the same result in the audio signal.

31. The device as claimed in claim 30, wherein the means for processing the neural activity pattern includes a means for a pattern recognition, which is implemented to detect, in a two-dimensional representation formed by the neural activity pattern over time, a straight or curved line-shaped pattern as a trajectory to determine a temporal position of the trajectory and to provide a time information belonging to the trajectory as the improved analysis representation of the audio signal.

32. The device as claimed in claim 31, wherein the means for a pattern recognition is further implemented to provide an information about a shape of the trajectory as a part of the improved analysis representation.

33. The device as claimed in any of claims 30 to 32, wherein the means for processing the neural activity pattern is implemented to distort a two-dimensional representation of the neural activity pattern step-by-step in order to obtain a distorted two-dimensional representation of the neural activity pattern in order to detect when, in a distorted two-dimensional representation of the neural activity pattern, an approximately straight line is contained to detect the straight line as a trajectory to determine a temporal position of the trajectory and to provide a time information belonging to the detected trajectory.

34. The device as claimed in any of claims 30 to 33, wherein the device for processing the neural activity pattern is a device for performing a Hough transformation.

35. A computer-implemented method for analyzing an audio signal to obtain an analysis representation of the audio signal, comprising the following steps:

converting the audio signal to a representation representing a neurotransmitter vesicle occurrence in clefts between a plurality of nerve fibers and inner auditory cells of an ear model; and
calculating a neural activity pattern over time, on the plurality of nerve fibers, which results from the neurotransmitter vesicle occurrence, wherein the neural activity pattern is the analysis representation of the audio signal;
wherein the method includes calculating an occurrence of neurotransmitters that is quantized in the form of vesicles,
wherein a release probability for neurotransmitter vesicles is calculated by analyzing mechanical, chemical and electrical processes within an auditory cell, and wherein neurotransmitter vesicle release is calculated on the basis of said release probability by means of a stochastic evaluation;
wherein action potentials on nerve fibers are derived from neurotransmitter vesicle occurrences,
wherein vesicle release over time and frequency comprises trajectories, and wherein said trajectories are mapped onto trajectories of action potentials by a modeling a synaptic cleft;
wherein said calculating of the neural activity pattern includes calculating a voltage in a nerve cell which results from the neurotransmitter vesicle occurrence (122, 124, 126), and deciding about a release of an action potential, which forms a peak associated with a nerve fiber in the neural activity pattern, on the basis of the voltage of the nerve cell;
wherein said converting of the audio signal includes
converting the audio signal to a representation describing a movement (230) of a basilar membrane in the ear model,
converting the representation (230) describing the movement of the basilar membrane to a representation (250) describing a deflection of a stereocilium of a hair cell coupled to the basilar membrane, and

converting the representation (250) describing the deflection of the stereocilium to a representation describing the neurotransmitter vesicle occurrence;

wherein said converting of the representation (330) describing the movement of the basilar membrane to the representation (250) describing the deflection of a stereocilium includes describing the stereocilium by a model of a harmonic oscillator which comprises a spring return force and an attenuation due to a laminar flow resistance;

wherein the model (400) of the harmonic oscillator is implemented so that excitation of the harmonic oscillator is performed by a second force component $F_{stoch}$, which describes a stochastic thermal force.

36. The method as claimed in claim 35, wherein the model (400) of the harmonic oscillator is described by a movement equation of the following form

$$m\ddot{x} = -Dx - K\dot{x} + F_{ext}(t) + F_{stoch}(t)$$

wherein -Dx represents a spring return force;

wherein - $K\dot{x}$ represents a laminar flow resistance;

wherein $F_{ext}$ represents an external force proportional to a velocity v(t) of the basilar membrane; and

wherein $F_{brown}$ represents a stochastic thermal force by an impact movement of atoms.

37. A computer program having a program code for performing the method as claimed in claims 35 or 36, when the computer program runs on a computer.

**Revendications**

1. Dispositif (100) pour analyser un signal audio (110) pour obtenir une représentation d'analyse (150) du signal audio (110), aux caractéristiques suivantes:

un moyen (120) destiné à convertir le signal audio (110) en une représentation qui reproduit une apparition de vésicules de neurotransmetteurs (122, 124, 126) dans des fentes entre une pluralité de fibres nerveuses et des cellules auditives internes d'un modèle d'oreille; et

un moyen (130, 132, 134) destiné à calculer un modèle d'activité nerveuse dans le temps sur la pluralité de fibres nerveuses résultant de l'apparition de vésicules de neurotransmetteurs (122, 124, 126), où le modèle d'activité nerveuse est la représentation d'analyse (150) du signal audio,

dans lequel le moyen destiné à convertir le signal audio est conçu pour calculer une apparition de neurotransmetteurs quantifiée sous forme de vésicules,

dans lequel le moyen de conversion du signal audio est conçu pour calculer une probabilité de libération de vésicules de neurotransmetteurs par analyse de processus mécaniques, chimiques et électriques dans une cellule auditive et pour calculer par une évaluation stochastique, sur base de la probabilité de libération, une libération de vésicules de neurotransmetteurs;

le dispositif étant conçu pour dériver des potentiels d'action sur les fibres nerveuses de l'apparition de vésicules de neurotransmetteurs,

dans lequel une libération de vésicules dans le temps et en fréquence présente des trajectoires, et dans lequel les trajectoires sont reproduites par une modélisation d'un fente synaptique sur les trajectoires de potentiels d'action;

dans lequel le moyen (130, 132, 134) destiné à calculer le modèle d'activité nerveuse présente un moyen (610) destiné à calculer une tension dans une cellule nerveuse due à l'apparition de vésicules de neurotransmetteurs (122, 124, 126) ainsi qu'un moyen (620) destiné à décider sur une libération d'un potentiel d'action qui forme une pointe associée à une fibre nerveuse dans le modèle d'activité nerveuse sur base de la tension de la cellule nerveuse;

dans lequel le moyen (120) destiné à convertir le signal audio comporte

un moyen (220) destiné à convertir le signal audio en une représentation qui décrit un mouvement (230) d'une membrane basilaire dans le modèle d'oreille,

un moyen (240) destiné à convertir la représentation (230) qui décrit le mouvement de la membrane basilaire en une représentation (250) qui décrit une déflexion d'un stéréocilium d'une cellule ciliée couplée à la membrane basilaire, et

un moyen (260) destiné à convertir la représentation (250) qui décrit la déflexion du stéréocilium en une représentation qui décrit l'apparition de vésicules de neurotransmetteurs;

dans lequel le moyen (240) destiné à convertir la représentation (330) qui décrit le mouvement de la membrane basilaire en la représentation (250) qui décrit la déflexion d'un stéréocilium est conçu pour décrire le stéréocilium par un modèle d'un oscillateur harmonique qui présente une force de rappel par ressort et un amortissement dû à une résistance à l'écoulement laminaire;

dans lequel le modèle (400) de l'oscillateur harmonique est conçu de sorte qu'une excitation de l'oscillateur harmonique ait lieu par une deuxième composante de force $F_{stoch}$ qui décrit une force thermique stochastique.

2. Dispositif selon la revendication 1, dans lequel la tension dans la cellule nerveuse décrit un potentiel post-synaptique qui est appliqué à une membrane post-synaptique.

3. Dispositif selon la revendication 1 ou 2, dans lequel le moyen (610) destiné à calculer la tension dans la cellule nerveuse est conçu pour augmenter la tension lorsqu'il apparaît une vésicule de neurotransmetteurs, et dans lequel le moyen (620) destiné à décider sur la libération d'un potentiel d'action est conçu pour générer un potentiel d'action lorsqu'il apparaît une vésicule de neurotransmetteurs et qu'une libération précédente d'une vésicule de neurotransmetteurs a eu lieu bien avant une période réfractaire prédéterminée.

4. Dispositif selon la revendication 1 ou 2, dans lequel le moyen (610) destiné à calculer la tension dans la cellule nerveuse est conçu pour calculer la tension dans la cellule nerveuse à l'aide d'un courant externe qui est déterminé par l'apparition de vésicules de neurotransmetteurs et d'un courant d'échange d'ions qui est déterminé par un échange d'ions à travers une membrane, en tenant compte d'une capacité électrique de la cellule nerveuse.

5. Dispositif selon l'une des revendications 1 à 4, dans lequel le moyen (620) destiné à décider sur une libération d'un potentiel d'action est conçu pour libérer un potentiel d'action lorsque la tension dans la cellule nerveuse est supérieure à une valeur de seuil, dans lequel une évolution dans le temps de la valeur de seuil dépend d'au moins une libération antérieure d'un potentiel d'action.

6. Dispositif (100) selon l'une des revendications 1 à 5, dans lequel le moyen (610) destiné à calculer la tension dans la cellule nerveuse due à l'apparition de vésicules de neurotransmetteurs (122, 124, 126) présente un moyen destiné à déterminer une concentration résiduelle de neurotransmetteurs qui est conçu pour tenir compte, lors de la détermination de la concentration résiduelle de neurotransmetteurs, d'une influence d'une diffusion de vésicules de neurotransmetteurs,

et dans lequel le moyen destiné à calculer la tension dans la cellule nerveuse est par ailleurs conçu pour calculer la tension dans la cellule nerveuse en fonction de la concentration résiduelle de neurotransmetteurs.

7. Dispositif selon la revendication 6, dans lequel le moyen destiné à déterminer la concentration résiduelle de neurotransmetteurs est conçu pour déterminer la concentration résiduelle de neurotransmetteurs en évaluant une intégrale de convolution sur un produit d'une apparition de vésicules de neurotransmetteurs en fonction du temps et d'un terme de diffusion.

8. Dispositif selon l'une des revendications 1 à 7, dans lequel le modèle (400) de l'oscillateur harmonique est conçu de sorte qu'une excitation de l'oscillateur harmonique ait lieu par une première composante de force qui est proportionnelle à une vitesse v(t) qui décrit le mouvement de la membrane basilaire.

9. Dispositif selon l'une des revendications 1 à 8, dans lequel la force thermique stochastique $F_{stoch}$ décrit un mouvement brownien.

10. Dispositif selon l'une des revendications 1 à 9, dans lequel le modèle (400) de l'oscillateur harmonique est décrit par une équation de mouvement de forme

$$m\ddot{x} = Dx - K\dot{x} + F_{ext}(t) + F_{stoch}(t)$$

où -$Dx$ représente une force de rappel de ressort;

où -$K\dot{x}$ représente une résistance à l'écoulement laminaire;

où $F_{ext}$ représente une force externe qui est proportionnelle à une vitesse v(t) de la membrane basilaire; et

où $F_{brown}$ représente une force thermique stochastique due à un mouvement d'impact d'atomes.

**11.** Dispositif selon l'une des revendications 1 à 10, dans lequel la constante de ressort D, qui décrit un rapport entre la force de rappel de ressort et la déflexion du stéréocilium, est comprise entre $1 \times 10^{-3}$ N/m et $6 \times 10^{-3}$ N/m.

**12.** Dispositif selon l'une des revendications 1 à 11, dans lequel le moyen (340) destiné à convertir la représentation (230) qui décrit le mouvement de la membrane basilaire en la représentation (250) qui décrit la déflexion du stéréocilium est conçu pour déterminer la déflexion du stéréocilium en appliquant un filtrage passe-bas à une vitesse $v(t)$ qui décrit le mouvement de la membrane basilaire.

**13.** Dispositif selon l'une des revendications 1 à 12, dans lequel le moyen (260) destiné à convertir la représentation (250) qui décrit la déflexion du stéréocilium en la représentation qui décrit l'apparition de vésicules de neurotransmetteurs comporte un moyen (510, 520, 530, 540) destiné à calculer une concentration de calcium $[Ca^{2+}](t)$ dans la cellule nerveuse sur base de la déflexion $u(t)$ du stéréocilium et un moyen (550, 560) destiné à déterminer une apparition de vésicules de neurotransmetteurs en fonction de la concentration de calcium $[Ca^{2+}](t)$ dans la cellule nerveuse.

**14.** Dispositif selon la revendication 13, dans lequel le moyen (510, 520, 530, 540) destiné à calculer une concentration de calcium $[Ca^{2+}](t)$ dans la cellule nerveuse comporte un moyen destiné à calculer un potentiel intracellulaire $V(t)$ de la cellule nerveuse et un moyen (530, 540) destiné à calculer la concentration de calcium $[Ca^{2+}](t)$ dans la cellule nerveuse sur base du potentiel intracellulaire $V(t)$.

**15.** Dispositif selon la revendication 14, dans lequel le moyen (510, 520) destiné à calculer le potentiel $V(t)$ dans la cellule nerveuse comporte un moyen (510) destiné à calculer une conductance de pointe $G(u)$ en fonction d'une déflexion $u(t)$ du stéréocilium et un moyen (520) destiné à calculer le potentiel intracellulaire $V(t)$ de la cellule nerveuse sur base de la conductance de pointe $G(u)$, dans lequel la conductance de pointe $G(u)$ décrit le degré de perméabilité aux ions des canaux ioniques de la cellule nerveuse.

**16.** Dispositif selon l'une des revendications 13 à 15, dans lequel le moyen (510, 520, 530, 540) destiné à calculer la concentration de calcium $[Ca^{2+}](t)$ dans la cellule nerveuse comporte un moyen (530) destiné à calculer un courant de calcium $I_{ca}(t)$ sur base du potentiel intracellulaire $V(t)$ et un moyen destiné à calculer la concentration de calcium $[Ca^{2+}](t)$.

**17.** Dispositif selon l'une des revendications 13 à 16, dans lequel le moyen (260) destiné à déterminer l'apparition de vésicules de neurotransmetteurs (550) comporte un moyen destiné à calculer un taux de libération de transmetteurs $k(t)$ sur base de la concentration de calcium $[Ca^{2+}](t)$ et un moyen destiné à déterminer l'apparition de vésicules de neurotransmetteurs sur base du taux de libération de transmetteurs $k(t)$.

**18.** Dispositif selon la revendication 17, dans lequel le moyen (560) destiné à calculer l'apparition de vésicules de neurotransmetteurs sur base du taux de libération de transmetteurs $k(t)$ est conçu pour décrire une libération quantifiée d'un neurotransmetteur sous forme de vésicules de neurotransmetteurs.

**19.** Dispositif selon la revendication 17 ou 18, dans lequel le moyen (560) destiné à déterminer l'apparition de vésicules de neurotransmetteurs sur base du taux de libération de transmetteurs $k(t)$ est conçu pour décrire la libération du neurotransmetteur sous forme de vésicules de neurotransmetteurs à l'aide d'une méthode stochastique.

**20.** Dispositif selon l'une des revendications 1 à 19, dans lequel le moyen (220) destiné à convertir le signal audio en une représentation qui décrit un mouvement de la membrane basilaire comporte un moyen destiné à calculer une transduction acoustique mécanique dans une oreille interne qui est conçu pour obtenir une représentation d'une excitation de la membrane tympanique du modèle de l'oreille,
un moyen destiné à calculer une transmission de son par l'intermédiaire d'osselets qui est conçu pour obtenir une représentation d'une excitation d'une fenêtre ovale entre une oreille moyenne et une cochlée sur base d'une représentation de l'excitation de la membrane tympanique;
un moyen destiné à calculer une excitation de vibrations de la cochlée qui est conçu pour déterminer l'excitation de vibrations de la cochlée sur base d'une représentation de l'excitation de la fenêtre ovale, et
un moyen destiné à calculer le mouvement de la membrane basilaire sur base de l'excitation de vibrations de la cochlée.

**21.** Dispositif selon l'une des revendications 1 à 20, dans lequel le modèle d'activité nerveuse constitue une représentation de potentiels d'action sur une pluralité de fibres nerveuses qui sont associées à différentes cellules auditives du modèle d'oreille.

**22.** Dispositif selon l'une des revendications 1 à 21, dans lequel le dispositif d'analyse comporte par ailleurs un moyen destiné à analyser un contenu de signal audio sur base du modèle d'activité nerveuse.

**23.** Dispositif selon la revendication 22, dans lequel le moyen (1800) destiné à analyser le contenu de signal audio est conçu pour comparer le modèle d'activité nerveuse (1830) avec un modèle d'activité nerveuse de référence (1840) mémorisé dans un banc de de données (1820).

**24.** Dispositif selon la revendication 22 ou 23, comprenant par ailleurs un dispositif (1920) destiné à l'extraction de caractéristiques qui est conçu pour identifier les fibres nerveuses sur lesquelles est présente une activité supérieure à la moyenne et pour déterminer, sur base des fibres nerveuses identifiées, une mesure de la hauteur tonale du signal audio.

**25.** Dispositif selon l'une des revendications 22 à 24, comportant par ailleurs un dispositif (1920) destiné à l'extraction de caractéristiques qui est conçu pour déterminer les changements de l'activité des fibres nerveuses dans les modèles d'activité nerveuse, pour détecter, sur base des changements de l'activité, un rythme du signal audio.

**26.** Dispositif selon l'une des revendications 22 à 25, dans lequel le moyen (1800) est conçu pour analyser le contenu du signal audio et pour détecter, sur base du modèle d'activité nerveuse, les voyelles et/ou les consonnes.

**27.** Dispositif selon l'une des revendications 22 à 26, comportant par ailleurs un moyen destiné à l'extraction de caractéristiques (1920) qui est conçu pour mémoriser le modèle d'activité nerveuse (1910) sous forme d'une empreinte digitale (1930) du signal audio dans un banc de données (1940).

**28.** Dispositif selon l'une des revendications 1 à 27, dans lequel le moyen destiné à analyser le signal audio comporte par ailleurs un moyen de couplage configuré pour sortir le modèle d'activité nerveuse vers une pluralité de fibres nerveuses d'un patient humain sous forme de stimuli électriques et/ou chimiques.

**29.** Dispositif selon la revendication 28, dans lequel le moyen de couplage est un implant cochléaire.

**30.** Dispositif selon l'une des revendications 1 à 29, comportant par ailleurs un moyen destiné à traiter le modèle d'activité nerveuse qui est conçu pour obtenir, comme représentation d'analyse améliorée, une séquence d'informations temporelles qui décrivent une position dans le temps de trajectoires successives, où une trajectoire comporte des impulsions d'activité sur différentes fibres nerveuses sur base du même événement dans le signal audio.

**31.** Dispositif selon la revendication 30, dans lequel le moyen de traitement du modèle d'activité nerveuse comporte un moyen de détection de modèle qui est conçu pour détecter, dans une représentation bidimensionnelle qui est formée dans le temps par le modèle d'activité nerveuse, un modèle en forme de ligne rectiligne ou courbe comme trajectoire pour déterminer une position dans le temps de la trajectoire et pour fournir une information temporelle relative à la trajectoire comme représentation d'analyse améliorée du signal audio.

**32.** Dispositif selon la revendication 31, dans lequel le moyen de détection de modèle est par ailleurs conçu pour fournir une information sur une forme de la trajectoire comme partie de la représentation d'analyse améliorée.

**33.** Dispositif selon l'une des revendications 30 à 32, dans lequel le moyen destiné à traiter le modèle d'activité nerveuse est conçu pour déformer par étapes une représentation bidimensionnelle du modèle d'activité nerveuse pour obtenir une représentation bidimensionnelle déformée du modèle d'activité nerveuse et pour détecter lorsqu'une ligne environ droite est contenue dans une représentation bidimensionnelle déformée du modèle d'activité nerveuse, pour détecter la ligne droite comme trajectoire pour déterminer une position dans le temps de la trajectoire et pour fournir une information temporelle relative à la trajectoire détectée.

**34.** Dispositif selon l'une des revendications 30 à 33, dans lequel le dispositif destiné à traiter le modèle d'activité nerveuse est un dispositif destiné à effectuer une transformation de Hough.

**35.** Procédé mis en œuvre par ordinateur d'analyse d'un signal audio pour obtenir une représentation d'analyse du signal audio, aux étapes suivantes consistant à:

# EP 1 896 123 B1

convertir le signal audio en une représentation qui reproduit une apparition de vésicules de neurotransmetteurs dans des fentes entre une pluralité de fibres nerveuses et des cellules auditives internes d'un modèle d'oreille; et calculer un modèle d'activité nerveuse dans le temps sur la pluralité de fibres nerveuses qui résulte du fait de l'apparition de vésicules de neurotransmetteurs, où le modèle d'activité nerveuse est la représentation d'analyse du signal audio;

le procédé comportant le fait de calculer une apparition quantifiée sous forme de vésicules de neurotransmetteurs,

dans lequel une probabilité de libération de vésicules de neurotransmetteurs est calculée par analyse de processus mécaniques, chimiques et électriques dans une cellule auditive, et dans lequel est calculée, sur base de la probabilité de libération, une libération de vésicules de neurotransmetteurs par une évaluation stochastique;

dans lequel les potentiels d'action sur les fibres nerveuses sont dérivés de l'apparition de vésicules de neurotransmetteurs,

dans lequel une libération de vésicules présente des trajectoires dans le temps et en fréquence, et dans lequel les trajectoires sont reproduits par une modélisation d'une fente synaptique sur les trajectoires de potentiels d'action;

dans lequel le calcul du modèle d'activité nerveuse comporte le fait de calculer une tension dans une cellule nerveuse due à l'apparition de vésicules de neurotransmetteurs (122, 124, 126) ainsi que de décider sur une libération d'un potentiel d'action qui forme une pointe associée à une fibre nerveuse dans le modèle d'activité nerveuse sur base de la tension de la cellule nerveuse;

dans lequel la conversion du signal audio comporte le fait de

convertir le signal audio en une représentation qui décrit un mouvement (230) d'une membrane basilaire dans le modèle d'oreille,

convertir la représentation (230) qui décrit le mouvement de la membrane basilaire en une représentation (250) qui décrit la déflexion d'un stéréocilium d'une cellule ciliée couplée à la membrane basilaire, et

convertir la représentation (250) qui décrit la déflexion du stéréocilium en une représentation qui décrit l'apparition de vésicules de neurotransmetteurs;

dans lequel la conversion de la représentation (330) qui décrit le mouvement de la membrane basilaire en la représentation (250) qui décrit la déflexion d'un stéréocilium comporte le fait de décrire le stéréocilium par un modèle d'un oscillateur harmonique qui présente une force de rappel de ressort et un amortissement sur base d'une résistance à l'écoulement laminaire;

dans lequel le modèle (400) de l'oscillateur harmonique est conçu de sorte qu'une excitation de l'oscillateur harmonique ait lieu par une deuxième composante de force $F_{stoch}$ qui décrit une force thermique stochastique.

36. Procédé selon la revendication 35, dans lequel le modèle (400) de l'oscillateur harmonique est décrit par une équation de mouvement de forme

$$m\ddot{x} = Dx - K\dot{x} + F_{ext}(t) + F_{stoch}(t)$$

où -Dx représente une force de rappel de ressort;
où $-K\dot{x}$ représente une résistance à l'écoulement laminaire;
où $F_{ext}$ représente une force externe qui est proportionnelle à une vitesse v(t) de la membrane basilaire; et
où $F_{brown}$ représente une force thermique stochastique par un mouvement d'impact d'atomes.

37. Programme d'ordinateur avec un code de programme pour la mise en œuvre du procédé selon la revendication 35 ou 36 lorsque le programme d'ordinateur est exécuté sur un ordinateur.

38

FIGUR 1

200

AUDIOSIGNAL

210

BERECHNEN EINER
BASILARMEMBRAN-
BEWEGUNG
220

230
BASILARMEMBRAN-BEWEGUNG

BERECHNUNG EINER
AUSLENKUNG EINES
STEREOZILIUMS
240

250
AUSLENKUNG EINES STEREOZILIUMS

BERECHNEN EINES
NEUROTRANSMITTER-
VESIKEL-AUFTRETENS
260

NEUROTRANSMITTER-
VESIKEL-AUFTRETEN

FIGUR 2

300

AUDIOSIGNAL

310

┌─────────────────────┐
│    MECHANISCHE      │
│   SCHALLWANDLUNG    │  320
│    IM INNENOHR      │
└─────────────────────┘

324

ANREGUNG DES TROMMELFELLS

┌─────────────────────┐
│   BERECHNUNG DER    │
│  SCHALLÜBERTRAGUNG  │  330
│    ÜBER GEHÖR-      │
│   KNÖCHELCHEN       │
└─────────────────────┘

334

ANREGUNG DES OVALEN FENSTERS
ZWISCHEN MITTELOHR UND COCHLEA

┌─────────────────────┐
│   BERECHNUNG DER    │
│ HYDROMECHANISCHEN   │  340
│   SCHWINGUNGSAN-    │
│  REGUNG DER COCHLEA │
└─────────────────────┘

344

SCHWINGUNGSANREGUNG DER COCHLEA

┌─────────────────────┐
│   BERECHNUNG DER    │
│  BASILARMEMBRAN-    │  350
│     BEWEGUNG        │
└─────────────────────┘

354

BASILARMEMBRAN-
BEWEGUNG

# FIGUR 3

400

BASILARMEMBRAN-BEWEGUNG
v(t)

STOCHASTISCHE
KRAFT $F_{stoch}(t)$

LÖSEN DER BEWEGUNGSGLEICHNUNG:
$$m\ddot{x} = -Dx - K\dot{x} + F_{ext}(t) + F_{stoch}(t)$$
$$F_{ext} = C_{bas}v(t)$$

AUSLENKUNG EINES
STEREOCILIUMS x(t)

m: EFFEKTIVE MASSE EINES STEREOCILIUMS
D: EFFEKTIVE FEDERKONSTANTE DES STEREOCILIUMS
K: KONSTANTE FÜR LAMINAREN STRÖMUNGSWIDERSTAND
    DES STEREOCILIUMS
$C_{bas}$: KONSTANTE FÜR ANREGUNG DES STEREOCILIUMS
      AUFGRUND BASILARMEMBRANBEWEGUNG

FIGUR 4

500

AUSLENKUNG EINES
STEREOZILIUMS u(t)

510

BERECHNEN EINES
SPITZEN-LEITWERKS

SPITZEN-LEITWERT Q(u)

520

BERECHNEN EINES INTRA-
ZELLULÄREN HAARZELLEN-
POTENTIALS

INTRAZELLULÄRES HAARZELLEN-POTENTIAL v(t)

530

BERECHNEN EINES
CALCIUMSTROMS

CALCIUMSTROM $I_{Ca}(t)$

540

BERECHNEN EINER CALCIUM-
KONZENTRATION

CALZIUM-KONZENTRATION $[Ca^{2+}](t)$

550

BERECHNEN EINER TRANS-
MITTER-FREISETZUNGS-
RATE

TRANSMITTER-FREISETZUNGS-RATE k(t)

569

BESTIMMEN EINES NEURO-
TRANSMITTER-VESIKEL-
AUFTRETENS

NEUROTRANSMITTER-VESIKEL-
AUFTRETEN

FIGUR 5

600

NEUROTRANSMITTER-
VESIKEL-AUFTRETEN

610 — BERECHNEN EINER SPANNUNG IN EINER NERVEN-ZELLE

POSTSYNAPTISCHES POTENTIAL

620 — TREFFEN EINER ENTSCHEIDUNG ÜBER EINE FREI-SETZUNG EINES AKTIONS-POTENTIALS

AKTIONSPOTENTIAL

FIGUR 6

700

AUDIOSIGNAL

$\sqsubset$—710

BERECHNEN EINES NERVEN-
AKTIVITÄTSMUSTERS          OHRMODELL                    ~720

NERVEN-
AKTIVITÄTS-
MUSTER

t

$t_2$

NF1    NF2   NF3     NF4  NF5    ~750

~730

AP9  AP10

TRAJEKTORIE

AP6  AP7   AP8

~740

$t_1$

AP1  AP2   AP3   AP4   AP5

VERARBEITEN DES
NERVENAKTIVITÄTSMUSTERS                  ~754

760—⌐     $(t_2)$
              $(t_1)$

VERBESSERTE ANALYSEDARSTELLUNG

# FIGUR 7

NEURONALE REPRÄSENTATION

FIGUR 8

# BERECHNUNG DER VERZÖGERUNG ALS FUNKTION DER FREQUENZ

910

914

$$d_a = (1000/f_i) + 2 (ms)$$

920

924

LATENZZEIT DER AUDITORISCHEN NERVENFASER (ms)

12

9

6

3

0

DURCH EIN SINUSSIGNAL HERVOR-
GERUFENE LATENZZEIT DES HÖRNERVS

• Chinchilla (Ruggero & Rich, 1987)
— Cat (Goldstein et al, 1971)

0,1    0,5    1    2    4    8    16

CHARAKTERISTISCHE FREQUENZ (kHz)

912

930

BASILAR MEMBRAN

35    30    25    20    15    10    5    0

ANSTAND VON DER BASIS IN mm (SKAL. ZU EINER MENSCHLICHEN COCHLEA)

LATENZZEIT EINER ANREGUNG EINER EINZELNEN FASER DES HÖRNERVS
ALS FUNKTION DER SINUSFÖRMIGEN SIGNALFREQUENZ

## FIGUR 9

EP 1 896 123 B1

COCHLEAGRAM DES VOKALS i

DETAILS DES CHOCHLEAGRAMS ZEIGEN DIE FEINE ZEITLICHE UND RÄUMLICHE STRUKTUR
IN DER MODELLIERTEN ANTWORT AUF EINEN VOKAL /i/ (LINKS) UND AUF EINEN
NICHT-HARMONISCHEN TONKOMPLEX VON 700 Hz, 900 Hz UND 1100 Hz (RECHTS)

FIGUR 10

EP 1 896 123 B1

VOKAL "A":

CHOCHLEAGRAM
←—1110

FREISETZUNGS-
WAHRSCHEINLICHKEIT
←—1120

VESIKEL-FREISETZUNG
←—1130

EP 1 896 123 B1

FIGUR 11

DIE VERARBEITUNGSKETTE

1200

SCHALTUNGEN ZUR
DETEKTION DER ZEIT
ZWISCHEN ZWEI
SPITZEN

1220

1230

1260    1250

BASILARMEMBRAN

$F_0${

$F_1$

$F_2${

$F_3${

$F_0$

$F_1$

$F_2$

$F_3$

AUDIOSIGNAL

1210

AKKUSTISCHER
KERN

GEHIRN

1240

1260

FIGUR 12

EP 1 896 123 B1

FIGUR 13

FIGUR 14

## DAS SCHALTBILD DES HUBEL-WIESEL NETZES

**FIGUR 15**

VERZÖGERUNGSELEMENTE/ADDIERER

SCHWELLWERT-
REGISTER/
KOMPARATOREN

EP 1 896 123 B1

DAS HUBEL-WIESEL NETZ

1600

INPUT NEURONS <1:9>

1610

1620    1620

1610

1630    1630    1630    OUTPUT NEURONS <1:9>

DELAY CHAINS <1:9>

FIGUR 16

EP 1 896 123 B1

## DIE TRAININGSMUSTER

NEUN SINUSSE UNTERSCHIEDLICHER FREQUENZ

# FIGUR 17

1800

AUDIOSIGNAL-REPRÄSENTANT

—1830

VERGLEICHS-
EINRICHTUNG

1810—

1850

VERGLEICHS-
AUDIOSIGNAL-
REPRÄSENTANTEN

1840—

AUDIOSIGNAL
DATENBANK

1820—

## FIGUR 18

1900

AUDIOSIGNAL
REPRÄSENTANT

1910

MERKMALS-
EXTRAKTION

1920

AUDIOSIGNAL
FINGERABDRUCK

1930

DATEN-
BANK

1940

FIGUR 19

RECESSUS EPITYMPANICUS    INCUS
MALLEUS
M. TENSOR TYMPANI

TUBA EUSTACHII

STAPES

M. LEVATOR
VELI PALATINI

ABBILDUNG 3.1: AUDITORISCHE PERIPHERIE

## FIGUR 20

ABBILDUNG 3.2:
AUSSENOHRÜBERTRAGUNGS-
FUNKTION - ZYLINDRISCHE
OBERFLÄCHENDARSTELLUNG
DER BETRAGSANTWORT ALS
FUNKTION DES AZIMUTHWINKEL
AUF RADIALER ACHSE

## FIGUR 21

ABBILDUNG 3.3: SCHEMA VON MITTELOHR UND AUFGEROLLTER COCHLEA

## FIGUR 22

ABBILDUNG 3.4: HÖRFLÄCHE

## FIGUR 23

ABBILDUNG 3.5: QUERSCHNITT DER COCHLEA

## FIGUR 24

ABBILDUNG 3.6: SCHEMA DER HAARZELLE

## FIGUR 25

# ANATOMIE DER AUDITORISCHEN PERIPHERIE

TROMMELFELL

MITTELOHR

HÖRNERV

GERÄUSCH

AUDITORISCHER PFAD

COCHLEA

a) SPEKTRALANALYSE
b) UMWANDLUNG VON VIBRATION
   IN NEURALE IMPULSE

## FIGUR 26

EP 1 896 123 B1

MECHANISMUS DER SIGNALÜBERTRAGUNG I

3210

FIGUR 27

FIGUR 28

# DAS ERWEITERTE ZWICKER-MODELL

a) HYDROMECHANIK DES INNENOHRS

b) GEDRUCKTE SCHALTUNG VON a)

c) NICHTLINEARE RÜCKKOPPLUNG DER
ÄUSSEREN HAARZELLEN

EP 1 896 123 B1

## FIGUR 29

# SCHEMA: CORTISCHES ORGAN

1 IHC
2 AHC
3 CORTISCHER TUNNEL
4 BASILARMEMBRAN
5 RETIKULARMEMBRAN
6 TEKTORIALMEMBRAN
7 DEITERSCHE ZELLEN
8 NUELSCHER RAUM
9 HENSEN-ZELLEN
10 INNERER SPIRALSULCUS

FIGUR 30

EP 1 896 123 B1

# AUFBAU DER HAARZELLEN

1  NUCLEUS
2  STEREOCILIA
3  CUTICULAR PLATE
4  RADIAL AFFERENT ENDING
(DENDRITE OF TYPE I NEURON)
5  LATERAL EFFERENT ENDING
6  MEDIAL EFFERENT ENDING
7  SPIRAL AFFERENT ENDING
(DENDRITE OF TYPE II NEURON)

NUR CA. 3,500 IHCS UND 12,000 OHCS
IN DER HUMANEN COCHLEA ANGELEGT!

EP 1 896 123 B1

FIGUR 31

FIGUR 32

CUPULA

SENSOR-
ISCHE
GRUBE

KINOCILIUM

STEREOVILLUS

HAARZELLE

UNTER-
STÜTZENDE
ZELLE

SYNAPSE

NERVENFASER

FIGUR 33

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5381512 A
- WO 0199470 A1
- US 4536844 A
- US 3069654 A

**In der Beschreibung aufgeführte Nicht-Patentliteratur**

- **TOSHIO IRINO ; ROY D. PATTERSON.** Segregating Information about the size and shape of the vocal tract using a time domain auditory model: The Stabilized Wavelet-Mellin Transform. *Trennen von Informationen über die Größe und die Form des Vokaltrakts unter Verwendung eines Zeitdomänen-Gehörmodells: Die stabilisierte Wavelet-Mellin Transformation)(veröffentlicht in dem Elsevier-Journal for Speech Communication,* 2002, vol. 36, 181-203
- **A. BRÜCKMANN ; F. KLEFENZ ; A. WÜNSCHE.** A neural net for 2D-slope and sinusoidal shape detection. *CIST International Scientific Journal of Computing,* ISSN 1727-6209
- **MARCUS HOLMBERG ; WERNER HEMMERT.** Auditory Information Processing with Nerve-Action Potentials. *Proceedings der IEEE International Conference on Acoustics, Speech and Signal Processing,* 17. Mai 2004, vol. 4
- **SHIHAB A. SHAMMA ; RICHARD S. CHADWICK.** A biophysical model of cochelar processing: Intensity dependence of pure tone responses. *J. Acoust. Soc. Am,* Juli 1986, vol. 80 (1
- Ein psychophysiologisches Gehörmodell zur Nachbildung von Wahrnehmungsschwellen für die Audiocodierung. **F. BAUMGARTE.** Dissertation. Universität Hannover, 2000
- **C.J. SUMNER ; E.A. LOPEZ-POVEDA ; L.P. O'MARD ; R. MEDDIS.** A revised model of the inner hair cell and auditory nerve complex. *J. Acoust. Soc. Am.,* Mai 2002, vol. 111 (5
- **ERWIN NEHER ; TAKESHI SAKABA.** Estimating Transmitter Release Rates from Postsynaptic Current Fluctuations. *The Journal of Neuroscience,* 15. Dezember 2001, vol. 21 (24), 9638-9654
- **S. GREENBERG ; D. POEPPEL ; T. ROBERTS.** A Space-Time Theory of Pitch and Timbre Based on Cortical Expansion of the Cochelar Travelling Wave Delay. *XIth International Symposium on Hearing*
- **A. BRÜCKMANN ; S. KLEFENZ ; A. WÜNSCHE.** A neural net for 2D-slope and sinusoidal shape detection. *CIST International Scientific Journal of Computing,* ISSN 1727-6209
- **E. NEHER ; T. SAKABA.** Quantal release parameters estimated from noise. *J. Neurosience,* 15. Dezember 2001, vol. 21 (24), 9638-9654